(19) Europäisches Patentamt / European Patent Office / Office européen des brevets

(11) **EP 3 401 684 B1**

(12) **EUROPEAN PATENT SPECIFICATION**

(45) Date of publication and mention of the grant of the patent:
**16.03.2022 Bulletin 2022/11**

(21) Application number: **17210306.1**

(22) Date of filing: **09.10.2009**

(51) International Patent Classification (IPC):
***G01N 33/68*** (2006.01) ***C07K 14/435*** (2006.01)
***A61K 38/17*** (2006.01) ***A61P 35/00*** (2006.01)

(52) Cooperative Patent Classification (CPC):
**G16B 5/00; A61P 1/04; A61P 3/06; A61P 3/10; A61P 7/00; A61P 7/02; A61P 9/00; A61P 9/10; A61P 9/12; A61P 11/06; A61P 13/12; A61P 15/00; A61P 19/02; A61P 25/00; A61P 25/28;** (Cont.)

(54) **CHEMICAL MODULATORS OF PRO-APOPTOTIC BAX AND BCL-2 POLYPEPTIDES**
CHEMISCHE MODULATOREN PRO-APOPTOTISCHER BAX- UND BCL-2 POLYPEPTIDE
MODULATEURS CHIMIQUES DES POLYPEPTIDES BAX ET BCL-2 PRO-APOPTOTIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.10.2008 US 136906 P**

(43) Date of publication of application:
**14.11.2018 Bulletin 2018/46**

(62) Document number(s) of the earlier application(s) in accordance with Art. 76 EPC:
**09819587.8 / 2 338 056**


(73) Proprietor: **Dana Farber Cancer Institute, Inc.**
**Boston, Massachusetts 02215 (US)**

(72) Inventors:
- **WALENSKY, Loren, D.**
  **Chestnut Hill, MA 02467 (US)**
- **GAVATHIOTIS, Evripidis**
  **Boston, MA 02115 (US)**

(74) Representative: **Hiebl, Inge Elisabeth**
**Kraus & Weisert**
**Patentanwälte PartGmbB**
**Thomas-Wimmer-Ring 15**
**80539 München (DE)**



(56) References cited:
**WO-A2-2004/076640**

- **WALENSKY LOREN D ET AL: "A stapled BID BH3 helix directly binds and activates BAX", MOLECULAR CELL, CELL PRESS, CAMBRIDGE, MA, US, vol. 24, no. 2, 20 October 2006 (2006-10-20), pages 199-210, XP009107957, ISSN: 1097-2765, DOI: 10.1016/J.MOLCEL.2006.08.020**
- **OLTERSDORF T ET AL: "an inhibitor of BCL-2 family proteins induces regression of solid tumours", NATURE: INTERNATIONAL WEEKLY JOURNAL OF SCIENCE, NATURE PUBLISHING GROUP, UNITED KINGDOM, vol. 435, 2 June 2005 (2005-06-02), pages 677-681, XP002491257, ISSN: 0028-0836, DOI: 10.1038/NATURE03579**
- **RAMZI MOHAMMAD ET AL: "Small-Molecule Inhibitors of Bcl-2 Family Proteins as Therapeutic Agents in Cancer", RECENT PATENTS ON ANTI-CANCER DRUG DISCOVERY, vol. 3, no. 1, 1 January 2008 (2008-01-01) , pages 20-30, XP055050403, ISSN: 1574-8928, DOI: 10.2174/157489208783478676**
- **SUZUKI ET AL: "Structure of Bax: coregulation of dimer formation and intracellular localization.", CELL, vol. 103, no. 4, 1 November 2000 (2000-11-01), pages 645-654, XP055050116, ISSN: 0092-8674**
- **SATTLER M ET AL: "STRUCTURE OF BCL-XL-BAK PEPTIDE COMPLEX: RECOGNITION BETWEEN REGULATORS OF APOPTOSIS", SCIENCE, AMERICAN ASSOCIATION FOR THE ADVANCEMENT OF SCIENCE, WASHINGTON, DC; US, vol. 275, no. 5302, 14 February 1997 (1997-02-14), pages 983-986, XP001120657, ISSN: 0036-8075, DOI: 10.1126/SCIENCE.275.5302.983**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).





**(Cont. next page)**

- **YOULE RICHARD J ET AL: "The BCL-2 protein family: opposing activities that mediate cell death.", NATURE REVIEWS. MOLECULAR CELL BIOLOGY JAN 2008, vol. 9, no. 1, January 2008 (2008-01), pages 47-59, XP009166365, ISSN: 1471-0080**
- **EVRIPIDIS GAVATHIOTIS ET AL: "BAX activation is initiated at a novel interaction site", NATURE, vol. 455, no. 7216, 23 October 2008 (2008-10-23), pages 1076-1081, XP055050074, ISSN: 0028-0836, DOI: 10.1038/nature07396**
- **Anonymous: "Hit2Lead | Search | Screening Compounds | Search by ID; 5258079; 3-methyl-1-(4-phenyl-1,3-thiazol-2-yl)-1H-pyrazole-4,5-dione 4-[(4-ethoxyphenyl)hydrazone]", , 28 July 2005 (2005-07-28), XP55719284, Retrieved from the Internet: URL:https://www.hit2lead.com/result.asp?search=30007416 [retrieved on 2020-07-30]**
- **Pubchem: "SID 3315682 - PubChem", , 28 July 2005 (2005-07-28), XP55719287, Retrieved from the Internet: URL:https://pubchem.ncbi.nlm.nih.gov/substance/3315682 [retrieved on 2020-07-30]**


(52) Cooperative Patent Classification (CPC): (Cont.)
**A61P 35/00; A61P 35/02; A61P 35/04; A61P 37/00; A61P 37/06; A61P 43/00; C07K 14/4747; G16B 35/00; G16C 20/60; G16C 20/64;** A61K 38/00

## Description

BACKGROUND

Programmed cell death or apoptosis is an essential physiological process for the normal development and homeostasis of multicellular organisms (Thompson, C. B. (1995) Apoptosis in the pathogenesis and treatment of disease, Science 267, 1456-1462.; Jacobson, M. D., Weil, M., and Raff, M. C. (1997) Programmed cell death in animal development, Cell 88, 347-354). Apoptosis further functions as a defense mechanism for controlling cell proliferation and for eliminating abnormal, misplaced, dysfunctional, or harmful cells. Deregulation of apoptosis can change the balance between cell proliferation and cell death, contributing to a wide variety of diseases characterized by too much or too little cell death such as in cancer (Adams, J. M., and Cory, S. (2007) The Bcl-2 apoptotic switch in cancer development and therapy, Oncogene 26, 1324-1337), autoimmunity (Krammer, P. H. (2000) CD95's deadly mission in the immune system, Nature 407, 789-795), neurodegenerative diseases (Yuan, J., and Yankner, B. A. (2000) Apoptosis in the nervous system, Nature 407, 802-809), and cardiovascular diseases (Kang, P. M., and Izumo, S. (2003) Apoptosis in heart: basic mechanisms and implications in cardiovascular diseases, Trends Mol Med 9, 177-182). Intensive investigation of the apoptotic signaling pathway over the last two decades has identified the BCL-2 protein family as a signaling network of pro-apoptotic and anti-apoptotic proteins whose interactions maintain the delicate balance between cellular life and death (Danial, N. N., and Korsmeyer, S. J. (2004) Cell death: critical control points, Cell 116, 205-219; Youle, R. J., and Strasser, A. (2008) The BCL-2 protein family: opposing activities that mediate cell death, Nat Rev Mol Cell Biol 9, 47-59). Biochemical and genetic studies have revealed a prominent role for the BCL-2 protein family in regulating the "point of no return" for apoptotic cell death.

BCL-2 family members are evolutionary conserved and include pro- and antiapoptotic members that regulate apoptosis through protein interactions (Youle, R. J., and Strasser, A. (2008) The BCL-2 protein family: opposing activities that mediate cell death, Nat Rev Mol Cell Biol 9, 47-59) (Figure 1). The anti-apoptotic proteins such as BCL-$X_L$ and BCL-2 protect against cell death by inhibiting pro-apoptotic proteins and share four BCL-2 homology domains (BH1-4) (Fig 2). Multidomain proapoptotic proteins such as BAX and BAK share three conserved domains (BH1-3) and, upon activation, inflict irreversible damage to the mitochondrion (Wei, M. C., Zong, W. X., Cheng, E. H., Lindsten, T., Panoutsakopoulou, V., Ross, A. J., Roth, K. A., MacGregor, G. R., Thompson, C. B., and Korsmeyer, S. J. (2001) Proapoptotic BAX and BAK: a requisite gateway to mitochondrial dysfunction and death, Science (New York, N.Y 292, 727-730; Green, D. R. (2005) Apoptotic pathways: ten minutes to dead, Cell 121, 671-674). A subgroup of pro-apoptotic proteins share only the conserved BH3 domain. These "BH3-only" pro-apoptotic proteins function as death messengers that are positioned throughout the cell, poised to transmit death signals to multidomain members under conditions of physiological stress or cellular injury (Letai, A., Bassik, M. C., Walensky, L. D., Sorcinelli, M. D., Weiler, S., and Korsmeyer, S. J. (2002) Distinct BH3 domains either sensitize or activate mitochondrial apoptosis, serving as prototype cancer therapeutics, Cancer Cell 2, 183-192; Chen, L., Willis, S. N., Wei, A., Smith, B. J., Fletcher, J. I., Hinds, M. G., Colman, P. M., Day, C. L., Adams, J. M., and Huang, D. C. (2005) Differential targeting of prosurvival Bcl-2 proteins by their BH3-only ligands allows complementary apoptotic function, Mol Cell 17, 393-403). Depending upon the nature of the apoptotic stimuli and the cellular context, the BH3-only protein's death signal will either be neutralized by anti-apoptotic proteins or delivered directly to the mitochondrial executioners BAX and BAK. BAX and BAK represent a gateway to cell death for inducing permeabilization of the outer mitochondrial membrane (Wei, M. C., Zong, W. X., Cheng, E. H., Lindsten, T., Panoutsakopoulou, V., Ross, A. J., Roth, K. A., MacGregor, G. R., Thompson, C. B., and Korsmeyer, S. J. (2001) Proapoptotic BAX and BAK: a requisite gateway to mitochondrial dysfunction and death, Science 292, 727-730). Once the outer mitochondrial membrane is permeabilized, a number of mitochondrial factors is released into the cytosol. One of these apoptogenic factors, cytochrome c, is critical component of a cytosolic complex termed the apoptosome (Riedl, S. J., and Salvesen, G. S. (2007) The apoptosome: signalling platform of cell death, Nat Rev Mol Cell Biol 8, 405-413), which activates caspase-9, leading to the irreversible execution of the death program (Li, P., Nijhawan, D., Budihardjo, I., Srinivasula, S. M., Ahmad, M., Alnemri, E. S., and Wang, X. (1997) Cytochrome c and dATP-dependent formation of Apaf-1/caspase-9 complex initiates an apoptotic protease cascade, Cell 91, 479-489; Luo, X., Budihardjo, I., Zou, H., Slaughter, C., and Wang, X. (1998) Bid, a Bcl2 interacting protein, mediates cytochrome c release from mitochondria in response to activation of cell surface death receptors, Cell 94, 481-490).

The discovery of the protein BCL-2 at the chromosomal breakpoint of t(14;18) lymphomas unveiled a strategy that cancer cells exploit to resist cell death, namely the overexpression of BCL-2 survival proteins and sequestration of the death executioner proteins BAX/BAK. BCL-2 family members operate at the crossroads of the cellular decision to live or die, and therefore, the development of targeted agents that modulate BCL-2 family protein activities may result in the capacity to therapeutically trigger or block cell death in diseases of unrestrained cell survival or premature cell death, respectively. We previously developed and applied a new technology termed "hydrocarbon stapling" to transform natural peptide segments of the BCL-2 family into pharmacologic entities, termed Stabilized Alpha-Helices of BCL-2 domains (SAHBs) that can selectively identify and target BCL-2 family members within cells (Walensky, L. D., Kung, A.

L., Escher, I., Malia, T. J., Barbuto, S., Wright, R. D., Wagner, G., Verdine, G. L., and Korsmeyer, S. J. (2004) Activation of apoptosis in vivo by a hydrocarbon-stapled BH3 helix, Science (New York, N.Y 305, 1466-1470). We developed SAHBs with unique biophysical properties, including dramatically enhanced α-helicity, proteolytic stability, cell permeability, and potent, selective target binding affinities. A discrete subset of the compounds demonstrated the distinctive capacity to bind to the essential mitochondrial executioner protein BAX(Walensky, L. D., Pitter, K., Morash, J., Oh, K. J., Barbuto, S., Fisher, J., Smith, E., Verdine, G. L., & Korsmeyer, S. J. (2006) Molecular Cell 24, 199-210). This discovery prompted us to explore the structural basis for the interaction between a stapled BIM BH3 peptide and BAX using NMR spectroscopy. In pursuing these studies, we identified for the first time an explicit binding site on BAX for its direct activation Gavathiotis, E., Suzuki, M., Davis, M. L., Pitter, K., Bird, G. H., Katz, S. G., Tu, H.-C., Kim, H., Cheng, E. H.-Y., Tjandra, N., Walensky, L.D. (2008) Nature*,* in press). The trigger mechanism for BAX activation has been a longstanding mystery of the cell death field and the subject of intense debate. The location of this interaction site on BAX was unanticipated and defines both a new interaction mechanism for BCL-2 family proteins and a novel therapeutic target for modulating cell death by direct BAX engagement. Whereas blockade of the novel site may effectively repress BAX-induced cell death, ligand engagement may trigger BAX-mediated apoptosis. Thus, our identification of a novel BAX activation site has important implications for the development of pharmacologic agents to respectively activate or inhibit apoptosis in human diseases characterized by unrestrained cell survival or pathologic cell death. Because BAX is only one of three known homologous pro-apoptotic multidomain BCL-2 family members, the implications of a direct trigger site for BAX may likewise extend to pro-apopototic BAK and BOK.

The document Anonymous "Hit2Lead | Search | Screening Compounds | Search by ID; 5258079; 3-methyl-1-(4-phenyl-1,3-thiazol-2-yl)-1H-pyrazole-4,5-dione 4-[(4-ethoxyphenyl)hydrazine]", 28 July 2005 (2005-07-28), XP55719284, as well as the document Pubchem: "SID 3315682 - PubChem", 28 July 2005 (2005-07-28), XP55719287, Retrieved from the Internet:URL:https://pubchem.ncbi.nlm.nih.gov/substance/3315682 [retrieved on 2020-07-30] provide technical information as regards compound 5258079. The involvement of said compound in cancer is neither taught nor suggested in said documents.

## SUMMARY OF THE INVENTION

The invention relates to a compound (Z)-4-(2-(4-ethoxyphenyl)hydrazono)-5-methyl-2-(4-phenylthiazol-2-yl)-2,4-dihydro-3H-pyrazol-3-one or a salt thereof for use in a method of treating cancer.

## BRIEF DESCRIPTION OF THE DRAWINGS

Figure 1 depicts how the three distinct classes of BCL-2 family proteins interact to regulate apoptosis.

Figure 2 displays a listing of select BCL-2 family members, highlighting their conserved BCL-2 homology (BH) domains.

Figure 3 depicts the BH3 binding pocket of anti-apoptotic BCL-2 family members.

Figure 4 illustrates the continuum of events that is initiated by direct activation of BAX, culminating in BAX-mediated mitochondrial damage.

Figure 5 demonstrates the location of the newly identified BH3 interaction site on BAX, as deteremined by NMR analysis. Importantly, BIM SAHB engages BAX at a structural location that is distinct from the canonical BH3 binding site identified for anti-apoptotic proteins and shown in Figure 3.

Figure 6 demonstrates the topography of the novel BH3 interaction site on BAX (part A) and the orientation of BIM BH3 at the BAX binding site (part B) as determined by NMR analysis of the BIM SAHB-BAX interaction

Figure 7 indicates the amino acid sequence of BAX (part A) with residues of the novel BH3 binding site on BAX highlighted. BAX residues engaged in BIM BH3 interactions are highlighted in ribbon (part B) and surface (part C) diagrams of BAX.

Figure 8 lists the sequences of BAX activator BH3 peptides and hydrocarbon stapled derivatives

Figure 9 shows how the identified molecules from a virtual screen of the novel interaction site decorate the horizontal

hydrophobic groove (part A), the superior juxta-loop region (part B), and the inferior juxta-loop region (part C).

Figure 10 indicates the amino acid sequence of BAX (part A) with those residues involved with ligand interactions at or adjacent to the novel BH3 binding site on BAX highlighted. BAX residues engaged in ligand interactions are highlighted in ribbon (part B) and surface (part C) diagrams of BAX.

Figure 11 demonstrates a competitive fluorescent polarization binding assay revealing that compounds identified by the virtual screen effectively and doseresponsively compete with FITC-BIM SAHB for BAX binding at the novel interaction site. The compound numbers presented in Figure 11 correspond to the compound numbers for the structures in Table 1.

Figure 12A demonstrates a BAX oligomerization assay involving the application of a direct BAX-activating compound, such as BIM SAHB, and monitoring the conversion of BAX from its monomeric to its oligomeric state by sizeexclusion chromatography (SEC). Figure 12B depicts how compounds identified by the virtual screen trigger BAX oligomerization as detected by this BAX oligomerization assay. The compound numbers presented in Figure 12B correspond to the compound numbers for the structures in Table 1.

Figure 13 depicts how compounds identified by the virtual screen trigger BAX oli-gomerization as detected by conversion of the BAX monomer to its oligomeric form using dynamic light scattering. The compound numbers presented in Figure 13 correspond to the compound numbers for the structures in Table 1.

Figure 14 depicts how compounds identified by the virtual screen trigger recombinant BAX-mediated cytochrome c release from *Bax*-/- *Bak*-/- mitochondria, as detected by ELISA assay. The compound numbers presented in Figure 14 correspond to the compound numbers for the structures in Table 1.

Figure 15 depicts how identified compounds 5285738 (part A) and 5258079 (part B) selectively trigger dose-responsive apoptosis in BAX-reconstituted *Bax*-/- *Bak*-/- mouse em-bryo fibroblasts (DKO MEF), but not in DKO MEFs. The compound numbers presented in Figure 15 correspond to the compound numbers for the structures in Table 1.

DETAILED DESCRIPTION

**I. *Definitions***

As used herein, the term, "BCL-2 family polypeptide" refers to an evolutionary conserved family of proteins having as few as one to as many as four conserved BCL-2 homology domains (BH1, BH2, BH3 and/or BH4). The BH domains are alpha-helical segments and are present in both the anti-apoptotic and proapoptotic polypeptides of the BCL-2 family. BCL-2 family polypeptides include BCL-2, BCL-XL, BCL-w, MCL-1, BCL-B, A1/BFL-1, BOO/DIVA, Nr-13, CED-9, BAX, BAK, BOK/MTD, BID, BAD, BIK/NBK, BLK, HRK, BIM/BOD, BNIP3, NIX, NOXA, PUMA, BMF, EGL-1, and viral homologues, including, but not limited to M11L and E1B-19K.

The term "active site" refers to a region of a BCL-2 family polypeptide, as a result of its shape, amino acid content, and charge potential, that favorably interacts or associates with another agent (including, without limitation, a protein, polypeptide, peptide, molecule, nucleic acid, compound, antibiotic or drug, or combination thereof) via various covalent and/or non-covalent binding forces. The "active site" includes a hydrophobic groove surrounded by a perimeter of charged and hydrophilic residues that is capable of binding a stabilized alpha helix of BCL-2 domain, such as human hydrocarbon-stapled BIM BH3 (SEQ ID NO:3), and which is formed by the juxtaposition of alpha helices 1 and 6 of BAX. In one embodiment, the active site includes two or more amino acids corresponding to Glu17, Met20, Lys21, Thr22, Ala24, Leu25, Leu27, Gln28, Gly29, Ile31, Gln 32, Leu47, Asp48, Pro49, Val50, Pro51, Gln52, Asp53, Thr56, Arg89, Phe92, Phe93, Pro130, Glul31, Ile 133, Arg 134, Thr135, Met137, Gly138, Trp139, Leu141, Asp142, Phe143, Arg145, Glu146 of SEQ ID NO:1.

The term "binding site" refers to a region of a BCL-2 family polypeptide, as a result of its shape, amino acid content, and charge potential, that favorably interacts or associates with another agent (including, without limitation, a protein, polypeptide, peptide, molecule, compound, antibiotic or drug) via various covalent and/or non-covalent binding forces. A "bidning site" includes one or more amino acids corresponding to Glu17, Gln18, Met20, Lys21, Thr22, Ala24, Leu25, Leu27, Gln28, Gly29, Ile31, Gln 32, Asp33, Arg34, Ala35, Gly36, Arg37, Met38, Gly39, Gly40, Glu41, Ala 42, Leu47, Asp48, Pro49, Val50, Pro51, Gln52, Asp53, Ala54, Ser55, Thr56, Lys57, Lys58, Leu59, Ser60, Glu61, Lys64, Arg89, Phe92, Phe93, Leu122, Leu125, Thr127, Lys128, Val129, Pro130, Glu131, Leu132, Ile 133, Arg 134, Thr135, Met137, Gly138, Trp139, Leu141, Asp142, Phel43, Arg145, Glu146, Arg 147, Leu149, Gly150, Gly156, Gly157, Trp158 Asp 159, Leu161, Leu162 of SEQ ID NO:1.

The term "BCL-2 family polypeptide variant" refers to polypeptides that vary from a reference BCL-2 family polypeptide by the addition, deletion or substitution of at least one amino acid. It is well understood in the art that some amino acids may be changed to others with broadly similar properties without changing the nature of the activity of the polypeptide (conservative substitutions) as described hereinafter. Accordingly, BCL-2 family polypeptide variants as used herein encompass polypeptides that have pro- or anti-apoptotic activity. The term "variant" refers to a protein having at least 30% amino acid sequence identity with a reference BCL-2 homology domain within a protein or any other functional domain thereof. More specifically, the term "variant" includes, but is not limited to, a BCL-2 family polypeptide comprising either 1) an active site characterized by a three dimensional structure comprising the relative structural coordinates of at least two BAX amino acid residues corresponding to Glu17, Met20, Lys21, Thr22, Ala24, Leu25, Leu27, Gln28, Gly29, Ile31, Gln 32, Leu47, Asp48, Pro49, Val50, Pro51, Gln52, Asp53, Thr56, Arg89, Phe92, Phe93, Pro130, Glu131, Ile 133, Arg 134, Thr135, Met137, Gly138, Trp139, Leu141, Asp142, Phe143, Arg145, Glul46 of SEQ ID NO:1 or 2) a binding site characterized by a three dimensional structure comprising the relative structural coordinates of at least one BAX amino acid residues corresponding to Glu17, Gln18, Met20, Lys21, Thr22, Ala24, Leu25, Leu27, Gln28, Gly29, Ile31, Gln 32, Asp33, Arg34, Ala35, Gly36, Arg37, Met38, Gly39, Gly40, Glu41, Ala 42, Leu47, Asp48, Pro49, Val50, Pro51, Gln52, Asp53, Ala54, Ser55, Thr56, Lys57, Lys58, Leu59, Ser60, Glu61, Lys64, Arg89, Phe92, Phe93, Leu122, Leu125, Thr127, Lys128, Val129, Pro130, Glu131, Leu132, Ile 133, Arg 134, Thr135, Met137, Gly138, Trp139, Leu141, Asp142, Phe143, Arg145, Glu146, Arg 147, Leu149, Gly150, Gly156, Glyl57, Trpl58 Asp 159, Leu161, Leu 162 of SEQ ID NO:1, in each case, +/-a root mean square deviation from the conserved backbone atoms of those residues of not more than 1.1 angstroms, more preferably not more than 1.0 angstroms, and most preferably not more than 0.5 angstroms.

A "BCL-2 family polypeptide variant" further includes those polypeptides, or their biologically active fragments, that comprise an amino acid sequence which is at least 30%, 40%, 50%, 60%, 70%, 80%, 90%, 95%, 96%, 97%, 98%, 99% or more similar to an amino acid sequence of a BCL-2 homology domain (e.g., BH3 domain).

As used herein, the term "horizontal hydrophbic groove with or without a perimeter of charged and hydrophilic residues" refers to that region of the ligand interaction on BAX that includes all or part of the BIM BH3 interaction site on BAX, as depicted in Figures 6 and 8A.

As used herein, the term "superior juxta-loop" refers to that portion of the ligand interaction site that encompasses those residues located adjacent to the $\alpha$1-$\alpha$2 loop and extending from the midpoint of the horizontal hydrophobic groove upward, as depicted in Figure 8B.

As used herein, the term "inferior juxta-loop" refers to that portion of the ligand interaction site that encompasses those residues located adjacent to the $\alpha$1-$\alpha$2 loop and extending from the midpoint of the horizontal hydrophobic groove downward, as depicted in Figure 8B.

As used herein, the term "loop between $\alpha$1-$\alpha$2" refers to those residues located between $\alpha$-helix 1 and $\alpha$ -helix 2 of BAX..

The term "hydrophobic patch" refers to the portion of the active site that binds a hydrophobic moiety. In one embodiment, the hydrophobic patch contains 1, 2, 3 or more hydrophobic amino acid residues. In one particular embodiment, the hydrophobic pocket contains amino acid residues corresponding to Met20, Ala24, Leu25, Leu27, Gly29, Ile31, Leu47, Val50, Phe92, Phe93, Ile 133, Arg 134, Met137, Gly138, Trp139, Leu141, Phe143 of SEQ ID NO:1.

The term "charged/hydrophilic patch" refers to the portion of the active site that binds a charged or hydrophilic moiety. In one embodiment, the charged/hydrophilic patch contains 1, 2, 3 or more charged or hydrophilic amino acid residues. In one particular embodiment, the charged/hydrophilic patch contains amino acid residues corresponding to Glul7, Lys21, Thr22, Gln28, Gln 32, Asp 33, Asp48, Gln52, Asp53, Thr56, Arg89, Glu131, Arg 134, Thr135, Asp142, Arg145, Glu146, Arg 147 of SEQ ID NO:1.

The term "hydrophobic amino acid" means any natural or non-natural amino acid or mimetic thereof having an uncharged, non-polar side chain that is relatively insoluble in water. Examples of naturally occurring hydrophobic amino acids are alanine, leucine, isoleucine, valine, proline, phenylalanine, tryptophan and methionine.

The term "hydrophilic amino acid" means any natural or non-natural amino acid or mimetic thereof having an uncharged, polar side chain that is relatively soluble in water. Examples of naturally occurring hydrophilic amino acids are serine, threonine, tyrosine, asparagine, glutamine, and cysteine.

The term "negatively charged amino acid" includes any naturally occurring or unnatural amino acid or mimetic thereof having a negatively charged side chain under normal physiological conditions. Examples of negatively charged naturally occurring amino acids are aspartic acid and glutamic acid.

The term "positively charged amino acid" includes any naturally occurring or unnatural amino acid or mimetic thereof having a positively charged side chain under normal physiological conditions. Examples of positively charged naturally occurring amino acids are arginine, lysine and histidine.

The term "anti-apoptotic polypeptide" refers to BCL-2 family polypeptides characterized by having one or more amino acid homology domains, BH1, BH2, BH3, and/or BH4, and that promote cell survival by attenuating or inhibiting apoptosis. The "anti-apoptotic polypeptides" further include those proteins, or their biologically active fragments, that

are at least 30%, 40%, 50%, 60%, 70%, 80%, 90%, 95%, 96%, 97%, 98%, 99% or more similar in amino acid sequence to an antiapoptotic BCL-2 homology domain within a BCL-2 family polypeptide. In a preferred embodiment, the BCL-2 homology domain comprises one or more conserved amino acid residues, such as amino acid residues corresponding to Leu 97, Gly 101 and Asp 102 of Bcl-2 (SEQ ID NO:8): Anti-apoptotic polypeptides include but are not limited to BCL-2, BCL-XL, BCL-w, MCL-1, A1/BFL-1, BCL-B, BOO/DIVA, Nr-13 or CED-9.

The term "pro-apoptotic polypeptide" refers to BCL-2 family polypeptides characterized by having one or more amino acid homology domains, BH1, BH2, and/or BH3, and that promote cell death by activating apoptosis. The "pro-apoptotic polypeptides" further include those proteins, or their biologically active fragments, that are at least 30%, 40%, 50%, 60%, 70%, 80%, 90%, 95%, 96%, 97%, 98%, 99% or more similar in amino acid sequence to a pro-apoptotic BCL-2 homology domain within a BCL-2 family polypeptide. In a preferred embodiment, the BCL-2 homology domain comprises one or more conserved amino acid residue, such as amino acid residues corresponding to Leu 92, Gly 96 and Asp 97 of BAX (SEQ ID NO: 1). Proapoptotic polypeptides include but are not limited to BAX, BAK, BOK/MTD, BID BAD, BIK/NBK, BLK, HRK, BIM/BOD, BNIP3, NIX, NOXA, PUMA, BMF AND EGL-1.

As used herein, the term "apoptosis" refers to a regulated network of biochemical events which lead to a selective form of cell death that is characterized by readily observable morphological and biochemical changes, such as the fragmentation of the deoxyribonucleic acid (DNA), condensation of the chromatin, which may or may not be associated with endonuclease activity, chromosome migration, margination in cell nuclei, the formation of apoptotic bodies, mitochondrial swelling, widening of the mitochondrial cristae, opening of the mitochondrial permeability transition pores and/or dissipation of the mitochondrial proton gradient.

The term "compound" is used herein to denote a chemical agent, polypeptide, nucleic acid or combination thereof, or a mixture or synthetic combination of chemical compounds and/or polypeptides and/or nucleic acids (e.g. DNA and/or RNA derivative), salts and solvates thereof, and the like. Preferably, a compound of the invention binds to the active site of a BCL-2 family polypeptide. A "modulator" is a compound which modulates the activity of a BCL-2 family polypeptide.

The term "candidate compound" is used herein to denote a chemical compound, polypeptide, nucleic acid or combination thereof, or a mixture or synthetic combination of chemical compounds and/or polypeptides and/or nucleic acids, salts and solvates thereof, and the like, which is tested by a method of the invention and is found to bind to active site of a BCL-2 family polypeptide, and thus is believed to modulate the activity of the BCL-2 family polypeptide.

The term "modulate" as used herein with reference to a compound refers to the activation or inhibition of anti-apoptotic or pro-apoptotic activity of a BCL-2 family polypeptide or other protein-protein interaction involving a BCL-2 family member that regulates a biochemical pathway (e.g. unfolded protein response, glucose-stimulated insulin secretion). Methods for assaying both anti-apoptotic, pro-apoptotic, and other biochemical activities (e.g. unfolded protein response, glucose-stimulated insulin secretion) are well known in the art and described herein.

As used herein, the term "interacts" or "binds" refers to a condition of proximity between a compound, or portions thereof, and the active site of a BCL-2 family polypeptide or portions thereof. The interaction is between one or more moieties on the compound and one or more moieties on amino acids of the active site region. The association may be non-covalent--wherein the juxtaposition is energetically favored by hydrogen bonding or van der Waals or electrostatic interactions--or it may be covalent.

The term, "activates" refers to an increase in the anti-apoptotic or proapoptotic activity of a BCL-2 family polypeptide or other defined biochemical activity based upon protein-protein interaction. A compound that activates a proapoptotic activity will bind to an active site of a BCL-2 family polypeptide and cause a 1.5x, 2x, 3x, 4x, 5x, 6x, 7x, 8x, 9x, 10x, 15x, 20x or more increase in the proapoptotic activity of the BCL-2 family polypeptide when compared with a control lacking the compound. In another embodiment, a compound that activates an antiapoptotic activity will bind to an active site of a BCL-2 family polypeptide and cause a 1.5x, 2x, 3x, 4x, 5x, 6x, 7x, 8x, 9x, 10×, 15x, 20x or more increase in the antiapoptotic (survival) activity of the BCL-2 family polypeptide when compared with a control lacking the compound. Assays for assessing the activation of an antiapoptotic or pro-apoptotic activity are known in the art and described herein.

The term, "inhibits" refers to a decrease or blocking of the anti-apoptotic or pro-apoptotic activity of a BCL-2 family polypeptide, or other defined biochemical activity based upon protein-protein interaction. For example, a compound that inhibits a pro-apoptotic activity will bind to an active site of a BCL-2 family polypeptide and prevent activation or reduce the activity of the BCL-2 family polypeptide. Thus, the compound will inhibit or decrease the effects of a proapoptotic activity. Thus, pro-apoptotic activity, e.g., cell death, will be less than 75%, 70%, 60%, 50%, 40%, 30%, 20%, 10%, 5% or less in a population of cells in which an inhibitor is present than compared to a control cell population where the compound is not present.

A compound that inhibits an anti-apoptotic activity will bind to an active site or binding site of a BCL-2 family polypeptide and prevent or inhibit anti-apoptotic activity. Thus, anti-apoptotic activity, e.g., cell survival, will be less than 75%, 70%, 60%, 50%, 40%, 30%, 20%, 10%, 5% or less in a population of cells in which the inhibitor is present than compared to a control cell population where the compound is not present.

As used herein, the term "BH3 SAHB" refers to the BCL-2 homology domain 3 of a BCL-2 family polypeptide that has been hydrocarbon stapled so as to form a stabilized alpha helix. The amino acid sequence of numerous BH3 domains are described herein. Methods of making BH3 SAHBs are known in the art and described in U.S. Patent Publication No. US2005/0250680, filed November 5.

As used herein, the term "BIM BH3 polypeptide" refers to a polypeptide having a BCL-2 homology domain 3 of BIM. In one embodiment, the BIM BH3 polypeptide has an amino acid sequence which is 30%, 40%, 50%, 60%, 70%, 80%, 90%, 95%, 96%, 97%, 98%, 99% or more identical to SEQ ID NO:3 (Figure 8) and includes one or more of amino acid residues corresponding to Leu152, Glyl56, and Asp157 of SEQ ID NO:2 or conservative substitutions thereof. In a preferred embodiment, the BIM BH3 polypeptide has the amino acid sequence of SEQ ID NO:3 or SAHB derivatives thereof (Figure 8).

As used herein, the term "BID BH3 polypeptide" refers to a polypeptide having a BCL-2 homology domain 3 of BID. In one embodiment, the BID BH3 polypeptide has an amino acid sequence which is 30%, 40%, 50%, 60%, 70%, 80%, 90%, 95%, 96%, 97%, 98%, 99% or more identical to SEQ ID NO: 5 (Figure 8) and includes one or more of amino acid residues corresponding to Leu90, Gly94, and Asp95 of SEQ ID NO:4 or conservative substitutions thereof. In a preferred embodiment, the BID BH3 polypeptide has the amino acid sequence of SEQ ID NO:5 or SAHB derivatives thereof (Figure 8).

As used herein, the term "PUMA BH3 polypeptide" refers to a polypeptide having a BCL-2 homology domain 3 of PUMA. In one embodiment the PUMA BH3 polypeptide has an amino acid sequence which is 30%, 40%, 50%, 60%, 70%, 80%, 90%, 95%, 96%, 97%, 98%, 99% or more identical to SEQ ID NO: 7 (Figure 8) and includes one or more of amino acid residues corresponding to Leu 141, Ala 145 and Asp 146 of SEQ ID NO:6 or conservative substitutions thereof. In a preferred embodiment, the PUMA BH3 polypeptide has the amino acid sequence of SEQ ID NO:7 or SAHB derivatives thereof (Figure 8).

As used herein, the term "BAX BH3 polypeptide" refers to a polypeptide having a BCL-2 homology domain 3 of BAX. In one embodiment, the BAX BH3 polypeptide has an amino acid sequence which is 30%, 40%, 50%, 60%, 70%, 80%, 90%, 95%, 96%, 97%, 98%, 99% or more identical to SEQ ID NO: 8 (Figure 8) and includes one or more of amino acid residues corresponding to Leu 63, Gly 67 and Asp 68 of SEQ ID NO:1 or conservative substitutions thereof. In a preferred embodiment, the BAX BH3 polypeptide has the amino acid sequence of SEQ ID NO:8 or SAHB derivatives thereof (Figure 8).

As used herein, the term "BAX activator BH3 consensus polypeptide" refers to a polypeptide containing a consensus sequence for BAX binding at the new interaction site. In one embodiment, the BAX activator BH3 consensus polypeptide has an amino acid sequence which is 30%, 40%, 50%, 60%, 70%, 80%, 90%, 95%, 96%, 97%, 98%, 99% or more identical to SEQ ID NO: 9 (Figure 8) or SAHB derivatives thereof.

The terms "pharmacologically effective amount," "therapeutically effective amount", "pharmacologically effective dose" or simply "effective amount" refers to that amount of an agent effective to produce the intended pharmacological, therapeutic or preventive result. The pharmacologically effective amount results in the amelioration of one or more symptoms of a disorder, or prevents the advancement of a disorder, or causes the regression of the disorder. For example, with respect to the treatment of a disorder or excessive cellular survival or proliferation, a therapeutically effective amount preferably refers to the amount of a therapeutic agent that decreases the rate of tumor growth, decreases tumor mass, decreases the number of metastases, increases time to tumor progression, or increases survival time by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. For example, with respect to the treatment of a disorder associated with increased cellular death, e.g., ischemia, a therapeutically effective amount preferably refers to the amount of a therapeutic agent that prevents or limits tissue and/or cellular damage that would otherwise occur if treatment was not administered. The therapeutic agent decreases tissue and/or cellular damage by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% compared to damage that occurs without the administration of a therapeutic agent of the invention.

The terms "treat," and "treating," as used herein with reference to a disorder (e.g., hyperpoliferative disorder, excessive cellular survival or proliferation), refers to a decrease in the occurrence of pathological cells (e.g., hyperproliferative or neoplastic cells) in an animal. The prevention may be complete, e.g., the total absence of pathological cells in a subject. The prevention may also be partial, such that the occurrence of pathological cells in a subject is less than that which would have occurred without the present invention. In some embodiments, such terms refer to one, two, three or more results following the administration of one or more therapies: (1) a stabilization, reduction or elimination of the cancer cell population, (2) an increase in the length of remission, (3) a decrease in the recurrence rate of cancer, (4) an increase in the time to recurrence of cancer, and (6) an increase in the survival of the patient.

The terms "treat," and "treating," as used herein with reference to a disorder associated with increased cellular death, e.g., ischemia, refer to a decrease in the occurrence of tissue and/or cellular damage in an animal. The prevention

may be complete, e.g., the total absence of tissue damage in a subject. The prevention may also be partial, such that the occurrence of tissue damage in a subject is less than that which would have occurred without the therapeutic agent.

As used herein, a "BCL-2 associated disorder", refers to a disorder associated with a deregulated BCL-2 family member. BCL-2 associated disorders are associated with excessive cellular survival and/or proliferation, e.g., cancer, or excessive cellular death, e.g., Alzheimer's disease. BCL-2 associated disorders include those described herein.

As used herein, a "hyperproliferative disorder" means cancer, neoplastic growth, hyperplastic or proliferative growth or a pathological state of abnormal cellular development or survival and includes solid tumors, non-solid tumors, and any abnormal cellular proliferation or accumulation, such as that seen in leukemia.

The terms "anticancer agent" and "anticancer drug," as used herein, refer to any therapeutic agents (e.g., chemotherapeutic compounds and/or molecular therapeutic compounds), antisense therapies, nucleic acid therapies (e.g. RNAi), radiation therapies, used in the treatment of hyperproliferative diseases such as cancer. In one embodiment, the invention is directed to methods of treating a BCL-2 associated disorder comprising administering an effective dose of an anticancer agent and a compound which binds to the active site, as described herein, of a BCL-2 family peptide.

As used herein, the term "structural coordinates" refers to Cartesian coordinates corresponding to an atom's spatial relationship to other atoms in a molecule or molecular complex. Structural coordinates may be obtained using x-ray crystallography techniques or NMR techniques, or may be derived using molecular replacement analysis or homology modeling. Various software programs allow for the graphical representation of a set of structural coordinates to obtain a three dimensional representation of a molecule or molecular complex. Structural coordinates for the BCL-2 family members are known in the art and are publicly available.

The term "interaction template" refers to a three dimensional model built using Cartesian coordinates corresponding to an atom's spatial relationship to other atoms in a molecule or molecular complex. Structural coordinates may be obtained using x-ray crystallography techniques or NMR techniques, or may be derived using molecular replacement analysis or homology modeling. Various software programs allow for the graphical representation of a set of structural coordinates to obtain a three dimensional representation of a molecule or molecular complex. The structural coordinates of BCL-2 family polypeptides are known in the art and can be found for example at Protein Data Bank ("PDB") (Research Collaboratory for Structural Bioinformatics; http://www. rcsb.org). For example, known BCL-2 family structural coordinates include BAX (PDB ID No. 1f16), BAK (PDB ID No. 2ims), BCL-2 (PDB ID No. 1g5m), BIM (PDB ID No. 2pqk) and BCL-XL (PDB ID No. 1lxl), in addition to those disclosed here: BIM BH3-BAX (PDB ID No. 2k7w), as well as others known in the art.

Preferably, the interaction template is of a BAX polypeptide having the amino acids sequence set forth in SEQ ID NO:1, wherein the active site of the BAX polypeptide is accessible to solvent and available for interaction with modulators, e.g., activators. This three-dimensional form of BAX is used to facilitate the identification of compounds which bind in the active site. As used herein, the "interaction template" includes templates created by comparing the sequence/structual alignment of BAX to other BCL-2 family polypeptides. Identification of conserved and non-conserved residues allows a skilled artisan to identify a corresponding active site in other BCL-2 family polypeptides and design/screen modulators of the polypeptide.

As used herein in relation to the position of an amino acid, e.g., Ala 149 of SEQ ID NO:1, the term "corresponding to" refers to an amino acid in a first polypeptide sequence, e.g., BAX, that aligns with a given amino acid in a reference polypeptide sequence, e.g., BAK, when the first polypeptide and reference polypeptide sequences are aligned by homology or other algorithms (e.g., structural comparison). Alignment is performed by one of skill in the art using software designed for this purpose, for example, BLASTP version 2.2.2 with the default parameters for that version. Corresponding amino acids can also be identified upon structural comparisons of a first polypeptide sequence and a second polypeptide sequence. Such structural comparisons are known in the art and described herein. For example, Petros et al. Biochimica et Biophysica Acta 1644; 83-94 (2004) and Suzuki et al., Cell. 103; 645-654 (2000) illustrated structural alignments between BCL-2 homology domains of BCL-2 family members.

The term "amino acid" refers to a molecule containing both an amino group and a carboxyl group. Suitable amino acids include, without limitation, both the Dand L-isomers of the 20 common naturally occurring amino acids found in peptides *(e.g.,* A, R, N, C, D, Q, E, G, H, I, L, K, M, F, P, S, T, W, Y, V (as known by the one letter abbreviations)) as well as the naturally occurring and unnaturally occurring amino acids prepared by organic synthesis or other metabolic routes.

A "non-essential" amino acid residue is a residue that can be altered from the wild-type sequence of a polypeptide (e.g., BIM BH3) without abolishing or substantially altering its BAX binding ability. An "essential" amino acid residue is a residue that, when altered from the wild-type sequence of the polypeptide, results in abolishing or substantially reducing the polypeptide's binding activity to a BAX active site or binding site. The essential and non-essential amino acid residues of the BH3 domains can readily be determined by methods well known in the art and described herein. The term "essential" amino acid residue as used herein, includes conservative substitutions of the essential amino acid. Generally, the "essential" amino acid residues are found at the interacting face (residues interacting with BAX) of the BH3 polypeptide.

A "conservative amino acid substitution" is one in which the amino acid residue is replaced with an amino acid

residue having a similar side chain or chemical mimetic thereof. For example, families of amino acid residues having similar side chains have been defined in the art. These families include amino acids with basic side chains *(e.g.,* lysine, arginine, histidine), acidic side chains *(e.g.,* aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains *(e.g.,* threonine, valine, isoleucine) and aromatic side chains (*e.g.,* tyrosine, phenylalanine, tryptophan, histidine). Other conserved amino acid substitutions can also occur across amino acid side chain families, such as when substituting an asparagine for aspartic acid in order to modify the charge of a peptide. Thus, a predicted nonessential amino acid residue in a BH3 domain polypeptide, for example, is preferably replaced with another amino acid residue from the same side chain family or homologues across families (e.g. asparagines for aspartic acid, glutamine for glutamic acid). In addition, individual substitutions, deletions or additions that alter, add or delete a single amino acid or a small percentage of amino acids in an encoded sequence are also considered "conservative substitutions".

The terms "identical" or "percent identity," in the context of two or more nucleic acids or polypeptide sequences, refer to two or more sequences or subsequences that are the same or have a specified percentage of nucleotides or amino acids that are the same, when compared and aligned for maximum correspondence, as measured using one of the following sequence comparison algorithms, or by visual inspection.

"Similarity" or "percent similarity" in the context of two or more polypeptide sequences, refer to two or more sequences or subsequences that are the same or have a specified percentage of amino acid residues, or conservative substitutions thereof, that are the same when compared and aligned for maximum correspondence, as measured using one of the following sequence comparison algorithms, or by visual inspection.

By way of example, a first protein region can be considered similar to a region of an anti-apoptotic BCL-2 family member protein when the amino acid sequence of the first region is at least 30%, 40%, 50%, 60%, 70%, 75%, 80%, 90%, or even 95% identical, or conservatively substituted, to a region of the second anti-apoptotic BCL-2 family member protein when compared to any sequence of an equal number of amino acids as the number contained in the first region, or when compared to an alignment of anti-apoptotic BCL-2 family member proteins that has been aligned by a computer similarity program known in the art, as discussed below. Preferably, the polypeptide region of the first protein and the second protein includes one or more conserved amino acid residues.

## II. ***Description***

The invention provides a compound (Z)-4-(2-(4-ethoxyphenyl)hydrazono)-5-methyl-2-(4-phenylthiazol-2-yl)-2,4-dihydro-3H-pyrazol-3-one or a salt thereof for use in a method of treating cancer.

In a further embodiment, said cancer is selected from the group consisting of solid tumor, leukemia, and lymphoma. In a further embodiment, said cancer is a chemoresistant cancer. In another further embodiment, said chemoresistant cancer is resistant to ABT-737, ABT-263, obatoclax, or other BCL-2 survival protein inhibitors.

## III. ***Structural insights into BCL-2 family function***

The BCL-2 family of proteins includes both pro- and anti-apoptotic polypeptides that provide the checks and balances that govern susceptibility to cell death. Deregulation of this pathway has been documented in the pathogenesis of a wide spectrum of human diseases, including many cancers.

Members of the evolutionarily conserved BCL-2 family are important regulators of apoptotic cell death and survival. The proteins BCL-2, BCL-XL, Bcl-w, BFL1/A1 and MCL-1 are death antagonists while BAX, BAK, BAD, BCL-XS, BID, BIM, and BIK are examples of death agonists (Kroemer et al., Nature Med. 6:614 20 (1997)).

The BCL-2 family is defined by the presence of up to four conserved "BCL-2 homology" (BH) domains designated BH1, BH2, BH3, and BH4, all of which include alpha-helical segments (Chittenden et al. 1995 EMBO 14:5589; Wang et al. 1996 Genes Dev. 10:2859). Anti-apoptotic proteins, such as BCL-2 and BCL-XL, display sequence conservation in all BH domains. Pro-apoptotic proteins are divided into "multidomain" members (e.g. BAK, BAX, BOK), which possess homology in the BH1, BH2, and BH3 domains, and the "BH3-domain only" members (e.g. BID, BAD, BIM, BIK, NOXA, PUMA), that contain sequence homology exclusively in the BH3 amphipathic alpha-helical segment. BCL-2 family members have the capacity to form homo- and heterodimers, suggesting that competitive binding and the ratio between pro- and anti-apoptotic protein levels dictates susceptibility to death stimuli. Antiapoptotic proteins function to protect cells from pro-apoptotic excess, i.e., excessive programmed cell death. In certain cell types, death signals received at the plasma membrane trigger apoptosis via a mitochondrial pathway. The mitochondrial apoptotic pathway can also be activated by internal cellular stresses and signaling pathways. The mitochondria can serve as a gatekeeper of cell death by sequestering cytochrome c, a critical component of a cytosolic complex which activates caspase 9, leading to fatal downstream proteolytic events. Multidomain proteins such as BCL-2/BCL-XL and BAK/BAX play dueling roles of guardian and executioner at the mitochondrial membrane, with their activities further regulated by upstream BH3-only members

of the BCL-2 family. For example, BID is a member of the "BH3-domain only" subset of pro-apoptotic proteins, and transmits death signals received at the plasma membrane to effector pro-apoptotic proteins at the mitochondrial membrane. Select BH3-only members, such as BID and BIM, have been termed "activators" (Letai, A., et al. Cancer Cell 2, 183-192 (2002)), and have the unique capability of interacting with both pro- and anti-apoptotic proteins (Walensky Mol Cell 2006). Upon caspase 8 activation, BID is cleaved and the truncated adduct, tBID, triggers cytochrome c release and mitochondrial apoptosis through engagement of BCL-2 family proteins.

Deletion and mutagenesis studies determined that the amphipathic alpha-helical BH3 segment of pro-apoptotic family members functions as a death domain and thus represents a critical structural motif for interacting with multidomain apoptotic proteins. Structural studies have demonstrated that the BH3 helix interacts with anti-apoptotic proteins by inserting into a hydrophobic groove formed by the interface of BH1, 2 and 3 domains. tBID and BIM can be bound and sequestered by anti-apoptotic proteins (e.g., BCL-2 and BCL-$X_L$) and can trigger activation, indirectly or directly, of the pro-apoptotic proteins BAX and BAK, leading to cytochrome c release and a mitochondrial apoptosis program.

BCL-2-related ovarian killer (BOK) is the third member of the pro-apoptotic multidomain subgroup and is also bound by activator SAHB ligands, such as BID and BIM SAHBs. BOK was cloned from an ovarian cDNA library and found to be highly expressed in ovary, uterus, and testis. BOK mRNA species have since been identified in a broader distribution of tissues, including heart, spleen, liver, colon, lung, intestine, thyroid gland, adrenal, pancreas, and bone marrow, and select cancer cell lines.

A major breakthrough in BCL-2 biology was achieved by Fesik and co-workers at Abbott Laboratories a decade ago when the first X-ray and NMR structure of a BCL-2 family protein was reported (Muchmore, S. W., Sattler, M., Liang, H., Meadows, R. P., Harlan, J. E., Yoon, H. S., Nettesheim, D., Chang, B. S., Thompson, C. B., Wong, S. L., Ng, S. L., and Fesik, S. W. (1996) X-ray and NMR structure of human Bcl-xL, an inhibitor of programmed cell death, Nature 381, 335-341). The structure of BCL-$X_L$ is comprised of eight $\alpha$-helices, two of which, (helices 5 and 6) form a central hydrophobic core reminescent of the membrane insertion domains of the diphtheria and colicin pore-forming toxins (Muchmore, S. W., Sattler, M., Liang, H., Meadows, R. P., Harlan, J. E., Yoon, H. S., Nettesheim, D., Chang, B. S., Thompson, C. B., Wong, S. L., Ng, S. L., and Fesik, S. W. (1996) X-ray and NMR structure of human Bcl-xL, an inhibitor of programmed cell death, Nature 381, 335-341). This structural homology provided the basis for studies that demonstrated the capacity of BCL-2 family members to form pores in liposomal and mitochondrial systems (Nouraini, S., Six, E., Matsuyama, S., Krajewski, S., and Reed, J. C. (2000) The putative pore-forming domain of Bax regulates mitochondrial localization and interaction with Bcl-X(L), Mol Cell Biol 20, 1604-1615; Narita, M., Shimizu, S., Ito, T., Chittenden, T., Lutz, R. J., Matsuda, H., and Tsujimoto, Y. (1998) Bax interacts with the permeability transition pore to induce permeability transition and cytochrome c release in isolated mitochondria, Proc Natl Acad Sci U S A 95, 14681-14686). Subsequently, the structure of a BAK BH3 peptide in complex with BCL-$X_L$ revealed a paradigm for protein-protein interactions among pro- and anti-apoptotic BCL-2 family members: the pro-apoptotic $\alpha$-helical BH3 domain inserts into a hydrophobic groove formed by the juxtaposition of the BH1-3 domains (BH1: portions of helices $\alpha 4$ - $\alpha 5$; BH2: $\alpha 7$- $\alpha 8$; BH3: $\alpha 2$) of the anti-apoptotic protein (Figure 3). This structural paradigm was confirmed in subsequent NMR structures of BCL-2 (Petros, A. M., Nettesheim, D. G., Wang, Y., Olejniczak, E. T., Meadows, R. P., Mack, J., Swift, K., Matayoshi, E. D., Zhang, H., Thompson, C. B., and Fesik, S. W. (2000) Rationale for Bcl-xL/Bad peptide complex formation from structure, mutagenesis, and biophysical studies, Protein Sci 9, 2528-2534), BCL-w (Denisov, A. Y., Chen, G., Sprules, T., Moldoveanu, T., Beauparlant, P., and Gehring, K. (2006) Structural model of the BCL-w-BID peptide complex and its interactions with phospholipid micelles, Biochemistry 45, 2250-2256), MCL-1 (Day, C. L., Chen, L., Richardson, S. J., Harrison, P. J., Huang, D. C., and Hinds, M. G. (2005) Solution structure of prosurvival Mcl-1 and characterization of its binding by proapoptotic BH3-only ligands, J Biol Chem 280, 4738-4744), and BFL-1/A1 (Smits, C., Czabotar, P. E., Hinds, M. G., and Day, C. L. (2008) Structural plasticity underpins promiscuous binding of the prosurvival protein Al, Structure 16, 818-829) in complex with BH3-only peptides, suggesting that communication among BCL-2 proteins is mediated by the network of homo- and hetero-complexes formed between hydrophobic grooves and BH3 death helices. Preferences for hetero-associations are dictated by discrete differences in the amino acid composition of anti-apoptotic grooves and the BH3 peptides of binding partners. The structural determinations of anti-apoptotic complexes with BH3 peptides provided critical mechanistic insights into the function of BCL-2 family members and have led to the development of promising pharmacologic modulators of BCL-2 regulated apoptotic pathways (Walensky, L. D., et al. (2004) Activation of apoptosis in vivo by a hydrocarbon-stapled BH3 helix, Science (New York, N.Y 305, 1466-1470; Oltersdorf, T., et al. (2005) An inhibitor of Bcl-2 family proteins induces regression of solid tumours, Nature 435, 677-681; Nguyen, M., et al. (2007) Small molecule obatoclax (GX15-070) antagonizes MCL-1 and overcomes MCL-1-mediated resistance to apoptosis, Proc Natl Acad Sci USA 104, 19512-195). For example, selective BCL-2 inhibitors like the small molecule ABT-737 target the hydrophobic groove of anti-apoptotic BCL-2/BCL-$X_L$ and reactivate apoptosis in select tumors (Oltersdorf, T., et al. (2005) An inhibitor of Bcl-2 family proteins induces regression of solid tumours, Nature 435, 677-681).

Strikingly, the NMR structures of pro-apoptotic BAX (Suzuki, M., Youle, R. J., and Tjandra, N. (2000) Structure of Bax: coregulation of dimer formation and intracellular localization, Cell 103, 645-654) and BH3-only BID (McDonnell, J. M., et al.(1999) Solution structure of the proapoptotic molecule BID: a structural basis for apoptotic agonists and

antagonists, Cell 96, 625-634; Chou, J. J., et al. (1999) Solution structure of BID, an intracellular amplifier of apoptotic signaling, Cell 96, 615-624) revealed structural similarities with the anti-apoptotic proteins. BAX and BID likewise possess two central core helices that are surrounded by 6 or 7 amphipathic helices, respectively. Another critical structural similarity was revealed at the N-terminal regions of BAX and BID. An unstructured loop between $\alpha 1$ and $\alpha 2$ (BH3) is also present in select anti-apoptotic proteins such as BCL-2 (Petros, A. M., et al. (2001) Solution structure of the antiapoptotic protein bcl-2, Proc Natl Acad Sci USA 98, 3012-3017) and BCL- $X_L$ (Muchmore, S. W., et al. (1996) X-ray and NMR structure of human Bcl-xL, an inhibitor of programmed cell death, Nature 381, 335-341). The loop regions of these proteins vary in primary sequence and in length, and are hypothesized to regulate their apoptotic functions. Indeed, phosphorylation of the BCL- $X_L$ /BCL-2 loop (Ito, T., Deng, X., Carr, B., and May, W. S. (1997) Bcl-2 phosphorylation required for anti-apoptosis function, The Journal of biological chemistry 272, 11671-11673) inhibits anti-apoptotic function depending of the cellular context, and caspase-8-mediated cleavage of the BCL-2 loop transforms the protein into a potent pro-apoptotic protein (Cheng, E. H., Kirsch, et al. (1997) Conversion of Bcl-2 to a Bax-like death effector by caspases, Science 278, 1966-1968), presumably by exposing its BH3 death domain. The pro-apoptotic loop region of cytosolic BAX and BID undergoes enzymatic cleavage by calpain and caspase-8 respectively, producing truncated forms that exhibit enhanced mitochondrial targeting and pro-apoptotic activity (Li, H., et al. (1998) Cleavage of BID by caspase 8 mediates the mitochondrial damage in the Fas pathway of apoptosis, Cell 94, 491-501; Cartron, P. F., et al. (2004) The p18 truncated form of Bax behaves like a Bcl-2 homology domain 3-only protein, J Biol Chem 279, 11503-11512; Wood, D. E., et al.(1998) Bax cleavage is mediated by calpain during drug-induced apoptosis, Oncogene 17, 1069-1078).

Multidomain pro- and anti-apoptotic proteins contain a C-terminal transmembrane region that is enriched in hydrophobic residues and functions as an anchor for mitochondrial outer membrane targeting and insertion (Suzuki, M., Youle, R. J., and Tjandra, N. (2000) Structure of Bax: coregulation of dimer formation and intracellular localization, Cell 103, 645-654). Pro-apoptotic BAX contains a C-terminal $\alpha 9$ helix, presumed to function as a transmembrane region once BAX is deployed to the mitochondrion. In monomeric BAX, $\alpha 9$ engages the BAX hydrophobic groove through complementary hydrophobic interactions, precluding both access to the hydrophobic groove and exposure of its BH3 domain (Sattler, M., et al. (1997) Structure of Bcl-xL-Bak peptide complex: recognition between regulators of apoptosis, Science 275, 983-986). In healthy cells, BAK is constitutively localized to the mitochondrial outer membrane; however, BAX exists as an inactive monomer in the cytosol (Suzuki, M., Youle, R. J., and Tjandra, N. (2000) Structure of Bax: coregulation of dimer formation and intracellular localization, Cell 103, 645-654; Hsu, Y. T., and Youle, R. J. (1998) Bax in murine thymus is a soluble monomeric protein that displays differential detergent-induced conformations, J Biol Chem 273, 10777-1078). Upon receiving an apoptotic stimulus, BAX is believed to undergo a conformational change, leading to its translocation to the mitochondria, homo-oligomerization, and formation of a pore within the outer mitochondrial membrane (Hsu, Y. T., and Youle, R. J. (1998) Bax in murine thymus is a soluble monomeric protein that displays differential detergent-induced conformations, J Biol Chem 273, 10777-1078; Soane, L., and Fiskum, G. (2005) Inhibition of mitochondrial neural cell death pathways by protein transduction of Bcl-2 family proteins, J Bioenerg Biomembr 37, 179-190; Tan, Y. J., Beerheide, W., and Ting, A. E. (1999) Biophysical characterization of the oligomeric state of Bax and its complex formation with Bcl-XL, Biochem Biophys Res Commun 255, 334-339). Previous studies suggested that release of the C-terminal transmembrane region from its groove may be required for translocation and membrane anchoring (Schinzel, A., Kaufmann, T., Schuler, M., Martinalbo, J., Grubb, D., and Borner, C. (2004) Conformational control of Bax localization and apoptotic activity by Pro168, J Cell Biol 164, 1021-1032). Additionally, a conformational change at the N-terminal region, as detected by the monoclonal antibody 6A7, selectively detects a conformationally activated form of BAX (Hsu, Y. T., and Youle, R. J. (1998) Bax in murine thymus is a soluble monomeric protein that displays differential detergent-induced conformations, J Biol Chem 273, 10777-10783; Yethon, J. A., Epand, R. F., Leber, B., Epand, R. M., and Andrews, D. W. (2003) Interaction with a membrane surface triggers a reversible conformational change in Bax normally associated with induction of apoptosis, J Biol Chem 278, 48935-48941) . Exposure of this N-terminal epitope is believed to be a prerequisite for mitochondrial targeting and $\alpha 9$ release (Schinzel, A., Kaufmann, T., Schuler, M., Martinalbo, J., Grubb, D., and Borner, C. (2004) Conformational control of Bax localization and apoptotic activity by Pro168, J Cell Biol 164, 1021-1032). Although a variety of models for BAX/BAK-mediated apoptosis induction have been proposed, the explicit molecular trigger mechanism for BAX/BAK activation has remained unknown. Walensky and co-workers have now identified a novel BH3 interaction site on pro-apoptotic BAX that triggers its activation (Gavathiotis, E., Suzuki, M., Davis, M.L., Pitter, K., Bird, G.H., Katz, S.G., Tu, H.-C., Kim, H., Cheng, E. H.-Y. Tjandra, N. and Walensky, L.D. BAX activation is initiated at a novel interaction site. Nature, in press, 2008). Of note, this new interaction site is located in a distinct geographic region of the BCL-2 family protein, as compared to the BH3 interaction site identified for anti-apoptotic proteins (Figure 5). Just as solving the structure of the *anti*-apoptotic groove in complex with the BH3 helix has led to targeted inhibitors of survival proteins for cancer therapy, elucidating the BH3 interaction site on BAX and defining BAX/BAK structures along their activation continuum will provide new pharmacologic opportunities to modulate apoptosis in human disease (Figure 4).

## IV. *A Novel Interaction Site on BAX that Triggers Its Activation*

To investigate the initiating event for BAX activation, the interaction of BAX with the BH3 ligand BIM SAHB was studied. BIM SAHB was previously demonstrated to recapitulate the α-helical character of native death domains and bound directly to BAX (Walensky, L. D., et al. (2006) A stapled BID BH3 helix directly binds and activates BAX, Mol Cell 24, 199-210). BIM SAHB binding to BAX was monitored using Nuclear Magnetic Resonance (NMR) spectroscopy. Compared to the $^{1}H$-$^{15}N$ correlation spectrum of BAX, the addition of BIM SAHB broadened and shifted select NMR cross-peaks, indicating fast exchange between the bound and unbound conformations of BAX. The overall features of the NMR spectra were quite similar except for significant changes in the loop residues between α1 and α2 upon BIM SAHB binding. Chemical shift perturbation mapping of BAX with BIM SAHB titration revealed interactions at a discrete subset of BAX residues. The largest changes were observed for residues localized in the α1 and α6 helices, as well as residues in the flexible loop between α1 and α2. Significant changes were also observed for the side-chain $NH_2$ of Q28, Q32, and Q52. In the BAX structure (Suzuki, M., Youle, R. J., and Tjandra, N. (2000) Structure of Bax: coregulation of dimer formation and intracellular localization, Cell 103, 645-654), the α1 and α6 helices are positioned adjacent to one another, and the residues impacted by BIM SAHB binding localized to a discrete site at the juxtaposition of these helices on one side of the protein structure. Of note, no residues on the carboxy terminal side of the protein were affected by BIM SAHB titration under these conditions, thus placing the novel binding site on the completely opposite face of the protein from the canonical BH3 binding site of anti-apoptotic proteins (Figure 5). The binding site of BIM SAHB on BAX is thus defined by the two helices α1 and α6, with the interhelical junction forming a hydrophobic cleft surrounded by a perimeter of hydrophilic and charged residues (Figure 6).

Using the structurally-defined topography of the novel interaction site on BAX, an *in silico* screening was undertaken and identified a series of small molecules predicted to engage the new BH3-binding groove on BAX. The identified molecules were then subjected to a series of biochemical assays to validate their functional activity, including competitive fluorescence polarization, BAX oligomerization, cytochrome c release, and cell-based apoptosis induction assays.

## V. *Computer based drug design*

Identification of a binding site aids the development and identification of compounds that are capable of modulating BAX and other BCL-2 family polypeptides having a corresponding binding site. For example, using this information, a three-dimensional computer generated interaction template of BAX can be generated by one of ordinary skill in the art and used to design activators and inhibitors specific for the BAX active site. In another embodiment, one of ordinary skill in the art can apply the BAX active site to identify corresponding active sites in other BCL-2 family members. This information may then be used to identify/develop compounds capable of modulating the other BCL-2 family polypeptides.

Determination of the three dimensional structure of the BCL-2 polypeptide and specifically the binding site is critical to the rational identification and/or design of agents that may act as modulators of BCL-2 family polypeptide activity. This is advantageous over conventional drug assay techniques, in which the only way to identify such an agent is to screen thousands of test compounds until an agent having the desired inhibitory effect on a target compound is identified. Necessarily, such conventional screening methods are expensive, time consuming, and do not elucidate the method of action of the identified agent on the target protein. Using such a three dimensional structure, researchers identify putative binding sites and then identify or design agents to interact with these binding sites. These agents are then screened for a modulating effect upon the target molecule. In this manner, not only are the number of agents to be screened for the desired activity greatly reduced, but the mechanism of action on the target compound is better understood.

It is contemplated that identification of the BAX binding site can be used to computationally screen small molecule databases for compounds that can bind in whole, or in part, to one or more of the regions of the BCL-2 family polypeptide's binding site. In one variant of this method, the quality or fit of the compound identified to the regions of the binding site can be judged either by shape complementarity or by estimated interaction energy (Meng et al., J. Comp. Chem. 13:505-524, 1992).

In a further variant, potential modulators that can be analyzed according to the methods disclosed herein can be obtained using any of the numerous approaches in combinatorial library methods known in the art. In one variant, potential modulators are first identified for pro-apoptotic or anti-apoptotic activity using the in vitro assays described herein or known in the art. Once potential modulators are identified, and their structures determined, further optimization can be carried out by computational analyses using the structure information of the BAX binding site described herein. In another variant, a potential modulator is first identified in a screen using an interaction template developed from the structure coordinates of the BCL-2 family binding site and further subjected to optimization by additional computational analyses. Alternatively, further optimization can be carried out by determining the NMR structural coordinates of co-complexes of the potential modulator and the BCL-2 family binding site using the methods described herein.

Various combinatorial libraries that can be used in the methods of the present disclosure include, but are not limited to: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam (1997) Anticancer Drug Des. 12:145).

In a preferred variant, the library of compounds is a digital library. The binding interaction is performed with a database searching program which is capable of scanning a database of small molecules of known three-dimensional structure for candidates that fit into the binding site. Suitable software programs include CATALYST (Molecular Simulations Inc., San Diego, CA), UNITY (Tripos Inc., St Louis, MO), FLEXX (Rarey et al., J. Mol. Biol. 261: 470- 489 (1996)), CHEM-3-DBS (Oxford Molecular Group, Oxford, UK), DOCK (Kuntz et al., J. Mol. Biol 161: 269-288 (1982)), and MACCS-3-D (MDL Information Systems Inc., San Leandro, CA) and LUDI (Boehm, J. Comp. Aid. Mol. Des. 6:61-78 (1992)), CAVEAT (Bartlett et al. in "Molecular Recognition in Chemical and Biological Problems", special publication of The Royal Chem. Soc., 78:182-196 (1989)) and MCSS (Miranker et al. Proteins 11: 29-34 (1991)), GLIDE (Schrodinger LLC, New York, NY), and PHASE (Schrodinger LLC, New York, NY).

Further, examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner U.S. Pat. No. 5,223,409), spores (Ladner U.S. Pat. No. '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner supra.).

The potential modulator effect of a compound can be further analyzed prior to its actual synthesis and testing by use of computer modeling techniques using the structural coordinates of the BAX active site. If the computer modeling indicates an interaction, the molecule can then be synthesized using standard methods known to those skilled in the chemical arts, and then tested for its ability to modulate the activity of a BCL-2 family polypeptide using the assays set forth herein.

A modulator or other binding compound of a BCL-2 family polypeptide may be computationally evaluated and designed by means of a series of steps in which chemical entities or fragments are screened and selected for their ability to associate with the individual binding site. In other variants of the method of the invention, potential modulator compounds can be examined for their ability to associate with a BCL-2 family polypeptide's binding site and more particularly with a BAX binding site. This process can involve visual inspection of, for example, the binding site on a computer screen based on the structural coordinates of the BAX binding site. Selected compounds or chemical moieties can then be positioned in a variety of orientations, or docked, within an individual region of the binding site as defined herein. Docking can be accomplished using software such as Quanta and SYBYL, followed by energy minimization and molecular dynamics with standard molecular mechanics forcefields, such as CHARMM and AMBER.

Further disclosed as variants of the above method are methods involving the inputting of structural coordinates of BCL-2 family polypeptides into an electronic storage medium to generate a three-dimensional computer model of the polypeptide. In one variant, the complete structural coordinates of a BCL-2 family polypeptide are input. In an alternative variant, a fragment, or less than the complete structural coordinates, but including the binding site are inputted. The structural coordinates may be known in the art or based on homology modeling. For example, known BCL-2 family structural coordinates include BAX (PDB ID No. 1f16), BAK (PDB ID No. 2ims), BCL-2 (PDB ID No. 1g5m), BCL-XL (PDB ID No. 1lxl), in addition to those associated with this invention: BIM BH3-BAX (PDB ID No. 2k7w), as well as others known in the art. Structural coordinates for many known BCL-2 family polypeptides can be obtained from the Protein Data Bank ("PDB") (Research Collaboratory for Structural Bioinformatics; http://www.rcsb.org).

The present disclosure further provides that the structural coordinates of the present invention may be used with standard homology modeling techniques in order to determine the unknown three-dimensional structure of a molecule or molecular complex. Homology modeling involves constructing a model of an unknown structure using structural coordinates of one or more related protein molecules, molecular complexes or parts thereof (i.e., binding sites). Homology modeling may be conducted by fitting common or homologous portions of the protein whose three dimensional structure is to be solved to the three dimensional structure of homologous structural elements in the known molecule, specifically using the relevant (i.e., homologous) structural coordinates. Homology may be determined using amino acid sequence identity, homologous secondary structure elements, and/or homologous tertiary folds. Homology modeling can include rebuilding part or all of a three dimensional structure with replacement of amino acid residues (or other components) by those of the related structure to be solved.

Similar methods are known to those skilled in the art (Greer, 1985, Science 228, 1055; Bundell et al 1988, Eur. J. Biochem. 172, 513; Knighton et al., 1992, Science 258:130-135, http://biochem.vt.edu/courses/modeling/homology.htm). Computer programs that can be used in homology modeling include Quanta and the homology module in the Insight II modeling package (Accelrys, Inc., San Diego, CA) or MODELLER (Rockefeller University, www.iucr.ac:uk/sinristop/logical/prg-modeller.html, Sali's Modeller also from Accelrys, Inc., San Diego, CA).

Once an interaction template is prepared compounds which bind the BCL-2 family polypeptide's binding site can be identified. Specialized computer programs that can also be used in the process of selecting compounds or chemical entities include:

1. SYBYL Available from Tripos Inc., 1699 South Hanley Rd., St. Louis, Mo., 63144, USA
2. UNITY Available from Tripos Inc., 1699 South Hanley Rd., St. Louis, Mo., 63144, USA
3. FlexX Available from Tripos Inc., 1699 South Hanley Rd., St. Louis, Mo., 63144, USA
4. GRID (Goodford, P. J., "A Computational Procedure for Determining Energetically Favorable Binding Sites on Biologically Important Macromolecules", J. Med. Chem., 28, pp. 849-857 (1985)). GRID is available from Oxford University, Oxford, UK.
5. MCSS (Miranker, A. and M. Karplus, "Functionality Maps of Binding Sites: A Multiple Copy Simultaneous Search Method." Proteins: Structure. Function and Genetics, 11, pp. 29-34 (1991)). MCSS is available from Molecular Simulations, Burlington, Mass.
6. AUTODOCK (Goodsell, D. S. and A. J. Olsen, "Automated Docking of Substrates to Proteins by Simulated Annealing", Proteins: Structure. Function, and Genetics, 8, pp. 195-202 (1990)). AUTODOCK is available from Scripps Research Institute, La Jolla, Calif.
7. DOCK (Kuntz, I. D. et al., "A Geometric Approach to Macromolecule-Ligand Interactions", J. Mol. Biol., 161, pp. 269-288 (1982)). DOCK is available from University of California, San Francisco, Calif.

Once suitable compounds or chemical moieties have been selected, they can be assembled into a single compound or inhibitor. Assembly may be proceed by visual inspection of the relationship of the compounds or moieties to each other on the three-dimensional image displayed on a computer screen in relation to the structure coordinates of the BAX/BIM-BH3 NMR binding studies. This could then be followed by manual model building using software such as Quanta or SYBYL.

Other useful programs to aid one of skill in the art in connecting the individual compounds or chemical entities include:

1. CAVEAT (Bartlett, P. A. et al, "CAVEAT: A Program to Facilitate the Structure-Derived Design of Biologically Active Molecules". In "Molecular Recognition in Chemical and Biological Problems", Special Pub., Royal Chem. Soc., 78, pp. 182-196 (1989)). CAVEAT is available from the University of California, Berkeley, Calif.
2. 3D Database systems such as MACCS-3D (MDL Information Systems, San Leandro, Calif.). This area is reviewed in Martin, Y. C., "3D Database Searching in Drug Design", J. Med. Chem., 35, pp. 2145-2154 (1992)).
3. HOOK (available from Molecular Simulations, Burlington, Mass.).

In other embodiments, BCL-2 family polypeptide modulators can be designed as a whole or "de novo" using either an empty active site or optionally including some portion(s) of a known modulator(s). Programs which can aid in these methods include:

1. LUDI (Bohm, H.-J., "The Computer Program LUDI: A New Method for the De Novo Design of Enzyme Inhibitors", J. Comp. Aid. Molec. Design, 6, pp. 61-78 (1992)). LUDI is available from Biosym Technologies, San Diego, Calif.
2. LEGEND (Nishibata, Y. and A. Itai, Tetrahedron, 47, p. 8985 (1991)). LEGEND is available from Molecular Simulations, Burlington, Mass.
3. LeapFrog (available from Tripos Associates, St. Louis, Mo.).

Other molecular modeling techniques may also be employed in accordance with this invention. See, e.g., Cohen, N. C. et al., "Molecular Modeling Software and Methods for Medicinal Chemistry", J. Med. Chem., 33, pp. 883-894 (1990). See also, Navia, M. A. and M. A. Murcko, "The Use of Structural Information in Drug Design", Current Opinions in Structural Biology, 2, pp. 202-210 (1992).

Once a compound has been designed or selected by the above methods, the efficiency with which that compound modulates a BCL-2 family polypeptide can be tested and optimized by computational evaluation. An effective BCL-2 family polypeptide modulator must preferably demonstrate a relatively small difference in energy between its bound and free states (i.e., a small deformation energy of binding).

A compound designed or selected as a modulator of BCL-2 family polypeptide can be further computationally

optimized so that in its bound state it would preferably lack repulsive electrostatic interaction with the target protein. Such non-complementary (e.g., electrostatic) interactions include repulsive charge-charge, dipole-dipole and charge-dipole interactions. Specifically, the sum of all electrostatic interactions between the modulator and the enzyme when the modulator is bound to BCL-2 family polypeptide preferably make a neutral or favorable contribution to the enthalpy of binding.

Specific computer software is available in the art to evaluate compound deformation energy and electrostatic interaction. Examples of programs designed for such uses include: Gaussian 92, revision C, M. J. Frisch, Gaussian, Inc., Pittsburgh, Pa.; AMBER, version 4.0, P. A. Kollman, University of California at San Francisco; QUANTA/CHARMM, Molecular Simulations, Inc., Burlington, Mass.; and Insight II/Discover (Biosysm Technologies Inc., San Diego, Calif.). These programs may be implemented, for instance, using a Silicon Graphics workstation, IRIS 4D/35 or IBM RISC/6000 workstation model 550. Other hardware systems and software packages will be known to those skilled in the art.

Once a BCL-2 family polypeptide modulator has been optimally selected or designed, as described herein, substitutions can then be made in some of its atoms or side groups in order to improve or modify its binding properties, again using the information provided by the interaction and specificity templates to identify regions amiable to modification. Generally, initial substitutions are conservative, i.e., the replacement group will have approximately the same size, shape, hydrophobicity and charge as the original group. It should, of course, be understood that components known in the art to alter conformation should be avoided. Such substituted chemical compounds may then be analyzed for efficiency of fit to BCL-2 family polypeptides by the same computer methods described in detail, above.

In certain variants the modulators have a Kd for BCL-2 family polypeptides of less than 0.2 mM, less than 0.1 mM, less than 750 μM, less than 500 μM, less than 250 μM, less than 100 μM, less than 50 μM, less than 500 nM, less than 250 nM, less than 50 nM, less than 30nm, less than 20nM, less than 10 nM, less than 5 nM, less than 3 nM, less than 1 nM, or less than 0.5 nM.

Designed modulators can be further evaluated using in vitro or in vivo assays known in the art and described herein.

## VI. *Fragment-Based Drug Design*

Fragment-based drug discovery can also be used to identify compounds which interact with the new active site of BAX or the corresponding site on other BCL-2 polypeptides. These structural methods are known and computational tools for their use commercially available, for example "SAR by NMR" (Shukers, S. B., et al., Science, 1996, 274, 1531-1534), "SHAPES NMR" (Fejzo J, Lepre CA, Peng JW, Bemis GW, Ajay , Murcko MA, Moore JM., Chem Biol. 1999 Oct;6(10):755-69), "Fragments of Active Structures" (www.stromix.com; Nienaber, V. L., et al., Nat. Biotechnol., 2000, 18, 1105-1108), and "Dynamic Combinatorial X-ray Crystallography" (e.g., permitting self-selection by the protein molecule of selfassembling fragments; Congreve, M. S., et al., Angew. Chem., Int. Ed., 2003, 42, 4479-4482), and other fragment-based NMR and x-ray crystallographic methods (e.g. Congreve et al., J. Med. Chem., 2008, 51 (13), 3661-3680; Klagesa, J., Colesb, M., Kesslera, H. NMR-based screening: a powerful tool in fragment-based drug discovery in Exploiting Chemical Diversity for Drug Discovery, 2006, ed. Bartlett, P.A. and Entzeroth, M. RSC Publishing, Cambridge, UK; Erlanson, D.A., Wells, J.A., and Braisted, A.C. TETHERING: Fragment-Based Drug Discovery, Annual Review of Biophysics and Biomolecular Structure, 2004, 33, 199-223; Zartler ER and Shapiro MJ, Fragonomics: fragment-based drug discovery, Curr Opin Chem Biol, 2005, 9(4):366-70.

## VII. *In vitro assays for assessing BCL-2 family peptide modulation and compound binding*

Determining the ability of a compound, found to bind a binding site of a BCL-2 family polypeptide based on computer modeling, can be evaluate further for BCL-2 family polypeptide interaction by testing direct binding. Determining the ability of a test compound to bind to a BCL-2 family polypeptide can be accomplished, for example, by coupling the BCL-2 family polypeptide or compound with a radioisotope or enzymatic label such that binding of the BCL-2 family polypeptide to the potential modulator can be determined by detecting the labeled BCL-2 family polypeptide in a complex. For example, a compound can be labeled with $^{125}I$, $^{35}S$, $^{14}C$, or $^{3}H$, either directly or indirectly, and the radioisotope detected by direct counting of radioemmission or by scintillation counting. Alternatively, the compound can be enzymatically labeled with, for example, horseradish peroxidase, alkaline phosphatase, or luciferase, and the enzymatic label detected by determination of conversion of an appropriate substrate to product. As a further example, the compound can be labelled with fluorescent label such as fluorescein and binding interactions between ligand and BCL-2 family polypeptide quantitated using a fluorescence polarization assay. Additionally, compound binding to the target BCL-2 family polypeptide can be analyzed by NMR or x-ray crystallography of the complex using a variety of established methodologies known in the art, including SAR by NMR (Shukers, S. B., et al., Science, 1996, 274, 1531-1534).

In other variants, determining the ability of the modulator to bind to BCL-2 family polypeptides can be determined by detecting induction of a downstream event (e.g., polypeptide conformation change, apoptosis, release of mitochondrial

cytochrome c, etc.) or detecting another BCL-2 family-regulated cellular response.

In another variant, the assay is a cell-free assay in which a BCL-2 family protein or biologically active portion thereof containing a binding site is contacted with a test compound and the ability of the test compound to modulate the activity of the BCL-2 family protein or biologically active portion thereof is determined. Determining the ability of the test compound to modulate the activity of a BCL-2 family protein can be accomplished, for example, by determining the ability of the BCL-2 family protein to bind to another BCL-2 family target molecule (e.g., competition binding assay in which BAX binding to a hydrocarbon-stapled BIM BH3 polypeptide is monitored).

Determining the ability of the BCL-2 family protein to bind to a target molecule can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (e.g., BLAcore). Changes in the optical phenomenon of surface plasmon resonance (SPR) can be used as an indication of real-time reactions between biological molecules.

In an alternative variant, determining the ability of the test compound to modulate the activity of a BCL-2 family protein can be accomplished by determining the ability of the BCL-2 family protein to modulate the activity of a downstream BCL-2 family target molecule. For example, the activity of the effector molecule on an appropriate target can be determined, or the binding of the effector to an appropriate target can be determined as previously described.

In yet another variant, the cell-free assay involves contacting a BCL-2 family protein (e.g., BAX) or biologically active portion thereof containing a binding site, with a known compound which binds the BCL-2 family protein (e.g. a hydrocarbon-stapled BIM BH3 polypeptide) to form an assay, and determining the ability of the test compound to interact with the BCL-2 family protein, wherein determining the ability of the test compound to interact with the BCL-2 family protein comprises determining the ability of the test compound to preferentially bind to or modulate the activity of a BCL-2 family protein and displace the known compound.

In more than one variant of the above assay methods, it may be desirable to immobilize either the BCL-2 family polypeptide or its target molecule to facilitate separation of complexed from uncomplexed forms of one or both of the proteins, as well as to accommodate automation of the assay. Binding of a test compound to a BCL-2 family protein, or interaction of a BCL-2 family protein with a target molecule in the presence and absence of a candidate compound, can be accomplished in any vessel suitable for containing the reactants. Examples of such vessels include microtiter plates, test tubes, and microcentrifuge tubes. In one embodiment, a fusion protein can be provided which adds a domain that allows one or both of the proteins to be bound to a matrix. For example, glutathione-S-transferase/BCL-2 family fusion proteins or glutathione-S-transferase/target fusion proteins can be adsorbed onto glutathione sepharose beads (Sigma Chemical, St. Louis, Mo.) or glutathione derivatized microtitre plates, which are then combined with the test compound or the test compound and either the non-adsorbed target protein or BCL-2 family protein, and the mixture incubated under conditions conducive to complex formation (e.g., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of BCL-2 family binding or activity determined using standard techniques.

Other techniques for immobilizing proteins on matrices can also be used in the assays of the invention. For example, either a BCL-2 family protein or a BCL-2 family target molecule can be immobilized utilizing conjugation of biotin and streptavidin. Biotinylated BCL-2 family protein or target molecules can be prepared from biotin-NHS (N-hydroxy-succinimide) using techniques well known in the art (e.g., biotinylation kit, Pierce Chemicals, Rockford, Ill.), and immobilized in the wells of streptavidin-coated 96 well plates (Pierce Chemical). Alternatively, antibodies reactive with BCL-2 family protein or target molecules but which do not interfere with binding of the BCL-2 family protein to its target molecule can be derivatized to the wells of the plate, and unbound target or BCL-2 family protein trapped in the wells by antibody conjugation. Methods for detecting such complexes, in addition to those described above for the GST-immobilized complexes, include immunodetection of complexes using antibodies reactive with the BCL-2 family protein or target molecule, as well as enzyme-linked assays which rely on detecting an enzymatic activity associated with the BCL-2 family protein or target molecule. For example, using a microtitre plate assay design, a specialized antibody that recognizes the activated or inhibited conformer of the target BCL-2 family polypeptide can be coated onto the microtitre plate to capture the compound-induced alteration of the target BCL-2 family polypeptide, which is then detected by applying a second BCL-2 family antibody that is either conjugated to a fluorophore or enzyme, or recognized by a secondary antibody conjugated to a fluorphore or enzyme, for rapid and sensitive detection (i.e. "sandwich" ELISA assay).

The compounds that bind a binding site of BCL-2 family polypeptides may be demonstrated to inhibit tumor cell number *in vitro* or *in vivo* using a variety of assays known in the art, or described herein. Such assays can use cells of a cancer cell line or cells from a patient in the presence and absence of the compound of interest. Preferably the cell has a deregulated BCL-2 family polypeptide pathway. The ability of a compound or a regimen of the invention to reduce the number of cancer cells or inhibit their proliferation can be assessed by methods known in the art and described herein.

Disclosed are methods (also referred to herein as "screening assays") for identifying compounds which bind

to a binding site and modulate the activity of one or more BCL-2 family proteins. Importantly, these assays can be used to test and validate compounds identified by computer-based screening, but also employed in non-computational, empiric compound screening from exhaustive libraries.

The binding affinity of polypeptides described herein can be determined using, for example, a titration binding assay. A BCL-2 family polypeptide or polypeptide comprising a BH domain (e.g., BAX, etc.) can be exposed to varying concentrations of a candidate compound (e.g., 1 nM, 10 nM, 100 nM, 1 uM, 10 uM, 100 uM, 1 mM, and 10 mM) in the presence of a substrate such as a fluorescently labeled BH3 containing polypeptide or a fragment thereof (e.g., BID, BAD, BAK, BAX, etc.), or a hydrocarbon stapled derivative thereof. The effect of each concentration of candidate compound is then analyzed to determine the effect of the candidate compound on BCL-2 family polypeptide binding activity at varying concentrations, which can be used to calculate the Ki of the candidate compound. The candidate compound can modulate BCL-2 type activity in a competitive or non-competitive manner. Direct binding assays can also be performed between BCL-2 family proteins and fluorescently labeled candidate compounds to determine the Kd for the binding interaction. Candidate compounds could also be screened for biological activity in vitro, for example, by measuring their dose-responsive efficacy in triggering BCL-2 family protein conformational change as measured by conformation-specific antibodies or HSQC NMR analysis, a change in BCL-2 family protein (e.g. BAX) oligomerization state as monitored by size exclusion chromatography (SEC)-based analysis, fluorophore or protein-conjugated fluorophore-induced release from liposomes, and/or cytochrome c release from purified mitochondria. Cell permeability screening assays are also envisioned, in which fluorescently or otherwise labeled candidate compounds are applied to intact cells, which are then assayed for cellular fluorescence by microscopy or FACS analysis as described (Walensky et al Science 2004, Walensky et al Mol Cell 2006), or by high-throughput cellular fluorescence detection.

A compound, pharmaceutical composition, or regimen of the invention is preferably tested *in vitro* and then *in vivo* for the desired therapeutic or prophylactic activity prior to use in humans. For example, assays which can be used to determine whether administration of a specific compound is effective include cell culture assays in which a patient tissue sample (e.g., cancer cell) is grown in culture and exposed to, or otherwise contacted with, a compound of the invention, and the effect of such compound upon the tissue sample is observed. The tissue sample can be obtained by biopsy or blood/bone marrow draw from the patient. This test allows the identification of the therapeutically most effective therapy (e.g., prophylactic or therapeutic agent) for each individual patient.

The assays described herein can be performed with individual candidate compounds or can be performed with a plurality of candidate compounds. Where the assays are performed with a plurality of candidate compounds, the assays can be performed using mixtures of candidate compounds or can be run in parallel reactions with each reaction having a single candidate compound. The test compounds or agents can be obtained using any of the numerous approaches in combinatorial library methods known in the art.

In a preferred variant, cell-based assay is performed on a compound which is known to bind a binding site (e.g., identified via computer modeling, direct binding assay, NMR, or other method) of a BCL-2 family polypeptide in order to determine whether the compound also modulates the activity of the BCL-2 family polypeptide.

In one variant, an assay is a cell-based assay in which a cell that expresses a BCL-2 family protein or biologically active portion thereof is contacted with a candidate compound, and the ability of the candidate compound to bind to a biding site and modulate BCL-2 type activity is determined (e.g., in some instances increase in apoptosis and in other instances decrease apoptosis, via intrinsic or extrinsic cell death pathways). Determining the ability of the test compound to modulate BCL-2 type activity within cells can be accomplished by monitoring, for example, release of cytochrome c from the mitochondria or other relevant physiologic readout (e.g., annexin V binding, MTT assay, caspase activity assay, TUNEL assay). For example, to assay for a compound's induction of specific and direct BAX-mediated apoptosis, the response to compound of a cell that is genetically deleted for BAX/BAK (i.e. negative control cell) is compared to that same cell in which BAX has been replaced by transfection or retroviral infection (i.e. test cell), as reported and described herein (ref Gavathiotis et al. 2008, Nature, in press).

In vitro anti-tumor activity of the compounds found to bind to a binding site of a BCL-2 polypeptide can be assayed by measuring the ability of the compound to kill tumor cells. Examples of cell lines include: human lung (A549); resistant human lung with low topo II activity (A549-VP); murine melanoma (B16); human colon tumor (HCT116); human clone tumor with elevated p170 levels (HCTVM); human colon tumor with low topo II activity (HCTVP); P388 murine lymph leukemia cells; and human colon carcinoma cell line (Moser), and many others known in the art.

Tumor inhibition assays are described, for example, in Kelly, et al., U.S. Pat. No. No. 5,166,208, and in Pandley, et al., J. Antibiot. 3(11):1389-401 (1981). In one assay, the cells are allowed to grow for a 24 hour period under standard conditions. After the cells are allowed to attach to the plate for 24 hours (e.g., a 96-well flat bottom plate), the cells are incubated for 72 hours with serially diluted concentrations of the BCL-2 family modulator compound. From these data, the concentration of the compound at which 50% of the cells are killed or growth inhibited (IC50) is determined.

**VIII. *In vivo testing of compounds***

The compounds of the invention and the referential compounds identified by the above method can also be demonstrated to inhibit tumor formation *in vivo.* The compounds, pharmaceutical compositions, and regimens of the invention can be tested in suitable animal model systems prior to use in humans. Such animal model systems include, but are not limited to, rats, mice, chicken, cows, monkeys, pigs, dogs, rabbits, etc. Any animal system well-known in the art may be used. Several aspects of the procedure may vary; said aspects include, but are not limited to, the temporal regime of administering the therapeutic modalities (e.g., prophylactic and/or therapeutic agents), whether such therapeutic modalities are administered separately or as an admixture, and the frequency of administration of the therapeutic modalities.

In vivo anti-tumor activity of BCL-2 family modulator compounds of the invention can be assayed by a reduction of tumor cells in mammals (e.g., mice) and a resulting increase in survival time compared to untreated tumor bearing animals. For example, CDF1 mice are injected interperitoneally with a suspension of P388 murine lymph leukemia cells, Ehrlich carcinoma cells, B16 melanoma cells, or Meth-A fibrosarcoma cells. Some of the injected mice are then treated interperitoneally with a BCL-2 family modulator compound of the invention, and other mice are treated with saline or a control compound (e.g. enantiomer of small molecule, amino acid mutant of peptide). The in vivo activity of the compound is then determined in terms of the % T/C which is the ratio of the mean survival time of the treated group to the mean survival time of the saline treated group times 100. Yokoi, et al., U.S. Pat. No. 4,584,377; Kelly, et al., U.S. Pat. No. 5,155,208; Warnick-Pickle, et al., J. Antibiot. 34(11):1402-7 (1981); and Pandley et al., supra.

A vast number of animal models of hyperproliferative disorders, including tumorigenesis and metastatic spread, are known in the art and are disclosed herein (see Chapter 317, "Principals of Neoplasia," in Harrison's: Principals of Internal Medicine, 13th Edition, Isselbacher et al., eds., McGraw-Hill, New York, p. 1814, and Lovejoy et al., 1997, J. Pathol. 181:130-135). Hyperpoliferative disorders include cellular proliferation or apoptotic blockage disorders such as cancer and autoimmune disease. Examples of BCL-2 related cancers include, but are not limited to, solid tumors, leukemias, and lymphomas. In one variant, the disorder is a chemoresistant cancer. In a further variant, the chemoresistant cancer is resistant to ABT-737 or ABT-263 (available from Abbott; Abbott Park, Illinois) or obatoclax (available from Gemin X). Specific examples include for lung cancer, transplantation of tumor nodules into rats (Wang et al., 1997, Ann. Thorac. Surg. 64:216-219) or establishment of lung cancer metastases in SCID mice depleted of NK cells (Yono and Sone, 1997, Gan To Kagaku Ryoho 24:489-494); for colon cancer, colon cancer transplantation of human colon cancer cells into nude mice (Gutman and Fidler, 1995, World J. Surg. 19:226-234), the cotton top tamarin model of human ulcerative colitis (Warren, 1996, Aliment. Pharmacol. Ther. Supp 12:45-47) and mouse models with mutations of the adenomatous polyposis tumor suppressor (Polakis, 1997, Biochim. Biophys. Acta 1332:F127-F147); for breast cancer, kansgenic models of breast cancer (Dankort and Muller, 1996, Cancer Treat. Res. 83:71-88; Amundadittir et al., 1996, Breast Cancer Res. Treat. 39:119-135) and chemical induction of tumors in rats (Russo and Russo, 5 1996, Breast Cancer Res. Treat. 39:7-20); for prostate cancer, chemically-induced and transgenic rodent models, and human xenograft models (Royal et al., 1996, Semin. Oncol. 23:35-40), for genitourinary cancers, induced bladder neoplasm in rats and mice (Oyasu, 1995, Food Chem. Toxicol 33:747-755) and xenografts of human transitional cell carcinomas into nude rats (Jarrett et al., 1995, J. Endourol. 9:1 -7); and for hematopoietic cancers, transplanted allogeneic marrow in animals (Appelbaum, 1997, Leukemia 11 (Suppl. 4):S15- S17). Further, general animal models applicable to many types of cancer have been described, including, but not restricted to, the p53-deficient mouse model (Donehower, 1996, Semin. Cancer Biol. 7:269-278), the Min mouse (Shoemaker et al., 1997, Biochem. Biophys. Acta, 1332:F25-F48), and immune responses to tumors in rat 15 (Frey, 1997, Methods, 12:173-188).

For example, a compound of the invention as well as the referential compounds identified as above can be administered to a test animal, in one embodiment a test animal predisposed to develop a type of tumor, and the test animal subsequently examined for a decreased incidence of tumor formation in comparison with an animal not administered the compound. Alternatively, a compound can be administered to test animals having tumors (e.g., animals in which tumors have been induced by introduction of malignant, neoplastic, or trans-formed cells, or by administration of a carcinogen) and subsequently examining the tumors in the test animals for tumor regression in comparison to animals not admin-istered the compound. A compound of the invention or a referential compound is considered effective in treating a hyperpoliferative disorder when administration of a therapeutically effec-tive amount increases time to tumor progression or increases survival time by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Similarly, a compound of the invention or a referential compound is considered effective in treating a hyperpoliferative disorder when administration of a therapeutically ef-fective amount decreases the rate of tumor growth, decreases tumor mass, decreas-es the number of metastases by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Such results can be determined by one having ordinary skill in the relevant

art, e.g., oncologist, cancer biologist.

Further, any assays known to those skilled in the art can be used to evaluate the prophylactic and/or therapeutic utility of a compound or pharmaceutical composition disclosed herein for disorder associated with excessive cellular proliferation or cellular death or one or more symptoms thereof.

## IX. *Compounds identified by the in silico screen described herein (referential compounds)*

The following compounds in Table 1 were identified by computational screening using the structural coordinates of the new BH3 interaction site on BAX, as described herein. The compound numbers presented for the structures in Table 1 are synonymous with the Chembridge compound numbers for said structures.

Table 1

| | |
|---|---|
| 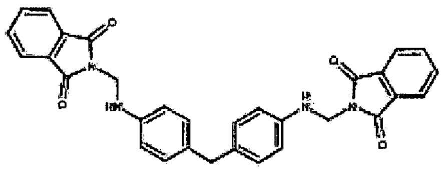 | 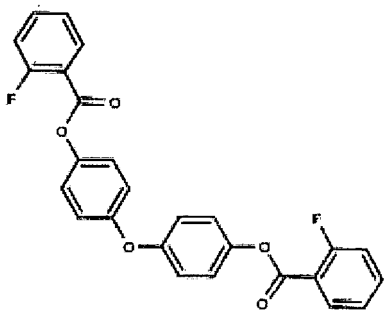 |
| 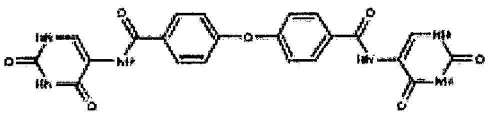 | 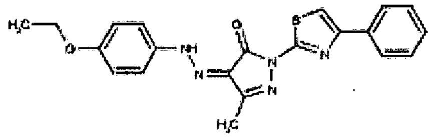 5258079 |
| 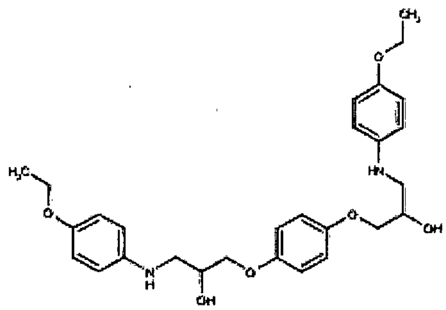 | 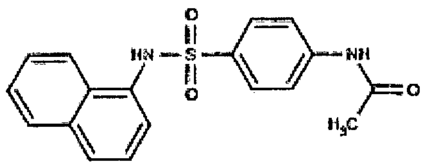 5285738 |
| 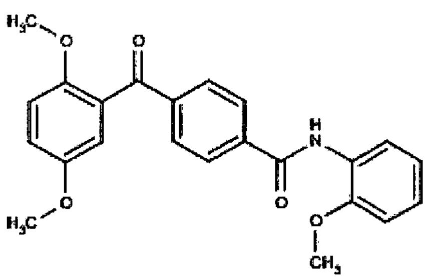 | 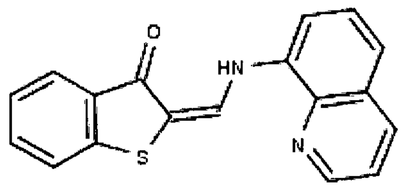 |
| 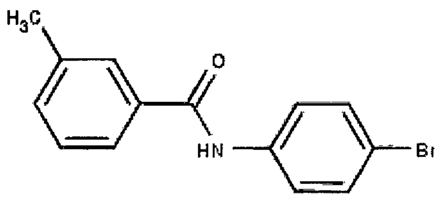 | 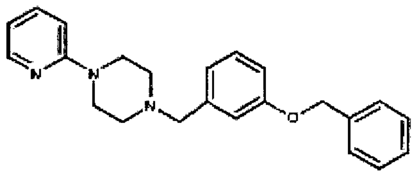 |

(continued)

| | |
|---|---|
| 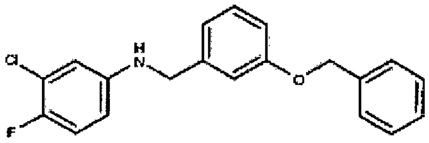 | 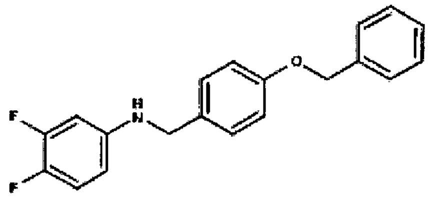 |
| 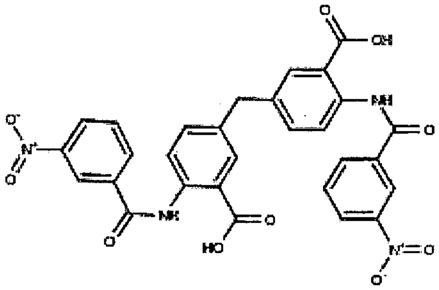 5467929 | 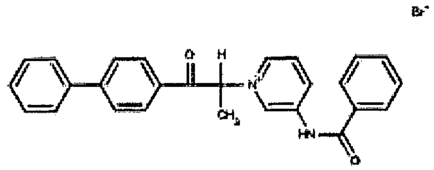 5476487 |
| 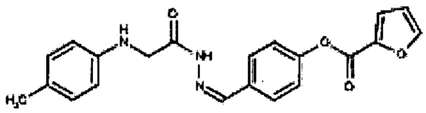 5523016 | 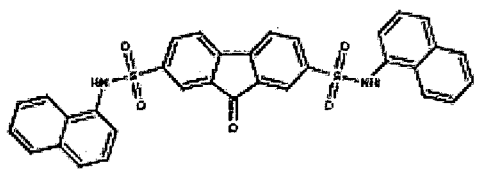 |
| 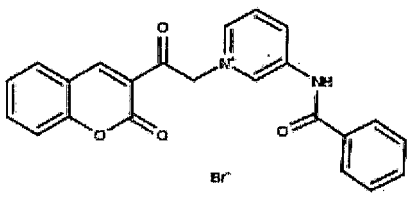 | 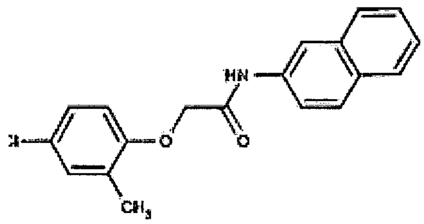 |
| 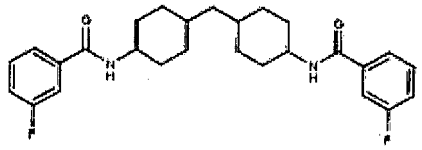 | 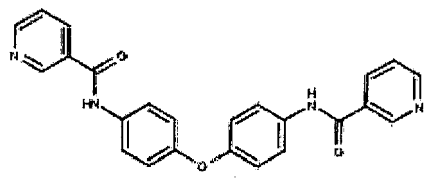 |
| 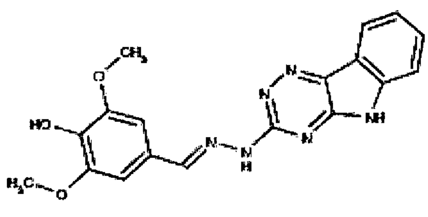 5625872 | 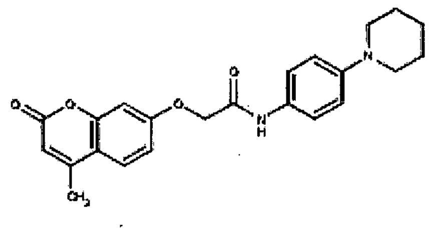 |
| 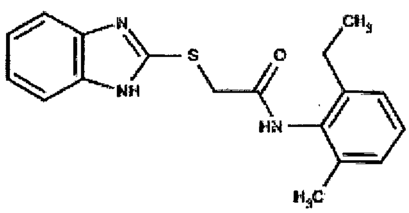 | 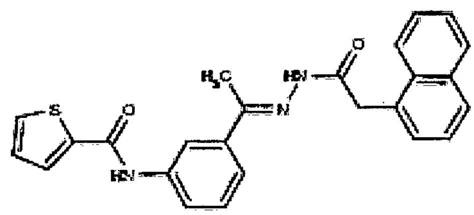 |

(continued)

| | |
|---|---|
| 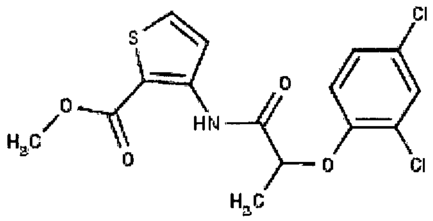 | 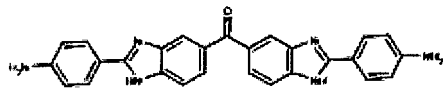 5768154 |
| 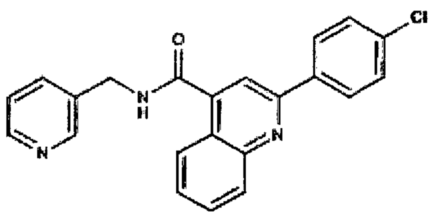 | 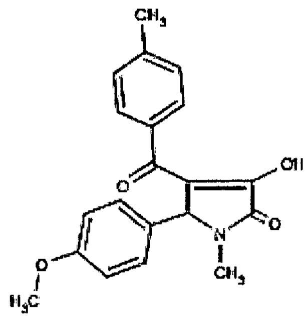 |
| 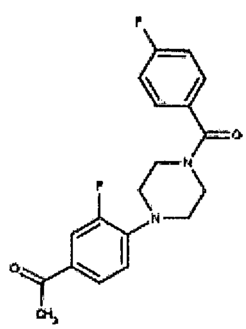 | 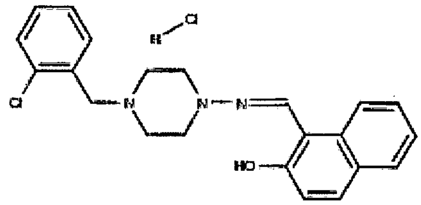 |
| 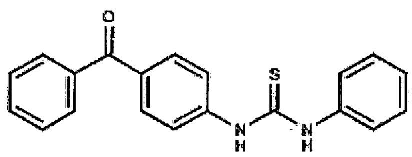 | 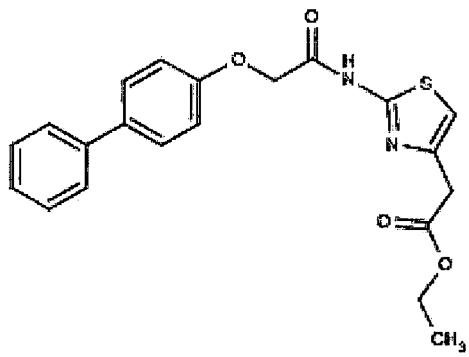 |
| 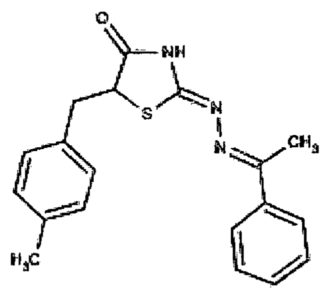 | 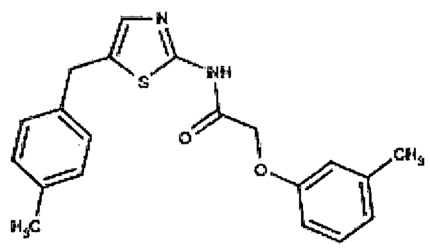 |

(continued)

| | |
|---|---|
| 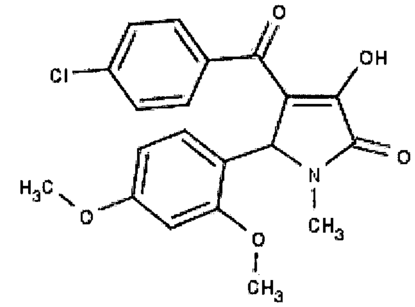 | 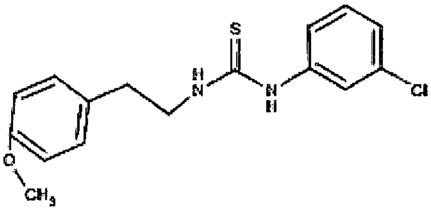 |
| 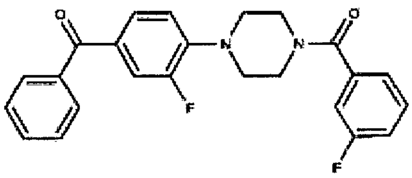 | 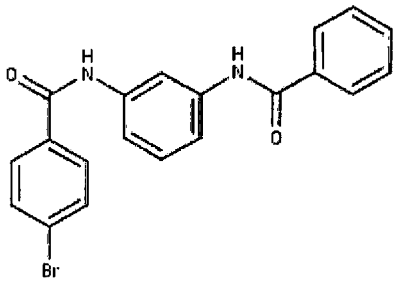 |

(continued)

| | |
|---|---|
| 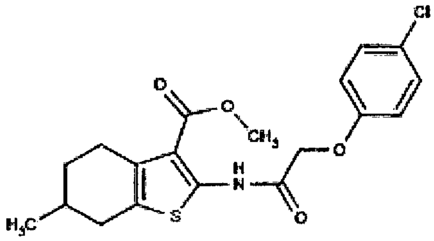 | 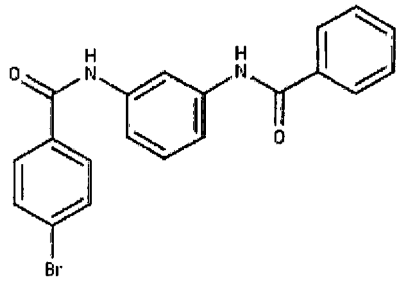 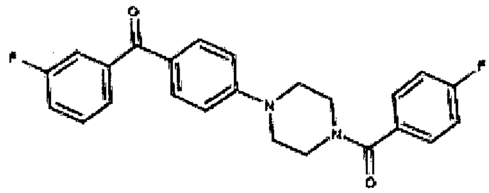 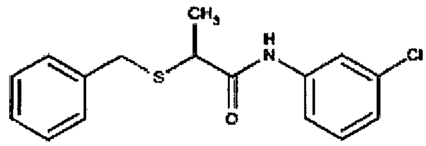 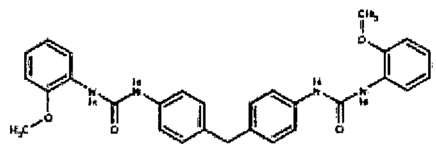 |
| 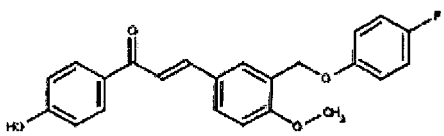 | 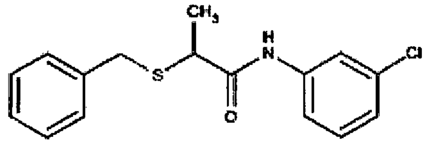 |

(continued)

| | |
|---|---|
| 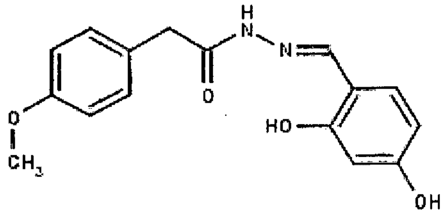 | 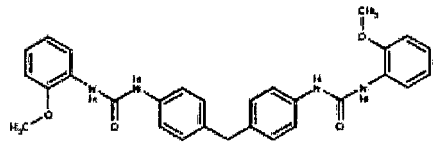 |
| 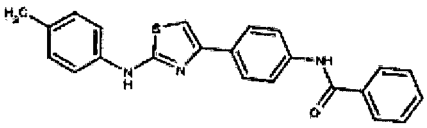 | 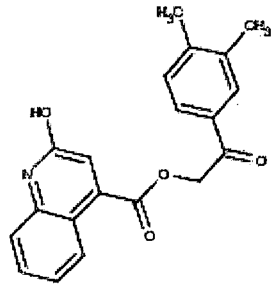 |
| 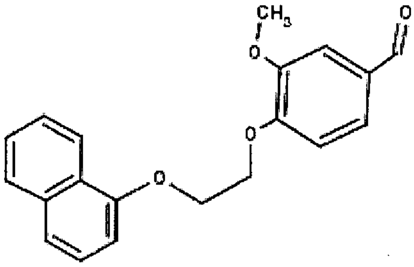 | 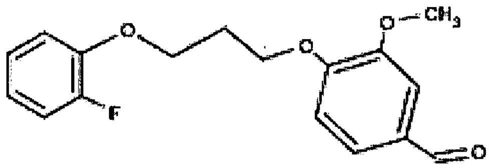 |
| 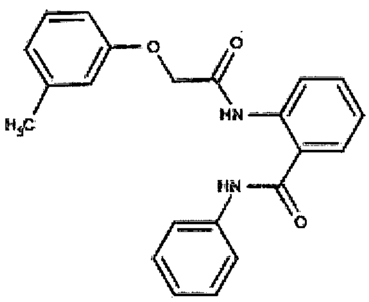 | 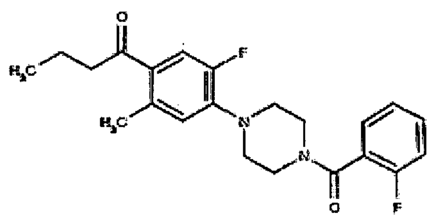 |

(continued)

| | |
|---|---|
| 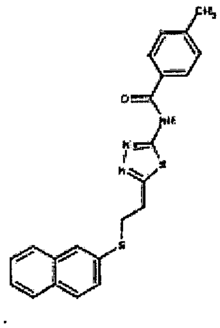 | 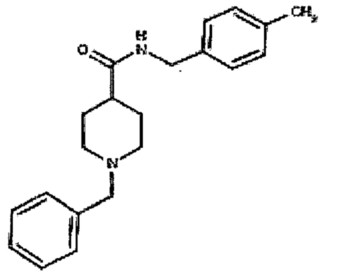 |
| 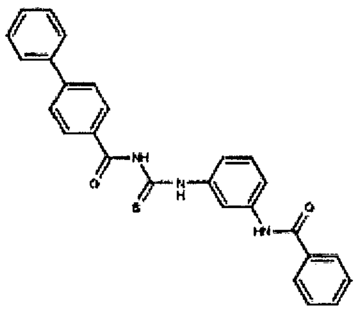 | 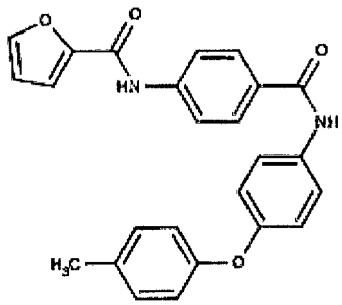 |
| 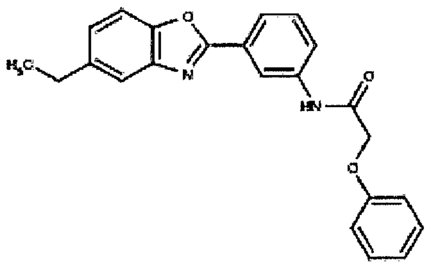 | 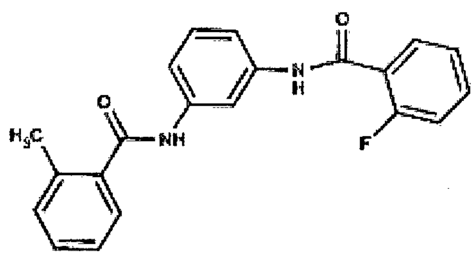 |
| 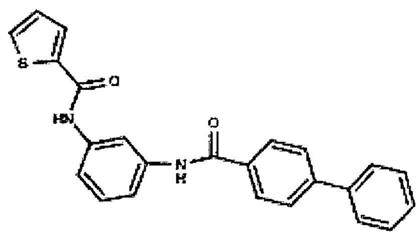 | 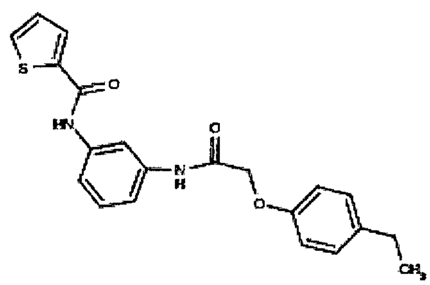 |

(continued)

| | |
|---|---|
| 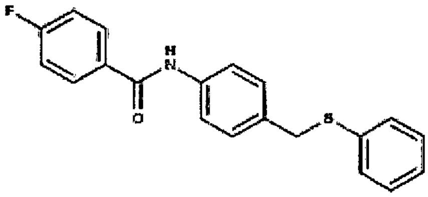 | 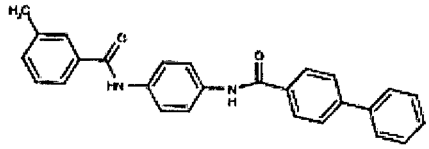 |
| 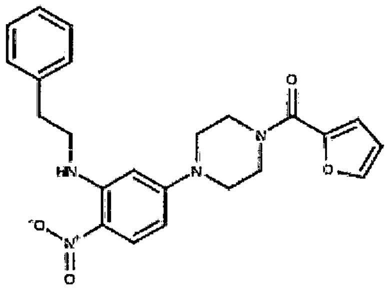 | 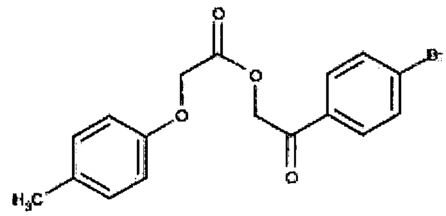 |
| 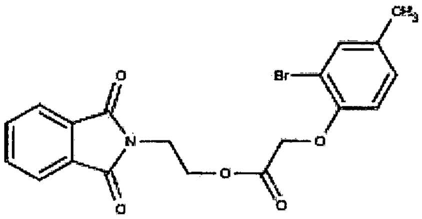 | 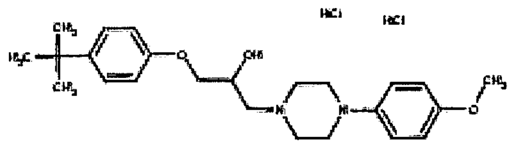 |
| 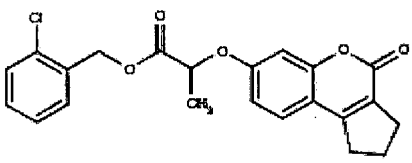 | 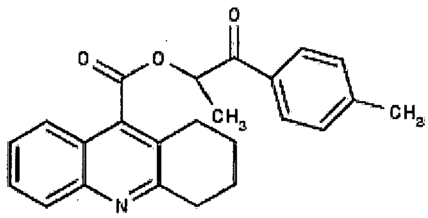 |

(continued)

| | |
|---|---|
| 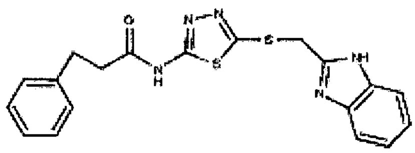 | 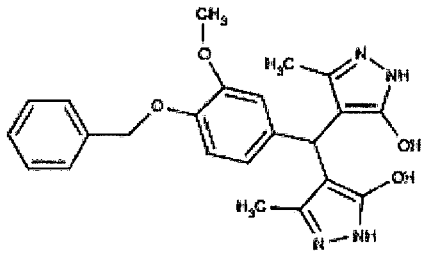 |
| 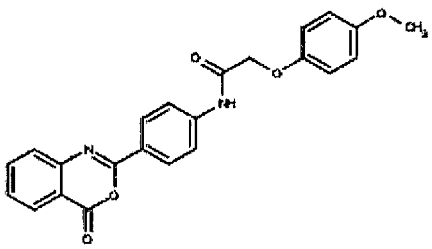 | 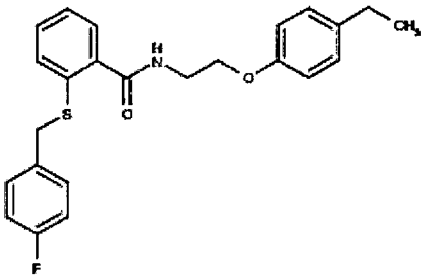 |
| 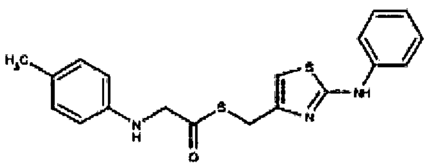 | 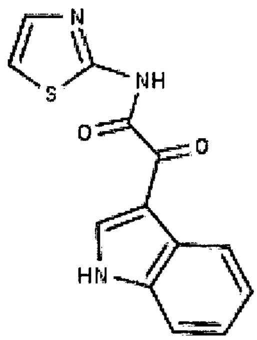 |
| 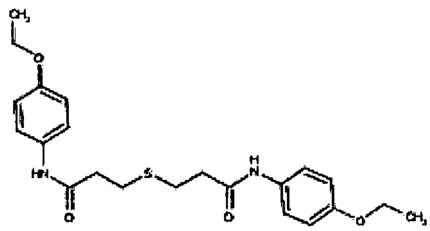 | 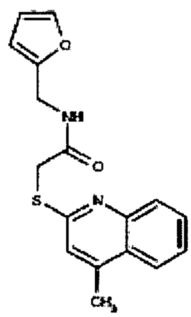 |

(continued)

| | |
|---|---|
| 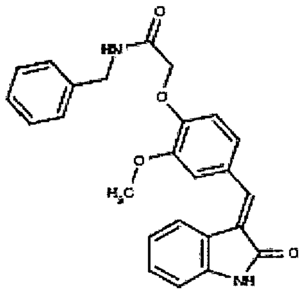 | 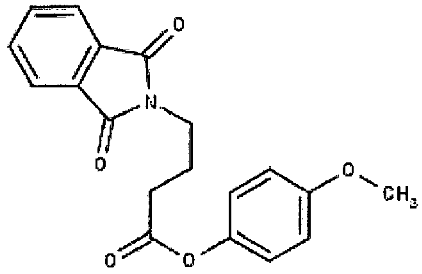 |
| 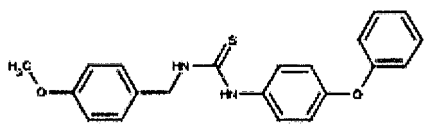 | 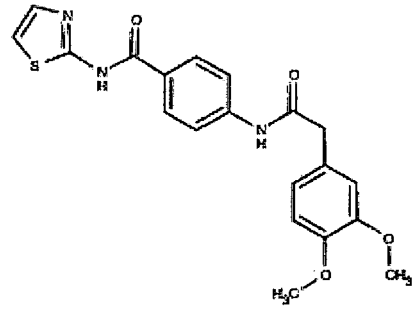 |
| 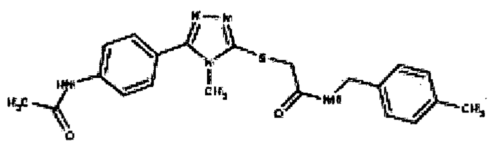 | 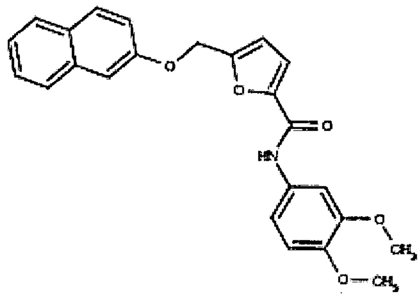 |
| 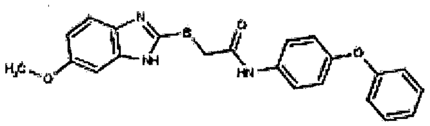 | 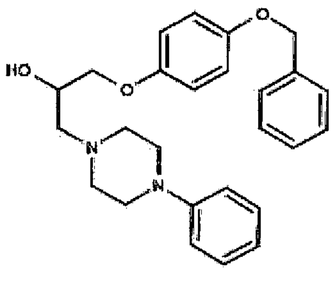 |

(continued)

| | |
|---|---|
| 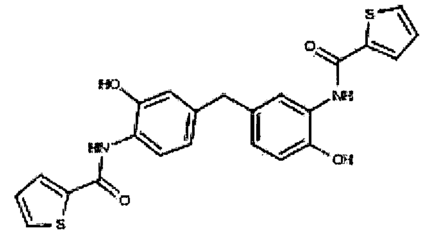 | 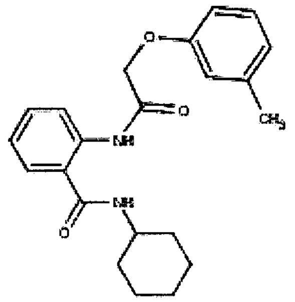 |
| 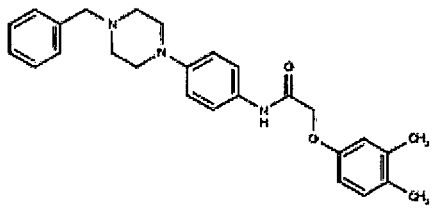 | 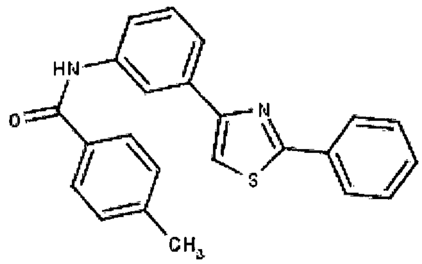 |
| 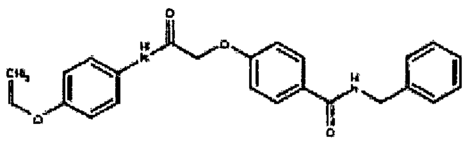 | 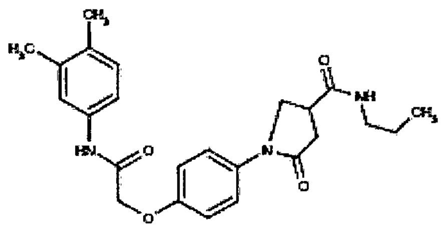 |
| 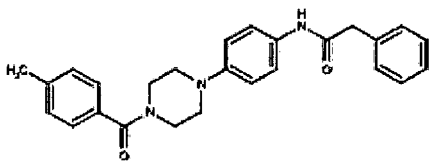 7683532 | 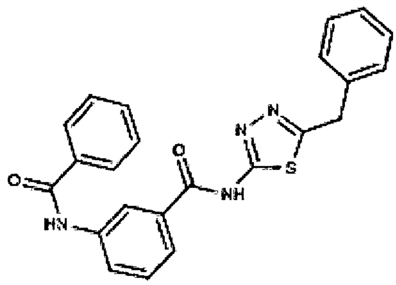 |
| 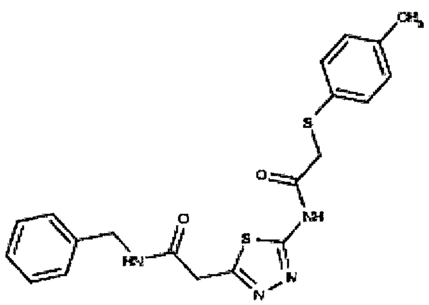 | 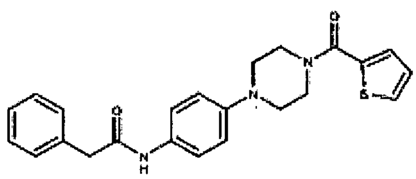 |

(continued)

| | |
|---|---|
| 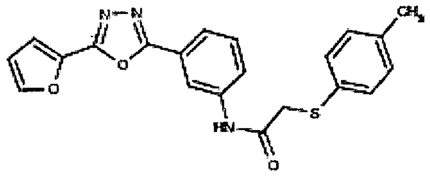 | 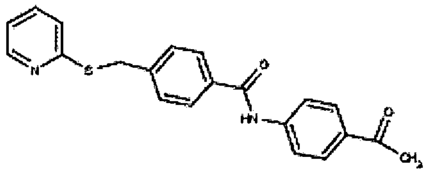 |
| 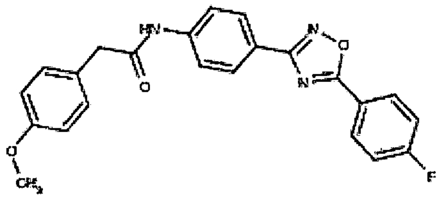 | 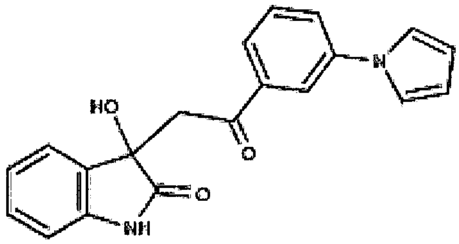 |
| 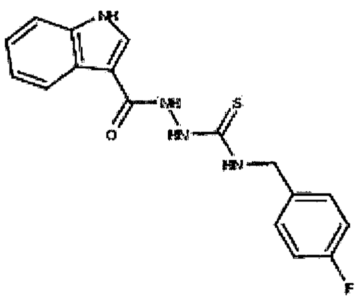 | 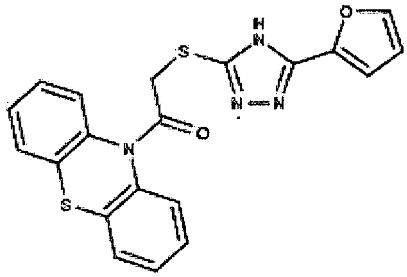 |
| 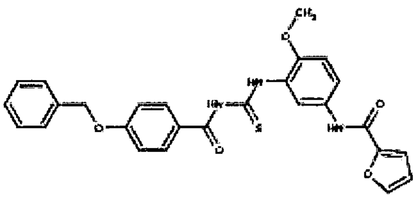 | 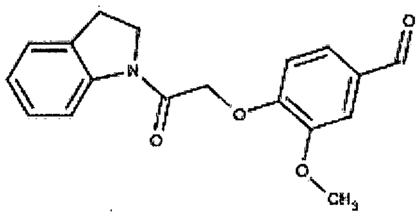 |

(continued)

| | |
|---|---|
| 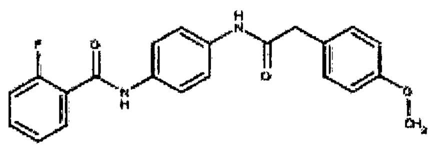 | 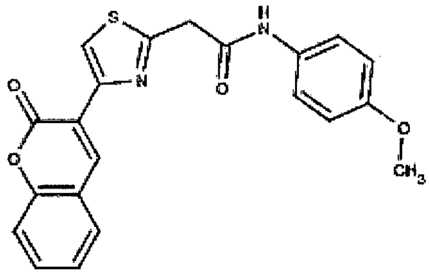 |
| 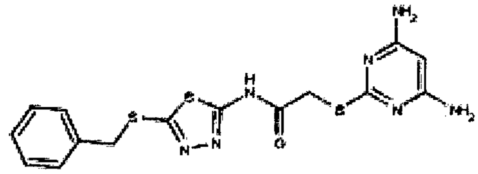 | 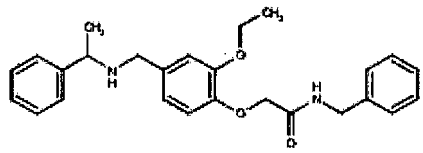 |
| 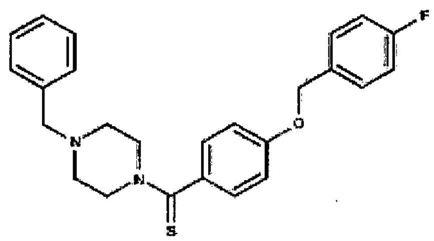 | 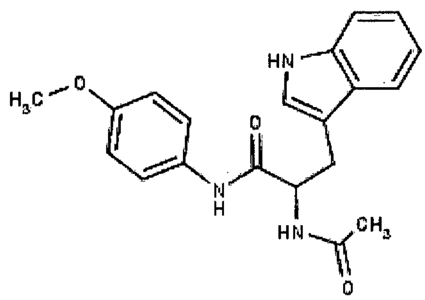 |
| 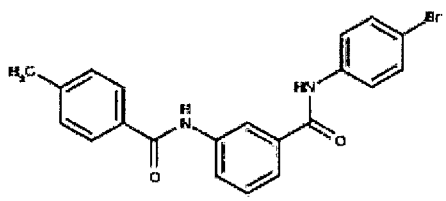 | 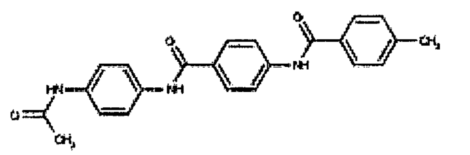 |
| 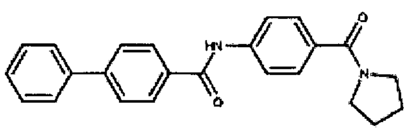 7944729 | 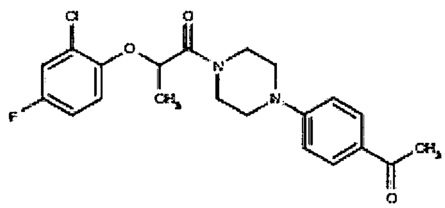 |

(continued)

| | |
|---|---|
| 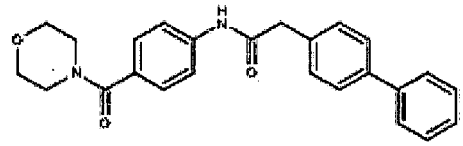 | 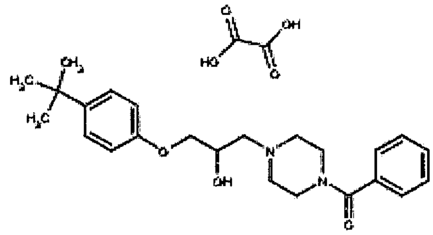 |
| 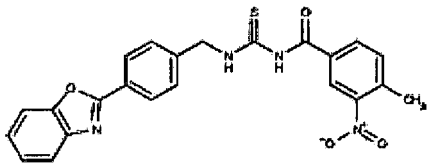 | 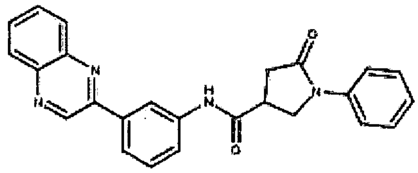 |
| 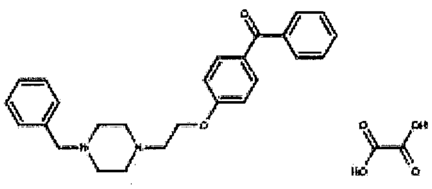 | 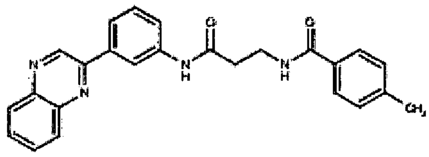 |
| 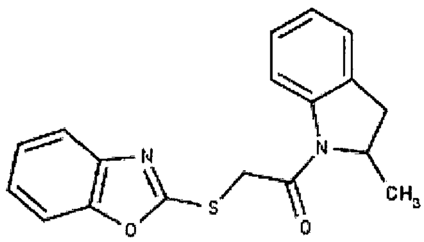 | 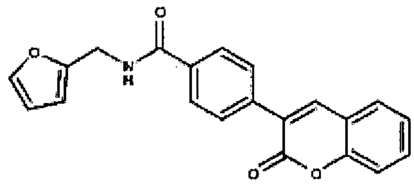 |
| 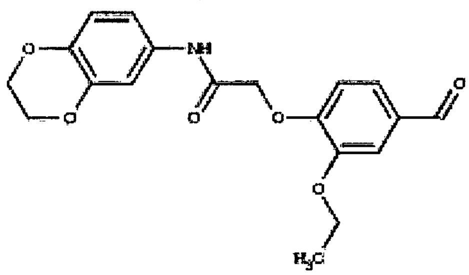 | 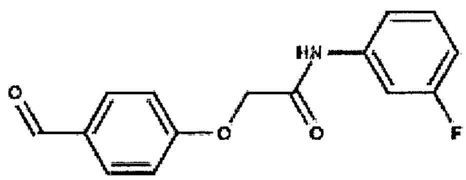 |

(continued)

| | |
|---|---|
| 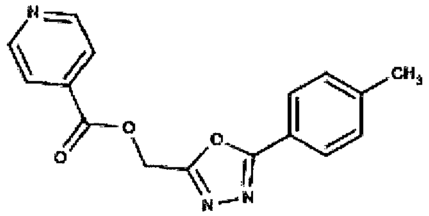 | 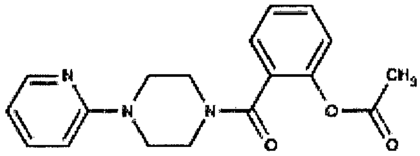 |
| 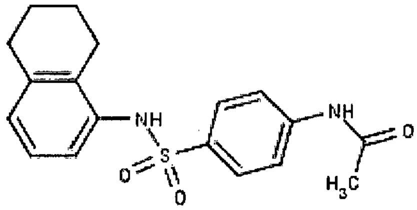 | 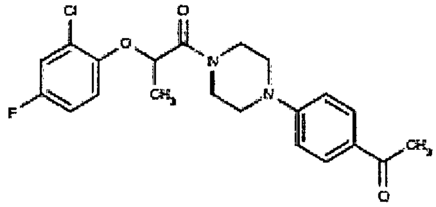 7946868 |
| 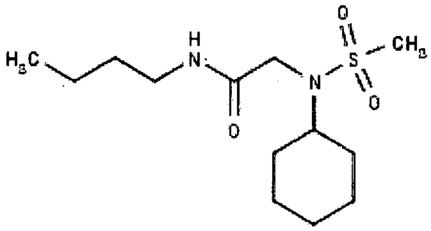 7942981 | |

## X. *Methods of treatment*

The references to methods of treatment in the following paragraphs are to be interpreted as references to the compounds, pharmaceutical compositions and medicaments of the present invention for use in a method for treatment of the human (or animal) body by therapy (or for diagnosis), respectively.

Agents of the present invention or the referential agents are useful for treating cells in which the cell death signal is downregulated and the affected cell has an inappropriately diminished propensity for cell death, which is referred to herein as being in a "decreased apoptotic state." Disclosed are methods for the administration to a subject of a therapeutically effective amount of an agent to treat an apoptosis-associated disease in which it is desirable to induce apoptosis in certain types of cells, such as virus-infected or autoantibody-expressing cells. Typically, the agent is substantially purified prior to administration. The subject can be an animal, including but not limited to, cows, pigs, horses, chickens, cats, dogs, and the like, and is typically a mammal, and in a particular embodiment human. In another specific embodiment, a non-human mammal is the subject.

The present invention provides for both prophylactic and therapeutic methods of treating a subject at risk of (or susceptible to) a disorder or having a disorder associated with aberrant (e.g., insufficient or excessive) BCL-2 family member expression or activity (e.g., extrinsic or intrinsic apoptotic pathway abnormalities). As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes, antisense oligonucleotides, other nucleic acid compositions, and combinations thereof.

BCL-2 type disorders can be caused, at least in part, by an abnormal level of one or more BCL-2 family members (e.g., over or under expression of BCL-2), or by the presence of one or more BCL-2 family members exhibiting abnormal activity. As such, the invention is directed to the reduction in the level and/or activity of the BCL-2 family member or the enhancement of the level and/or activity of the BCL-2 family member, which would bring about the amelioration of disorder symptoms. For example, a tumor maintained by excessive levels of an anti-apoptotic protein such as BCL-2, can be treated with a BAX activating modulator compound in order to circumvent apoptotic blockade and directly induce BAX-mediated apoptosis.

The compound of the invention or the referential compounds can be used to treat and prevent cancers and neoplastic conditions. As used herein, the terms "cancer", "hyperproliferative" and "neoplastic" refer to cells having the capacity for autonomous growth and defective cell death, i.e., an abnormal state or condition characterized by rapidly proliferating cell growth and/or apoptotic blockade. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

Examples of cellular proliferative and/or differentiative disorders include cancer, e.g., carcinoma, sarcoma, or metastatic disorders. The compounds can act as novel therapeutic agents for controlling breast cancer, ovarian cancer, colon cancer, lung cancer, metastasis of such cancers and the like. A metastatic tumor can arise from a multitude of primary tumor types, including but not limited to those of breast, lung, liver, colon and ovarian origin.

Examples of cancers or neoplastic conditions include, but are not limited to, a fibrosarcoma, myosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, gastric cancer, esophageal cancer, rectal cancer, pancreatic cancer, ovarian cancer, prostate cancer, uterine cancer, cancer of the head and neck, skin cancer, brain cancer, squamous cell carcinoma, sebaceous gland carcinoma, papillary carcinoma, papillary adenocarcinoma, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular cancer, small cell lung carcinoma, non-small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, lymphoma, or Kaposi sarcoma.

Examples of proliferative disorders include hematopoietic neoplastic disorders. As used herein, the term "hematopoietic neoplastic disorders" includes diseases involving hyperplastic/neoplastic cells of hematopoietic origin, e.g., arising from myeloid, lymphoid or erythroid lineages, or precursor cells thereof. Preferably, the diseases arise from poorly differentiated acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) Crit Rev. in Oncol./Hemotol. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Stemberg disease.

Examples of cellular proliferative and/or differentiative disorders of the breast include, but are not limited to, proliferative breast disease including, e.g., epithelial hyperplasia, sclerosing adenosis, and small duct papillomas; tumors, e.g., stromal tumors such as fibroadenoma, phyllodes tumor, and sarcomas, and epithelial tumors such as large duct papilloma; carcinoma of the breast including in situ (noninvasive) carcinoma that includes ductal carcinoma in situ (including Paget's disease) and lobular carcinoma in situ, and invasive (infiltrating) carcinoma including, but not limited to, invasive ductal carcinoma, invasive lobular carcinoma, medullary carcinoma, colloid (mucinous) carcinoma, tubular carcinoma, and invasive papillary carcinoma, and miscellaneous malignant neoplasms. Disorders in the male breast include, but are not limited to, gynecomastia and carcinoma.

Examples of cellular proliferative and/or differentiative disorders of the lung include, but are not limited to, bronchogenic carcinoma, including paraneoplastic syndromes, bronchioloalveolar carcinoma, neuroendocrine tumors, such as bronchial carcinoid, miscellaneous tumors, and metastatic tumors; pathologies of the pleura, including inflammatory pleural effusions, noninflammatory pleural effusions, pneumothorax, and pleural tumors, including solitary fibrous tumors (pleural fibroma) and malignant mesothelioma.

Examples of cellular proliferative and/or differentiative disorders of the colon include, but are not limited to, non-neoplastic polyps, adenomas, familial syndromes, colorectal carcinogenesis, colorectal carcinoma, and carcinoid tumors.

Examples of cellular proliferative and/or differentiative disorders of the liver include, but are not limited to, nodular hyperplasias, adenomas, and malignant tumors, including primary carcinoma of the liver and metastatic tumors.

Examples of cellular proliferative and/or differentiative disorders of the ovary include, but are not limited to, ovarian tumors such as, tumors of coelomic epithelium, serous tumors, mucinous tumors, endometeriod tumors, clear cell adenocarcinoma, cystadenofibroma, brenner tumor, surface epithelial tumors; germ cell tumors such as mature (benign) teratomas, monodermal teratomas, immature malignant teratomas, dysgerminoma, endodermal sinus tumor,

choriocarcinoma; sex cord-stomal tumors such as, granulosa-theca cell tumors, thecomafibromas, androblastomas, hill cell tumors, and gonadoblastoma; and metastatic tumors such as Krukenberg tumors.

The referential compounds described herein can also be used to treat or prevent conditions charaterised by overactive cell death or cellular death due to physiologic insult etc. Some examples of conditions characterized by premature or unwanted cell deth are or alternatively unwanted or excessive cellular proliferation include, but are not limited to ischemia, hypocellular/hypoplastic, acellular/aplastic, or hypercellular/hyperplastic conditions. Some examples include hematologic disorders including but not limited to fanconi anemia, aplastic anemia, thalaessemia, congenital neutropenia, myelodysplasia.

Compounds that act to decrease apoptosis can be used to treat disorders associated with an undesirable level of cell death. Thus, the anti-apoptotic compounds of the invention can be used to treat disorders such as those that lead to cell death associated with viral infection, e.g., infection associated with infection with human immunodeficiency virus (HIV). A wide variety of neurological diseases are characterized by the gradual loss of specific sets of neurons, and the anti-apoptotic peptides of the infection can be used in the treatment of these disorders. Such disorders include Alzheimer's disease, Parkinson's disease, amyotrophic lateral sclerosis (ALS) retinitis pigmentosa, spinal muscular atrophy, and various forms of cerebellar degeneration. The cell loss in these diseases does not induce an inflammatory response, and apoptosis appears to be the mechanism of cell death. In addition, a number of hematologic diseases are associated with a decreased production of blood cells. These disorders include anemia associated with chronic disease, aplastic anemia, chronic neutropenia, and the myelodysplastic syndromes. Disorders of blood cell production, such as myelodysplastic syndrome and some forms of aplastic anemia, are associated with increased apoptotic cell death within the bone marrow. These disorders could result from the activation of genes that promote apoptosis, acquired deficiencies in stromal cells or hematopoietic survival factors, or the direct effects of toxins and mediators of immune responses. Two common disorders associated with cell death are myocardial infarctions and stroke. In both disorders, cells within the central area of ischemia, which is produced in the event of acute loss of blood flow, appear to die rapidly as a result of necrosis. However, outside the central ischemic zone, cells die over a more protracted time period and morphologically appear to die by apoptosis. The anti-apoptotic compounds of the invention can be used to treat all such disorders associated with undesirable cell death. Some examples of immunologic disorders that can be treated with the compunds described herein include but are not limited to organ transplant rejection, arthritis, lupus, IBD, Crohn's disease, asthma, multiple sclerosis, diabetes etc.

Some examples of neurologic disorders that can be treated with the polypeptides described herein include but are not limited to Alzheimer's Disease, Down's Syndrome, Dutch Type Hereditary Cerebral Hemorrhage Amyloidosis, Reactive Amyloidosis, Familial Amyloid Nephropathy with Urticaria and Deafness, Muckle-Wells Syndrome, Idiopathic Myeloma; Macroglobulinemia-Associated Myeloma, Familial Amyloid Polyneuropathy, Familial Amyloid Cardiomyopathy, Isolated Cardiac Amyloid, Systemic Senile Amyloidosis, Adult Onset Diabetes, Insulinoma, Isolated Atrial Amyloid, Medullary Carcinoma of the Thyroid, Familial Amyloidosis, Hereditary Cerebral Hemorrhage With Amyloidosis, Familial Amyloidotic Polyneuropathy, Scrapie, Creutzfeldt-Jacob Disease, Gerstmann Straussler-Scheinker Syndrome, Bovine Spongiform Encephalitis, a Prion-mediated disease, and Huntington's Disease.

Some examples of endocrinologic disorders that can be treated with the polypeptides described herein include but are not limited to diabetes, hypthyroidism, hyopituitarism, hypoparathyroidism, hypogonadism, etc.

Examples of cardiovascular disorders (e.g., inflammatory disorders) that can be treated or prevented with the compounds and methods of the invention include, but are not limited to, atherosclerosis, myocardial infarction, stroke, thrombosis, aneurism, heart failure, ischemic heart disease, angina pectoris, sudden cardiac death, hypertensive heart disease; non-coronary vessel disease, such as arteriolosclerosis, small vessel disease, nephropathy, hypertriglyceridemia, hypercholesterolemia, hyperlipidemia, xanthomatosis, asthma, hypertension, emphysema and chronic pulmonary disease; or a cardiovascular condition associated with interventional procedures ("procedural vascular trauma"), such as restenosis following angioplasty, placement of a shunt, stent, synthetic or natural excision grafts, indwelling catheter, valve or other implantable devices. Preferred cardiovascular disorders include atherosclerosis, myocardial infarction, aneurism, and stroke.

## XI. *Administration of Modulators*

The references to methods of treatment in the following paragraphs are to be interpreted as references to the compounds, pharmaceutical compositions and medicaments of the present invention for use in a method for treatment of the human (or animal) body by therapy (or for diagnosis), respectively.

In one embodiment, the compound of the invention or the referential compounds are administered as monotherapy for the prevention, treatment, and/or management of a disorder disclosed herein. The compound of the invention is for use in a method of treating cancer. The prophylactic treatment is indicated for referential purposes.

Disclosed is a method of preventing, treating, and/or managing cancer in a patient (e.g., a human patient), the method comprising administering to the patient a prophylactically effective regimen or a therapeutically effective regimen,

the regimen comprising administering to the patient a compound of the invention or a composition of the invention, wherein the patient has been diagnosed with cancer. The amount of a compound of the invention used in the prophylactic and/or therapeutic regimens which will be effective in the prevention, treatment, and/or management of cancer can be based on the currently prescribed dosage of the compound as well as assessed by methods disclosed herein.

In one embodiment of this aspect, the patient has received or is receiving another therapy. In another embodiment of this aspect, the patient has not previously received a therapy for the prevention, treatment, and/or management of the cancer.

The medical practitioner can diagnose the patient using any of the conventional cancer screening methods including, but not limited to physical examination (e.g., prostate examination, breast examination, lymph nodes examination, abdominal examination, skin surveillance), visual methods (e.g., colonoscopy, bronchoscopy, endoscopy), PAP smear analyses (cervical cancer), stool guaiac analyses, blood tests (e.g., complete blood count (CBC) test), blood chemistries including liver function tests, prostate specific antigen (PSA) test, carcinoembryonic antigen (CEA) test, cancer antigen (CA)-125 test, alpha-fetoprotein (AFP)), karyotyping analyses, bone marrow analyses (e.g., in cases of hematological malignancies), histology, cytology, a sputum analysis and imaging methods (e.g., computed tomography (CT), magnetic resonance imaging (MRI), ultrasound, X-ray imaging, mammograph imaging, bone scans).

Another aspect of the invention relates to the compound of the invention for use in a method of preventing, treating, and/or managing a solid tumor in a patient *(e.g.,* a human patient), the method comprising administering to a patient in need thereof a prophylactically effective regimen or a therapeutically effective regimen, the regimen comprising administering to the patient a compound or composition of the invention wherein the patient has been diagnosed with a solid tumor, and wherein the patient has undergone a primary therapy to reduce the bulk of the tumor.

Another aspect of the invention relates to the compound of the invention for use in a method of preventing, treating, and/or managing cancer, the method comprising administering to a patient in need thereof a prophylactically effective regimen or a therapeutically effective regimen, the regimen comprising administering to the patient a compound of the invention (as described above), or a pharmaceutically acceptable salt thereof wherein the patient received another therapy. In some embodiments, the prior therapy is, for example, chemotherapy, radioimmunotherapy, toxin therapy, prodrug-activating enzyme therapy, antibody therapy, surgical therapy, immunotherapy, radiation therapy, targeted therapy or any combination thereof.

In some embodiments, the prior therapy has failed in the patient. In some embodiments, the therapeutically effective regimen comprising administration of a compound of the invention is administered to the patient immediately after patient has undergone the prior therapy. For instance, in certain embodiments, the outcome of the prior therapy may be unknown before the patient is administered a compound of the invention.

Another aspect of the invention relates to the compound of the invention for use in a method of preventing, treating, and/or managing cancer in a patient (e.g., a human patient), the method comprising administering to a patient in need thereof a prophylactically effective regimen or a therapeutically effective regimen, the regimen comprising administering to the patient a compound or composition of the invention, wherein the compound or composition of the invention is administered at a dose that is lower than the human equivalent dosage (HED) of the no observed adverse effect level (NOAEL) over a period of three months, four months, six months, nine months, 1 year, 2 years, 3 years, 4 years or more. The NOAEL, as determined in animal studies, is useful in determining the maximum recommended starting dose for human clinical trials. For instance, the NOAELs can be extrapolated to determine human equivalent dosages. Typically, such extrapolations between species are conducted based on the doses that are normalized to body surface area (*i.e*., $mg/m^2$). In specific embodiments, the NOAELs are determined in mice, hamsters, rats, ferrets, guinea pigs, rabbits, dogs, primates, primates (monkeys, marmosets, squirrel monkeys, baboons), micropigs or minipigs. For a discussion on the use of NOAELs and their extrapolation to determine human equivalent doses, *see* Guidance for Industry Estimating the Maximum Safe Starting Dose in Initial Clinical Trials for Therapeutics in Adult Healthy Volunteers, U.S. Department of Health and Human Services Food and Drug Administration Center for Drug Evaluation and Research (CDER), Pharmacology and Toxicology, July 2005.

In certain embodiments, the regimens comprise administering a prophylactically effective regimen and/or a therapeutically effective regimen, wherein the regimen results in a reduction in the cancer cell population in the patient. In one embodiment, the patient undergoing the regimen is monitored to determine whether the regimen has resulted in a reduction in the cancer cell population in the patient.

Typically, the monitoring of the cancer cell population is conducted by detecting the number or amount of cancer cells in a specimen extracted from the patient. Methods of detecting the number or amount of cancer cells in a specimen are known in the art. This monitoring step is typically performed at least 1, 2, 4, 6, 8, 10, 12, 14, 15, 16, 18, 20, or 30 days after the patient begins receiving the regimen.

In some embodiments, the specimen may be a blood specimen, wherein the number or amount of cancer cells per unit of volume (e.g., 1 mL) or other measured unit (e.g., per unit field in the case of a histological analysis) is quantitated. The cancer cell population, in certain embodiments, can be determined as a percentage of the total blood cells.

In other embodiments, the specimen extracted from the patient is a tissue specimen (e.g., a biopsy extracted from suspected cancerous tissue), where the number or amount of cancer cells can be measured, for example, on the basis of the number or amount of cancer cells per unit weight of the tissue.

The number or amount of cancer cells in the extracted specimen can be compared with the numbers or amounts of cancer cells measured in reference samples to assess the efficacy of the regimen and amelioration of the cancer under therapy. In one embodiment, the reference sample is a specimen extracted from the patient undergoing therapy, wherein the specimen from the patient is extracted at an earlier time point (e.g., prior to receiving the regimen, as a baseline reference sample, or at an earlier time point while receiving the therapy). In another embodiment, the reference sample is extracted from a healthy, noncancer-afflicted patient.

In other embodiments the cancer cell population in the extracted specimen can be compared with a predetermined reference range. In a specific embodiment, the predetermined reference range is based on the number or amount of cancer cells obtained from a population(s) of patients suffering from the same type of cancer as the patient undergoing the therapy.

If the reduction in the cancer cell population is judged too small upon comparing the number, amount, or percentage of cancer cells in the specimen extracted from the patients undergoing therapy with the reference specimen, then the medical practitioner has a number of options to adjust the therapeutic regimen. For instance, the medical practitioner can then either increase the dosage of the compound or composition of the invention administered, the frequency of the administration, the duration of administration, or any combination thereof. In a specific embodiment, after the determination is made, a second effective amount of a compound or composition of the invention can be administered to the patient.

In other embodiments, the compound for the use of the invention comprises administering the compound or composition of the invention, wherein the regimen results in a reduction in the number, amount, or percentage of cancer cells and a reduction in the number, amount, or percentage of cancer cells in the patient.

The amount of the compound of the invention used in the prophylactic and/or therapeutic regimens which will be effective in the prevention, treatment, and/or management of cancer can be based on the currently prescribed dosage of the compound as well as assessed by methods disclosed herein and known in the art. The frequency and dosage will vary also according to factors specific for each patient depending on the specific compounds administered, the severity of the cancerous condition, the route of administration, as well as age, body, weight, response, and the past medical history of the patient. For example, the dosage of the compound of the invention which will be effective in the treatment, prevention, and/or management of cancer can be determined by administering the compound to an animal model such as, *e.g.,* the animal models disclosed herein or known to those skilled in the art. In addition, *in vitro* assays may optionally be employed to help identify optimal dosage ranges.

In some embodiments, the prophylactic and/or therapeutic regimens comprise titrating the dosages administered to the patient so as to achieve a specified measure of therapeutic efficacy. Such measures include a reduction in the cancer cell population in the patient.

In certain embodiments, the dosage of the compound of the invention in the prophylactic and/or therapeutic regimen is adjusted so as to achieve a reduction in the number or amount of cancer cells found in a test specimen extracted from a patient after undergoing the prophylactic and/or therapeutic regimen, as compared with a reference sample. Here, the reference sample is a specimen extracted from the patient undergoing therapy, wherein the specimen is extracted from the patient at an earlier time point. In one embodiment, the reference sample is a specimen extracted from the same patient, prior to receiving the prophylactic and/or therapeutic regimen. In specific embodiments, the number or amount of cancer cells in the test specimen is at least 2%, 5%, 10%, 15%, 20%, 30%, 40%, 50%, 60%, 70%, 80%, 90%, 95% or 99% lower than in the reference sample.

In some embodiments, the dosage of the compound of the invention in the prophylactic and/or therapeutic regimen is adjusted so as to achieve a number or amount of cancer cells that falls within a predetermined reference range. In these embodiments, the number or amount of cancer cells in a test specimen is compared with a predetermined reference range.

In other embodiments, the dosage of the compound of the invention in prophylactic and/or therapeutic regimen is adjusted so as to achieve a reduction in the number or amount of cancer cells found in a test specimen extracted from a patient after undergoing the prophylactic and/or therapeutic regimen, as compared with a reference sample, wherein the reference sample is a specimen is extracted from a healthy, noncancer-afflicted patient. In specific embodiments, the number or amount of cancer cells in the test specimen is at least within 60%, 50%, 40%, 30%, 20%, 15%, 10%, 5%, or 2% of the number or amount of cancer cells in the reference sample.

In treating certain human patients having solid tumors, extracting multiple tissue specimens from a suspected tumor site may prove impracticable. In these embodiments, the dosage of the compound of the invention in the prophylactic and/or therapeutic regimen for a human patient is extrapolated from doses in animal models that are effective to reduce the cancer population in those animal models. In the animal models, the prophylactic and/or therapeutic regimens are adjusted so as to achieve a reduction in the number or amount of cancer cells found in a test specimen extracted from

an animal after undergoing the prophylactic and/or therapeutic regimen, as compared with a reference sample. The reference sample can be a specimen extracted from the same animal, prior to receiving the prophylactic and/or therapeutic regimen. In specific embodiments, the number or amount of cancer cells in the test specimen is at least 2%, 5%, 10%, 15%, 20%, 30%, 40%, 50% or 60% lower than in the reference sample. The doses effective in reducing the number or amount of cancer cells in the animals can be normalized to body surface area (*e.g.*, $mg/m^2$) to provide an equivalent human dose.

The prophylactic and/or therapeutic regimens disclosed herein comprise administration of the compound of the invention or pharmaceutical compositions thereof to the patient in a single dose or in multiple doses *(e.g.,* 1, 2, 3, 4, 5, 6, 7, 8, 10, 15, 20, or more doses).

In one embodiment, the prophylactic and/or therapeutic regimens comprise administration of the compound of the invention or pharmaceutical compositions thereof in multiple doses. When administered in multiple doses, the compound or pharmaceutical compositions are administered with a frequency and in an amount sufficient to prevent, treat, and/or manage the condition. In one embodiment, the frequency of administration ranges from once a day up to about once every eight weeks. In another embodiment, the frequency of administration ranges from about once a week up to about once every six weeks. In another embodiment, the frequency of administration ranges from about once every three weeks up to about once every four weeks.

Generally, the dosage of the compound of the invention administered to a subject to prevent, treat, and/or manage cancer is in the range of 0.01 to 500 mg/kg, and more typically, in the range of 0.1 mg/kg to 100 mg/kg, of the subject's body weight. In one embodiment, the dosage administered to a subject is in the range of 0.1 mg/kg to 50 mg/kg, or 1 mg/kg to 50 mg/kg, of the subject's body weight, more preferably in the range of 0.1 mg/kg to 25 mg/kg, or 1 mg/kg to 25 mg/kg, of the patient's body weight.

In a specific embodiment, the dosage of the compound of the invention administered to a subject to prevent, treat, and/or manage cancer in a patient is 500 mg/kg or less, preferably 250 mg/kg or less, 100 mg/kg or less, 95 mg/kg or less, 90 mg/kg or less, 85 mg/kg or less, 80 mg/kg or less, 75 mg/kg or less, 70 mg/kg or less, 65 mg/kg or less, 60 mg/kg or less, 55 mg/kg or less, 50 mg/kg or less, 45 mg/kg or less, 40 mg/kg or less, 35 mg/kg or less, 30 mg/kg or less, 25 mg/kg or less, 20 mg/kg or less, 15 mg/kg or less, 10 mg/kg or less, 5 mg/kg or less, 2.5 mg/kg or less, 2 mg/kg or less, 1.5 mg/kg or less, or 1 mg/kg or less of a patient's body weight.

In another specific embodiment, the dosage of the compound of the invention administered to a subject to prevent, treat, and/or manage cancer in a patient is a unit dose of 0.1 to 50 mg, 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 m g, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

In a specific embodiment, the dosage of the compound of the invention administered to a subject to prevent, treat, and/or manage cancer in a patient is in the range of 0.01 to 10 $g/m^2$, and more typically, in the range of 0.1 $g/m^2$ to 7.5 $g/m^2$, of the subject's body weight. In one embodiment, the dosage administered to a subject is in the range of 0.5 $g/m^2$ to 5 $g/m^2$, or 1 $g/m^2$ to 5 $g/m^2$ of the subject's body's surface area.

In other embodiments, the prophylactic and/or therapeutic regimen comprises administering to a patient one or more doses of an effective amount of the compound of the invention, wherein the dose of an effective amount achieves a plasma level of at least 0.1 $\mu$g/mL, at least 0.5 $\mu$g/mL, at least 1 $\mu$g/mL, at least 2 $\mu$g/mL, at least 5 $\mu$g/mL, at least 6 $\mu$g/mL, at least 10 $\mu$g/mL, at least 15 $\mu$g/mL, at least 20 $\mu$g/mL, at least 25 $\mu$g/mL, at least 50 $\mu$g/mL, at least 100 $\mu$g/mL, at least 125 $\mu$g/mL, at least 150 $\mu$g/mL, at least 175 $\mu$g/mL, at least 200 $\mu$g/mL, at least 225 $\mu$g/mL, at least 250 $\mu$g/mL, at least 275 $\mu$g/mL, at least 300 $\mu$g/mL, at least 325 $\mu$g/mL, at least 350 $\mu$g/mL, at least 375 $\mu$g/mL, or at least 400 $\mu$g/mL of the compound of the invention.

In other embodiments, the prophylactic and/or therapeutic regimen comprises administering to a patient a plurality of doses of an effective amount of the compound of the invention, wherein the plurality of doses maintains a plasma level of at least 0.1 $\mu$g/mL, at least 0.5 $\mu$g/mL, at least 1 $\mu$g/mL, at least 2 $\mu$g/mL, at least 5 $\mu$g/mL, at least 6 $\mu$g/mL, at least 10 $\mu$g/mL, at least 15 $\mu$g/mL, at least 20 $\mu$g/mL, at least 25 $\mu$g/mL, at least 50 $\mu$g/mL, at least 100 $\mu$g/mL, at least 125 $\mu$g/mL, at least 150 $\mu$g/mL, at least 175 $\mu$g/mL, at least 200 $\mu$g/mL, at least 225 $\mu$g/mL, at least 250 $\mu$g/mL, at least 275 $\mu$g/mL, at least 300 $\mu$g/mL, at least 325 $\mu$g/mL, at least 350 $\mu$g/mL, at least 375 $\mu$g/mL, or at least 400 $\mu$g/mL of the compound of the invention for at least 1 day, 1 month, 2 months, 3 months, 4 months, 5 months, 6 months, 7 months, 8 months, 9 months, 10 months, 11 months, 12 months, 15 months, 18 months, 24 months or 36 months.

In some embodiments, the prophylactic and/or therapeutic regimen comprises administration of the compound of the invention in combination with one or more additional anticancer therapeutics. Preferably, the dosages of the one or more additional anticancer therapeutics used in the combination therapy is lower than those which have been or are currently being used to prevent, treat, and/or manage cancer. The recommended dosages of the one or more additional anticancer therapeutics currently used for the prevention, treatment, and/or management of cancer can be obtained from any reference in the art including, but not limited to, Hardman et al., eds., Goodman & Gilman's The Pharmacological Basis Of Basis Of Therapeutics, 10th ed., Mc-Graw-Hill, New York, 2001; Physician's Desk Reference (60th ed., 2006).

The compound of the invention and the one or more additional anticancer therapeutics can be administered separately, simultaneously, or sequentially. In various embodiments, the compound of the invention and the additional anticancer therapeutic are administered less than 5 minutes apart, less than 30 minutes apart, less than 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more anticancer therapeutics are administered within the same patient visit.

In certain embodiments, the compound of the invention and the additional anticancer therapeutic are cyclically administered. Cycling therapy involves the administration of one anticancer therapeutic for a period of time, followed by the administration of a second anticancer therapeutic for a period of time and repeating this sequential administration, *i.e.,* the cycle, in order to reduce the development of resistance to one or both of the anticancer therapeutics, to avoid or reduce the side effects of one or both of the anticancer therapeutics, and/or to improve the efficacy of the therapies.

In a preferred embodiment, the anticancer therapeutics are administered concurrently to a subject in separate compositions. The combination anticancer therapeutics of the invention may be administered to a subject by the same or different routes of administration.

In a specific embodiment, cycling therapy involves the administration of a first anticancer therapeutic for a period of time, followed by the administration of a second anticancer therapeutic for a period of time, optionally, followed by the administration of a third anticancer therapeutic for a period of time and so forth, and repeating this sequential administration, *i.e.,* the cycle in order to reduce the development of resistance to one of the anticancer therapeutics, to avoid or reduce the side effects of one of the anticancer therapeutics, and/or to improve the efficacy of the anticancer therapeutics.

When a compound of the invention and the additional anticancer therapeutic are administered to a subject concurrently, the term "concurrently" is not limited to the administration of the anticancer therapeutics at exactly the same time, but rather, it is meant that they are administered to a subject in a sequence and within a time interval such that they can act together (e.g., synergistically to provide an increased benefit than if they were administered otherwise). For example, the anticancer therapeutics may be administered at the same time or sequentially in any order at different points in time; however, if not administered at the same time, they should be administered sufficiently close in time so as to provide the desired therapeutic effect, preferably in a synergistic fashion. The combination anticancer therapeutics of the invention can be administered separately, in any appropriate form and by any suitable route. When the components of the combination anticancer therapeutics are not administered in the same pharmaceutical composition, it is understood that they can be administered in any order to a subject in need thereof. For example, a compound of the invention can be administered prior to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of the additional anticancer therapeutic, to a subject in need thereof. In various embodiments, the anticancer therapeutics are administered 1 minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, the anticancer therapeutics are administered within the same office visit. In another embodiment, the combination anticancer therapeutics of the invention are administered at 1 minute to 24 hours apart.

## XII. ***Formulations***

The references to the compound of the invention and/or formulations comprising said compounds are to be interpreted as references to the compounds, pharmaceutical compositions and medicaments of the present invention for use in the claimed method for treatment of the human (or animal) body by therapy.

The present invention provides compositions that are suitable for veterinary and/or human administration (e.g., pharmaceutical compositions). The pharmaceutical compositions of the present invention can be in any form that allows for the composition to be administered to a subject, said subject preferably being an animal, including, but not limited to a human, mammal, or non-human animal, such as a cow, horse, sheep, pig, fowl, cat, dog, mouse, rat, rabbit, guinea pig, etc., and is more preferably a mammal, and most preferably a human.

The formulation of the compound of the invention used in the prophylactic and/or therapeutic regimens which will be effective in the prevention, treatment, and/or management of cancer can be based on the currently available formulation. Alternatively the compounds can be reformulated based on knowledge within the art and the teachings herein. For example, the compound may be in the form of a solid, liquid or gas (aerosol). Typical routes of administration may include, without limitation, oral, topical, parenteral, sublingual, rectal, vaginal, ocular, intradermal, intratumoral, intracerebral, intrathecal, and intranasal. Parenteral administration includes subcutaneous injections, intravenous, intramuscular, intraperitoneal, intrapleural, intrasternal injection or infusion techniques. In a specific embodiment, the compositions are administered parenterally. In a more specific embodiment, the compositions are administered intravenously. Pharmaceutical compositions of the invention can be formulated so as to allow a compound of the invention to be bioavailable upon administration of the composition to a subject. Compositions can take the form of one or more dosage units, where, for example, a tablet can be a single dosage unit, and a container of a compound of the invention in aerosol form can hold a plurality of dosage units.

Materials used in preparing the pharmaceutical compositions can be non-toxic in the amounts used. It will be evident to those of ordinary skill in the art that the optimal dosage of the active ingredient(s) in the pharmaceutical composition will depend on a variety of factors. Relevant factors include, without limitation, the type of subject (e.g., human), the overall health of the subject, the type of cancer the subject is in need of treatment of, the use of the composition as part of a multi-drug regimen, the particular form of the compound of the invention, the manner of administration, and the composition employed.

The pharmaceutically acceptable carrier or vehicle may be particulate, so that the compositions are, for example, in tablet or powder form. The carrier(s) can be liquid, with the compositions being, for example, an oral syrup or injectable liquid. In addition, the carrier(s) can be gaseous, so as to provide an aerosol composition useful in, e.g., inhalatory administration.

The term "carrier" refers to a diluent, adjuvant or excipient, with which the compound of the invention is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to a subject, the compounds of the invention and pharmaceutically acceptable carriers are sterile. Water is a preferred carrier when the compound of the invention is administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

The composition may be intended for oral administration, and if so, the composition is preferably in solid or liquid form, where semi-solid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more of the following can be present: binders such as ethyl cellulose, carboxymethylcellulose, microcrystalline cellulose, or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin, a flavoring agent such as peppermint, methyl salicylate or orange flavoring, and a coloring agent.

When the pharmaceutical composition is in the form of a capsule, e.g., a gelatin capsule, it can contain, in addition to materials of the above type, a liquid carrier such as polyethylene glycol, cyclodextrin or a fatty oil.

The pharmaceutical composition can be in the form of a liquid, e.g., an elixir, syrup, solution, emulsion or suspension. The liquid can be useful for oral administration or for delivery by injection. When intended for oral administration, a composition can comprise one or more of a sweetening agent, preservatives, dye/colorant and flavour enhancer. In a composition for administration by injection, one or more of a surfactant, preservative, wetting agent, dispersing agent, suspending agent, buffer, stabilizer and isotonic agent can also be included.

The liquid compositions of the invention, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. A parenteral composition can be enclosed in an ampoule, a disposable syringe or a multiple-dose vial made of glass, plastic or other material. Physiological saline is a

preferred adjuvant. An injectable composition is preferably sterile.

The pharmaceutical compositions comprise an effective amount of the compound of the invention such that a suitable dosage will be obtained. The pharmaceutical compositions may comprise the known effective amount of the compounds as currently prescribed for their respective disorders.

Typically, the effective amount is at least 0.01% of the compound of the invention by weight of the composition. When intended for oral administration, this amount can be varied to be between 0.1% and 80% by weight of the composition. Preferred oral compositions can comprise from between 4% and 50% of the compound of the invention by weight of the composition. Preferred compositions of the present invention are prepared so that a parenteral dosage unit contains from between 0.01% and 2% by weight of the compound of the invention.

The compound of the invention can be administered by any convenient route, for example, by infusion or bolus injection, by absorption through epithelial or mucocutaneous linings (e.g., oral mucosa, rectal and intestinal mucosa, etc.). Administration can be systemic or local. Various delivery systems are known, e.g., microparticles, microcapsules, capsules, etc., and may be useful for administering a compound of the invention. In certain embodiments, more than one compound of the invention is administered to a subject. Methods of administration may include, but are not limited to, oral administration and parenteral administration; parenteral administration including, but not limited to, intradermal, intramuscular, intraperitoneal, intravenous, subcutaneous; intranasal, epidural, sublingual, intranasal, intracerebral, intraventricular, intrathecal, intravaginal, transdermal, rectally, by inhalation, or topically to the ears, nose, eyes, or skin. The preferred mode of administration is left to the discretion of the practitioner, and will depend in-part upon the site of the medical condition (such as the site of cancer, a cancerous tumor or a pre-cancerous condition).

In one embodiment, the compound of the invention are administered parenterally. In a specific embodiment, the compounds of the invention are administered intravenously.

In specific embodiments, it can be desirable to administer the compound of the invention / referential compounds locally to the area in need of treatment (e.g., location of the tumor or ischemic condition). This can be achieved, for example, and not by way of limitation, by local infusion during surgery; topical application, e.g., in conjunction with a wound dressing after surgery; by injection; by means of a catheter; by means of a suppository; or by means of an implant, the implant being of a porous, non-porous, or gelatinous material, including membranes, such as sialastic membranes, or fibers. In one embodiment, administration can be by direct injection at the site (or former site) of a cancer, tumor, or precancerous tissue.

Pulmonary administration can also be employed, e.g., by use of an inhaler or nebulizer, and formulation with an aerosolizing agent, or via perfusion in a fluorocarbon or synthetic pulmonary surfactant. In certain embodiments, the compounds of the invention can be formulated as a suppository, with traditional binders and carriers such as triglycerides.

In yet another embodiment, the compound of the invention can be delivered in a controlled release system. In one embodiment, a pump can be used (*see* Sefton, CRC Crit. Ref Biomed. Eng. 1987, 14, 201; Buchwald et al., Surgery 1980, 88: 507; Saudek et al., N. Engl. J. Med. 1989, 321: 574). In another embodiment, polymeric materials can be used (*see* Medical Applications of Controlled Release, Langer and Wise (eds.), CRC Pres., Boca Raton, FL, 1974; Controlled Drug Bioavailability, Drug Product Design and Performance, Smolen and Ball (eds.), Wiley, New York, 1984; Ranger and Peppas, J. Macromol. Sci. Rev. Macromol. Chem. 1983, 23, 61; *see also* Levy et al., Science 1985, 228, 190; During et al., Ann. Neurol., 1989, 25, 351; Howard et al., J. Neurosurg., 1989, 71, 105). In yet another embodiment, a controlled-release system can be placed in proximity of the target of the compounds of the invention, e.g., the brain, thus requiring only a fraction of the systemic dose (*see, e.g.,* Goodson, in Medical Applications of Controlled Release, supra, vol. 2, 1984, pp. 115-138). Other controlled-release systems discussed in the review by Langer (Science 1990, 249, 1527-1533) can be used.

In another embodiment, polymeric materials can be used to achieve controlled or sustained release of the compound of the invention (*see, e.g.,* U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable.

Whether in solid, liquid or gaseous form, the compositions of the present invention can comprise an additional active agent selected from among those including, but not limited to, an additional prophylactic agent, an additional therapeutic agent, an antiemetic agent, a hematopoietic colony stimulating factor, an adjuvant therapy, a vaccine or other immune stimulating agent, an antibody/antibody fragment-based agent, an anti-depressant and an analgesic agent. For instance in a particular embodiment, the pharmaceutical composition comprises a compound of the invention, an additional anticancer agent, and a pharmaceutically acceptable carrier or vehicle.

The invention also provides a pharmaceutical pack or kit comprising one or more containers filled with one or more of the ingredients of the pharmaceutical compositions of the invention. Optionally associated with such container(s)

can be a notice in the form prescribed by a governmental agency regulating the manufacture, use or sale of pharmaceuticals or biological products, which notice reflects approval by the agency of manufacture, use or sale for human administration.

## EXAMPLES

### Example 1: *In silico* Screen.

A diverse *in silico* library was generated from the following commercially available libraries: ACB Blocks, Asinex, Chembridge, Maybridge, Microsource, NCI, Peakdale, FDA-approved drugs taken from the Zinc database (zinc.docking.org). The *in silico* library was converted in mol2 format and was filtered for drug-like features, ADME properties, and appropriate functional groups with Qikprop. Molecules were converted to 3D all-atom structures, generating a maximum of 4 stereoisomers, ionization states for pH 7.0 and pH 2.0, different tautomers and chiralities, and optimized for their geometry with Ligprep and Macromodel. The database of *in silico* 3D molecules totaled approximately 750,000 compounds. BAX structures for docking were prepared using an averaged BAX closed-loop structure and an averaged BAX open-loop structure with GROMACS software. The two structures were generated in suitable format for docking with Maestro and charged residues were confirmed for their ionization states and hydrogen-bonding conflicts. The receptor grid on BAX structures was generated from coordinates representing the center of the BIM SAHB binding site, ligands docked within 20Å, and their diameter midpoint constrained to 12Å from the supplied coordinates. No additional positional, hydrogen bond or hydrophobic constraints were included. Docking was performed using Glide in standard precision mode (SPVS) with the small molecule database for each BAX structure. Flexible docking was performed, allowing flips of 5- and 6- member rings, keeping the best 400 poses for energy minimization for each ligand and scaling the ligands' nonploar atoms van der Waals radii by a scaling factor of 0.9. The top 20,000 hits ranked based on Glidescore function for each BAX structural model were selected and re-docked to the BAX structures using extra precision docking mode (XPVS). The top 1000 hits from each docking calculation were visualized with the Glide pose viewer on the BAX structure and analyzed for their interactions with key BAX binding site residues and selected based on their favorable hydrogen bonds, hydrophobic contacts and molecular properties, leading to our identification of the compounds listed in Table 1 and depicted in Figure 9. Quickprop, Ligprep, Macromodel, Maesto, Glide are part of Schrodinger Suite 2006.

### Example 2: Synthesis of SAHBs that Directly Bind and Activate BAX.

Hydrocarbon-stapled peptides corresponding to the BH3 domains of BIM, BID, PUMA, and BAX and their mutants and FITC-βAla derivatives were synthesized, purified, and characterized using methodologies previously described. Examples of compositions of such SAHBs are listed in Figure 8 (Walensky, L. D., et al. (2004) Activation of apoptosis in vivo by a hydrocarbon-stapled BH3 helix, Science (New York, N.Y 305, 1466-1470; Walensky, L. D., et al. (2006) A stapled BID BH3 helix directly binds and activates BAX, Mol Cell 24, 199-210; Bird, G.H., Bernal, F., Pitter, K., and Walensky, L.D. Synthesis and biophysical characterization of stabilized alpha-helices of BCL-2 domains. Methods in Enzymology, 446: 369-386, 2008).

### Example 3: Preparation of BAX Suitable for NMR Analysis and Biological Testing.

The composition and method of producing BAX was altered from prior reports (Walensky, L. D., et al. (2006) A stapled BID BH3 helix directly binds and activates BAX, Mol Cell 24, 199-210; Suzuki, M., Youle, R. J., and Tjandra, N. (2000) Structure of Bax: coregulation of dimer formation and intracellular localization, Cell 103, 645-654) as necessary in order to optimize protein expression and purification of sufficiently stable and pure monomeric and other conformer species of BAX for the NMR analyses and biological assays described herein to identify and test BAX modulators. Full-length human BAX-encoding cDNA (aa 1-192) was cloned into pTYB1 plasmid (New England Biolabs) and fused at the N-terminus of chitin protein using restriction sites NdeI and SapI. Point mutagenesis of the BAX cDNA, including P168G, K21E, E69K, L45C, M137C, and combinations thereof, were generated by employing the QuikChange II site-directed mutagenesis kit (Stratagene, CA). The mutations and their associated open reading frames were confirmed by DNA sequence analysis. Fresh transformants in *Escherichia coli* BL21(DE3) cells were grown in Luria Broth (LB) media using flasks at OD: 0.8-1.0 or in enriched LB media (3.5g $KH_2PO_4$, 5.0g $K_2HPO4$, 3.5g $(NH_4)_2HPO_4$, 30g Glucose, 3.5g Tryptone, 5.0g Yeast Extract) using a 5L fermentor at OD: 14, following the manufacturer's protocol (New Brunswick, NJ). Cells were grown at 37°C and induction of expression was performed at 30°C with 1 mM IPTG for 4 hours. Cells were harvested by centrifugation at 5000 rpm for 25 min at 4°C and then resuspended in cold lysis buffer containing 20 mM Tris-HCl pH 7.6, 500 mM NaCl, 1 mM EDTA, and Roche protease inhibitor cocktail (50 ml buffer/25 g of pellet). Cells were aliquoted in Falcon tubes and frozen at -80°C. Once thawed, the cells were disrupted by sonication and separated from pellet by ultracentrifugation at 45,000 rpm for 1h at 4°C. The supernatant was loaded onto a disposable

gravity column (Bio-Rad) containing chitin beads (New England Biolabs) pre-equilibrated in lysis buffer at 4°C. The beads were washed with 20 bed volumes of lysis buffer and then 3 additional bed volumes of lysis buffer containing 50mM DTT. The column was capped at the top and bottom, and then chitin beads left overnight at 4°C for cleavage of the chitin fusion protein. BAX was eluted from the column with at least 10 bed volumes of lysis buffer. BAX protein was concentrated to 0.5 mL using a 10-KDa cut-off Centricon spin concentrator (Millipore) and then loaded onto a gel filtration column (Superdex 75, 10/300 GL, GE Healthcare Life Sciences), which was pre-equilibrated with gel filtration buffer (20 mM Hepes, 150 mM KCl, pH 7.0) at 4°C. Separation of a <0.25 mL injected sample volume yielded maximal BAX monomer at a flow rate of 0.25 mL/min with fractionation at 0.5mL intervals. Fractions containing BAX monomer were eluted at ~12 mL buffer volume, pooled, and then concentrated using a 10-KDa cut-off Centricon spin concentrator (Millipore) for prompt use in functional assays.

**Example 4: Identifying modulators of BAX by NMR Spectroscopy and Other Structural Methods**

Screening by NMR is conducted by recording $^1H$-$^{13}C$ filtered-ID experiments of uniformly $^{13}C$-labeled BAX at 25-50 $\mu$M concentrations, in the absence and presence of compounds at 50-100 $\mu$M concentrations. Binding is confirmed from changes in methyl groups of Ala, Val, Leu, Ile or Thr in the chemical resonance region between 1.0-0.2 p.p.m. The binding mode of test compounds is characterized by performing titrations of the molecules and recording $^1H$, $^{15}N$-HSQC or $^1H$-$^{13}C$ HSQC experiments of BAX in the absence or presence of the compounds and by monitoring the chemical shifts of the BAX backbone amides or side chain methyl groups, respectively. The weighted average chemical shift difference $\Delta$ is calculated as $\sqrt{\{(\Delta H)^2 + (\Delta N/5)^2\}/2}$ in p.p.m. and plotted as a function of the residue number of BAX. Significant chemical shift changes upon ligand titration are considered when induced $\Delta$ is greater than 0.1 p.p.pm. Uniformally labeled $^{15}N$ or $^{15}N/^{13}C$-labeled BAX samples are prepared and purified as described above, except for the following modifications to achieve isotopic labeling. Transformed *Escherichia coli* BL21 (DE3) cells are grown at 37°C and induced with 1 mM IPTG in either LB media (for unlabeled protein) or M9-minimal media substituted with $^{15}N$-$NH_4Cl$ (1 g/l) with or without $^{13}C$-glucose (2 g/l) to obtain uniformly $^{15}N$-labeled protein or $^{15}N$,$^{13}C$-labeled protein, respectively. NMR samples of BAX are prepared in 20 mM sodium acetate, 50mM NaCl buffer (pH 6.0) in $H_2O/D_2O$ (9:1). Test compound stocks are prepared, for example, in DMSO at 10 mM concentration. NMR experiments are processed with the NMRPipe spectral analysis package and chemical shifts variations measured with NMRView software. NMR spectra are recorded, for example, at 30 °C on Bruker Avance 600 MHz equipped with a z-shielded gradient, triple resonance cryoprobe.

Additional structural approaches for identifying and characterizing the binding properties of BAX interacting compounds include SAR by NMR, SHAPES NMR, and other fragment-based drug discovery methodologies that employ NMR and x-ray crystallography, as described and referenced above.

**Example 5: Competitive Fluorescence Polarization Binding Assay.**

Fluorescinated BIM SAHB (25 nM) was incubated with recombinant wild-type BAX or mutant derivatives thereof (100 nM) in binding buffer (140 mM NaCl, 50 mM Tris-HCl [pH 7.4]) at room temperature, in the presence or absence of acetylated BIM SAHB (1 $\mu$M), to establish baseline FPA values for FITC-BIM SAHB binding to BAX and competitive inhibition (Figure 11). To assess the BAX binding capacity of test compounds, serial dilutions starting from 50 $\mu$M were added to FITC-BIM SAHB (25 nM) and BAX (100 nM) and fluorescence polarization measured over time using a POLARstar OPTIMA microplate reader (BMG labtech) to identify competitive inhibitors of FITC-BIM SAHB/BAX binding. $IC_{50}$ values were determined by nonlinear regression analysis using Prism software 4.0 (Graphpad). This assay can also be employed for empiric screening of libraries to identify compounds with BAX-binding activity.

**Example 6: Oligomerization Assay.**

BIM SAHB was added to a 200 $\mu$L solution (20 mM Hepes/KOH pH 7.2-7.4, 150 mM KCl) containing monomeric BAX (38 $\mu$M) at a ratio of 0.5:1, 1 :1, 2:1 and 4:1 BIM SAHB:BAX. The mixtures and a sample of BAX monomer alone were incubated at 22° for 15 minutes and then subjected to analysis by size exclusion chromatography (SEC) using an SD75 column (Figure 12A). The chromatogram demonstrates the monomeric and oligomeric peaks at ~11.5 min and ~6.5 min, respectively. Protein standards (GE Healthcare) were used to calibrate the molecular weights of gel filtration peaks. For time-dependent analysis, BIM SAHB was added to monomeric BAX at a ratio of 1:1, and the mixtures were analyzed by SEC after incubation for 30, 60, and 90 minutes at 22°C. As a baseline for comparison, Bax monomer alone was analyzed at time 0 and the above time points. Test compounds identified by the *in silico* screen were evaluated in

this BAX oligomerization assay, and as demonstrated for select compounds, molecule-induced conversion of BAX from its monomeric to its oligomeric form was observed (Figure 12B). An alternative method employed for high throughput detection of BAX oligomerization involves dynamic light scattering (DLS) analysis (Wyatt Technologies) (Figure 13). The oligomerization assay using SEC or DLS read-outs can also be employed for empiric screening of libraries to identify compounds that directly modulate (i.e. activate or inhibit) BAX oligomerization.

**Example 7: Conformational Change Assay.**

BIM SAHB was added to a 20 μL PBS solution containing monomeric BAX (9 μM) at a ratio of 0.5:1, 1 :1, 2:1 and 4:1 BIM SAHB:BAX. The mixtures (10 μL) and a BAX monomer sample (10 μL) were incubated at 22°C for 15 minutes and then added to a 3% BSA in PBS solution (250 μL) containing 15 μL of 6A7 anti-BAX antibody for 1 hour incubation at 4°C. Additionally, 1 μL of each input sample (10%) was mixed with 50 μL of SDS-sample buffer to measure baseline BAX levels across specimens. Preclarified sepharose beads (50 μL) were added to the BIM SAHB:BAX and BAX monomer solutions for an additional 2 hour incubation at 4°C. The sepharose beads were spun down, washed 3 times with 1 mL of 3% BSA in PBS solution, resuspended in 50 μL of SDS-sample buffer and boiled at 95 °C for 2 minutes. Ten microliters each of inputs and immunoprecipitation samples were used for analysis. Samples were separated on 12% SDS-PAGE Bis-Tris gel, blotted on a PVDF membrane, and Western analysis performed using the rabbit polyclonal N20 anti-BAX antibody (Santa Cruz Biotechnology) and chemiluminescence-based detection (PerkinElmer). The capacity of test compounds to induce BAX conformational change is evaluated using this assay. In addition, this approach can be employed for empiric screening of libraries to identify compounds that directly modulate (i.e. activate or inhibit) BAX conformational change.

**Example 8: Crosslinking Assay.**

BAX (10 nM) was added to serial dilutions of test compounds starting at 10 μM, in the presence or absence of 100 nM BIM SAHB, for 1 hour at room temperature. Subsequently, 1 mM BMH (Pierce) was added for an additional 30 minutes incubation. The reaction was quenched with gel load buffer and samples were analyzed by electrophoresis and Western analysis using the N20 anti-BAX antibody (Santa Cruz Biotechnology, Inc.) to determine the presence or absence of BAX oligomeric forms. In the absence of BIM SAHB, the assay identifies compounds that trigger BAX oligomerization. When performed in the presence of BIM SAHB, compounds that synergize with or block BIM SAHB-induced BAX oligomerization can be identified.

**Example 8: Liposomal Release Assay.**

A liposomal release assay is used to measure the capacity of test ligands to directly activate the functional release activity of pro-apoptotic BAX in the absence of factors other than BAX, test ligand, and lipid vesicles). Large unilamellar vesicles (LUVs) with lipid composition resembling the mitochondrial outer-membrane contact sites (OMCT vesicles) (Ardail et al., 1990; Lutter et al., 2000) were generated and entrapped with FITC-labeled dextran as previously described (Oh et al., 2005; Oh et al., 2006, Pitter et al., 2008). The fluorescence dequenching assay was performed and FITC release was quantitated as reported (Oh et al., 2005, Pitter et al., 2008). Experiments are conducted using a lipid concentration of 10 mg/ml, 15-50 nM BAX, and serial dilutions of BAX-activating peptides or test compounds. Negative control studies include testing BAX alone, compounds alone, control compounds (e.g. enantiomers of small molecules, amino acid mutants of peptides), and the capacity of anti-apoptotic BCL-XL to block ligand-induced BAX activation, as previously described (Walensky et al, 2006, Gavathiotis et al, 2008). Such liposomal release assays identify compounds that trigger BAX-mediated release and can be conducted in high throughput. When performed in the presence of BIM SAHB, compounds that synergize with or block BIM SAHB-triggered, BAX-mediated liposomal release can be identified. This assay can also be employed for empiric screening of libraries to identify compounds that directly modulate (i.e. activate or inhibit) BAX-mediated liposomal release.

**Example 9: Cytochrome c Release Assay.**

A mitochondrial cytochrome c release assay is used to measure the capacity of test ligands to directly activate the functional release activity of pro-apoptotic BAX in the context of the organelle (Figure 14). *Bax*$^{-/-}$/*Bak*$^{-/-}$ mitochondria (0.5 mg/mL) were isolated and release assays performed as described (Pitter, K., Bernal, F., LaBelle, J.L., and Walensky, L.D. Dissection of the BCL-2 family signaling network using stabilized alpha-helices of BCL-2 domains. Methods in Enzymology, 446: 387-408, 2008). Mitochondria were incubated with BAX alone (50 nM), test compounds alone (e.g. serial dilution from 25 μM), and in combination (50 nM BAX + serial dilution of test compounds) in the presence or absence of BIM SAHB (250 nM). After 40 minutes, the pellet and supernatant fractions were isolated and cytochrome

c quantitated using a colorimetric ELISA assay (R&D Systems). Percent cytochrome c released into the supernatant (%cyto$c_{sup}$) from releasable mitochondrial pools was calculated according to the following equation: %cyto$c$=[(cyto$c_{sup}$-cyto$c_{backgr}$)/(cyto$c_{total}$-cyto$c_{backgr}$)]*100, where background release represents cytochrome c detected in the supernatant of vehicle-treated (1% DMSO) samples and total release represents cytochrome c measured in 1% Triton-X 100 treated samples. In the absence of BIM SAHB, the assay identifies compounds that trigger BAX-mediated cytochrome c release. When performed in the presence of BIM SAHB, compounds that synergize with or block BIM SAHB-triggered, BAX-mediated cytochrome c release can be identified. This assay can also be employed for empiric screening of libraries to identify compounds that directly modulate (i.e. activate or inhibit) BAX-mediated cytochrome c release.

**Example 10: Cellular assay for detecting induction of BAX-mediated apoptosis by a test ligand.**

*Bax*$^{-/-}$*Bak*$^{-/-}$ DKO MEFs were SV40 transformed and maintained in Dulbecco's modified Eagles medium supplemented with 10% fetal bovine serum following standard culture conditions and procedures. Reconstitution of BAX and its mutants into DKO cells was achieved by retroviral transduction of BAX-IRES-GFP as previously described (Cheng, E. H., et al. BCL-2, BCL-X(L) sequester BH3 domain-only molecules preventing BAX- and BAK-mediated mitochondrial apoptosis. Mol Cell 8, 705-711 (2001); Kim, H., et al. Hierarchical regulation of mitochondrion-dependent apoptosis by BCL-2 subfamilies. Nat Cell Biol 8, 1348-1358 (2006)), followed by MoFlo sorting for GFP positive cells. Expression of BAX protein was confirmed by anti-BAX Western analysis. BAX-reconstituted MEFs and control *Bax*$^{-/-}$*Bak*$^{-/-}$ DKO MEFs were exposed to a series of concentrations of test compounds and cell death was quantified by Annexin-V-Cy3 (BioVision, Mountain View, CA) staining according to the manufacturer's protocols, followed by flow cytometric analysis using a FACS Caliber (BD Bioscience, San Jose, CA) and CellQuest or FlowJo software (Figure 15). To monitor for inhibition of BAX-mediated cell death by the test compounds, the identical experiment was performed in the presence of staurosporine (e.g. 1 μM) or BIM SAHB (e.g. 10 μM), followed by annexin-V analysis as described above. This assay can also be employed for empiric screening of libraries to identify compounds that directly modulate (i.e. activate or inhibit) BAX-mediated apoptosis induction in a cellular context.

## Claims

1. A compound (Z)-4-(2-(4-ethoxyphenyl)hydrazono)-5-methyl-2-(4-phenylthiazol-2-yl)-2,4-dihydro-3H-pyrazol-3-one or a salt thereof for use in a method of treating cancer.

2. The compound for the use of claim 1, wherein the cancer is selected from a solid tumor, leukemia, and lymphoma.

3. The compound for the use of claim 1 or 2, wherein the cancer is selected from the group consisting of lung cancer, colon cancer, breast cancer, prostate cancer, and genitourinary cancer.

4. The compound for the use of any one of claims 1 to 3, wherein the cancer is a chemoresistant cancer.

5. The compound for the use of claim 4, wherein the chemoresistant cancer is resistant to ABT-737, ABT-263, obatoclax, or a BCL-2 survival protein inhibitor.

## Patentansprüche

1. Verbindung (Z)-4-(2-(4-Ethoxyphenyl)hydrazono)-5-methyl-2-(4-phenylthiazol-2-yl)-2,4-dihydro-3H-pyrazol-3-on oder ein Salz davon zur Verwendung in einem Verfahren zur Behandlung von Krebs.

2. Verbindung zur Verwendung nach Anspruch 1, wobei der Krebs aus festem Tumor, Leukämie und Lymphom ausgewählt ist.

3. Verbindung zur Verwendung nach Anspruch 1 oder 2, wobei der Krebs aus der Gruppe, bestehend aus Lungenkrebs, Kolonkrebs, Brustkrebs, Prostatakrebs und Urogenitalkrebs ausgewählt ist.

4. Verbindung zur Verwendung nach einem der Ansprüche 1 bis 3, wobei der Krebs ein chemoresistenter Krebs ist.

5. Verbindung zur Verwendung nach Anspruch 4, wobei der chemoresistente Krebs gegenüber ABT-737, ABT-263, Obatoclax oder einem BCL-2-Survival-Protein-Inhibitor resistent ist.

## Revendications

1. Composé (Z)-4-(2-(4-éthoxyphényl)hydrazono)-5-méthyl-2-(4-phénylthiazol-2-yl)-2,4-dihydro-3H-pyrazol-3-one ou

sel de celui-ci destiné à être utilisé dans un procédé de traitement du cancer.

2. Composé destiné à être utilisé selon la revendication 1, le cancer étant choisi parmi une tumeur solide, une leucémie et un lymphome.

3. Composé destiné à être utilisé selon la revendication 1 ou 2, le cancer étant choisi dans le groupe constitué du cancer du poumon, du cancer du côlon, du cancer du sein, du cancer de la prostate et du cancer génito-urinaire.

4. Composé destiné à être utilisé selon l'une quelconque des revendications 1 à 3, le cancer étant un cancer chimiorésistant.

5. Composé destiné à être utilisé selon la revendication 4, le cancer chimiorésistant étant résistant à ABT-737, ABT-263, à l'obatoclax ou à un inhibiteur de protéine de survie BCL-2.

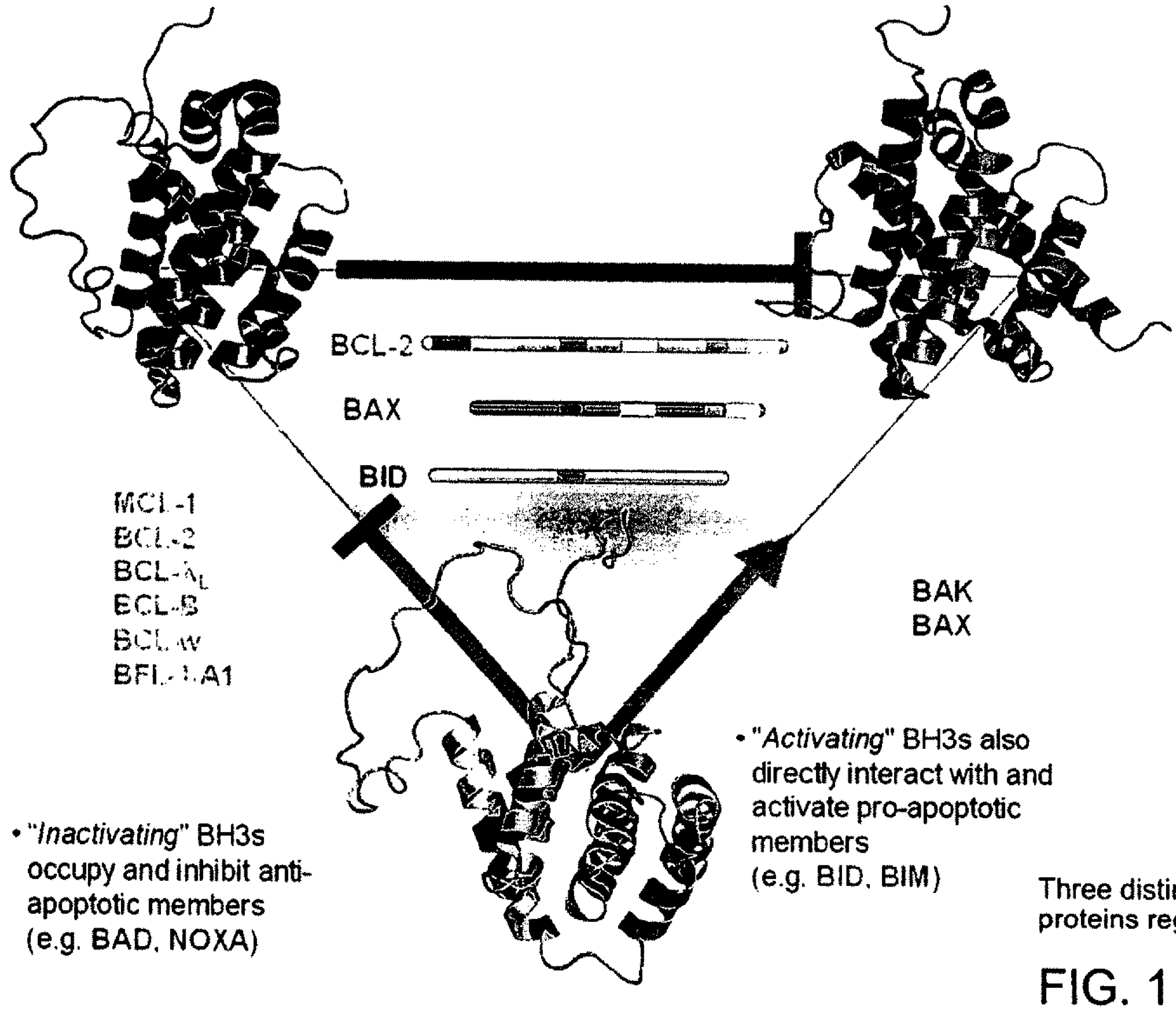


Three distinct classes of BCL-2 family proteins regulate apoptosis.

FIG. 1

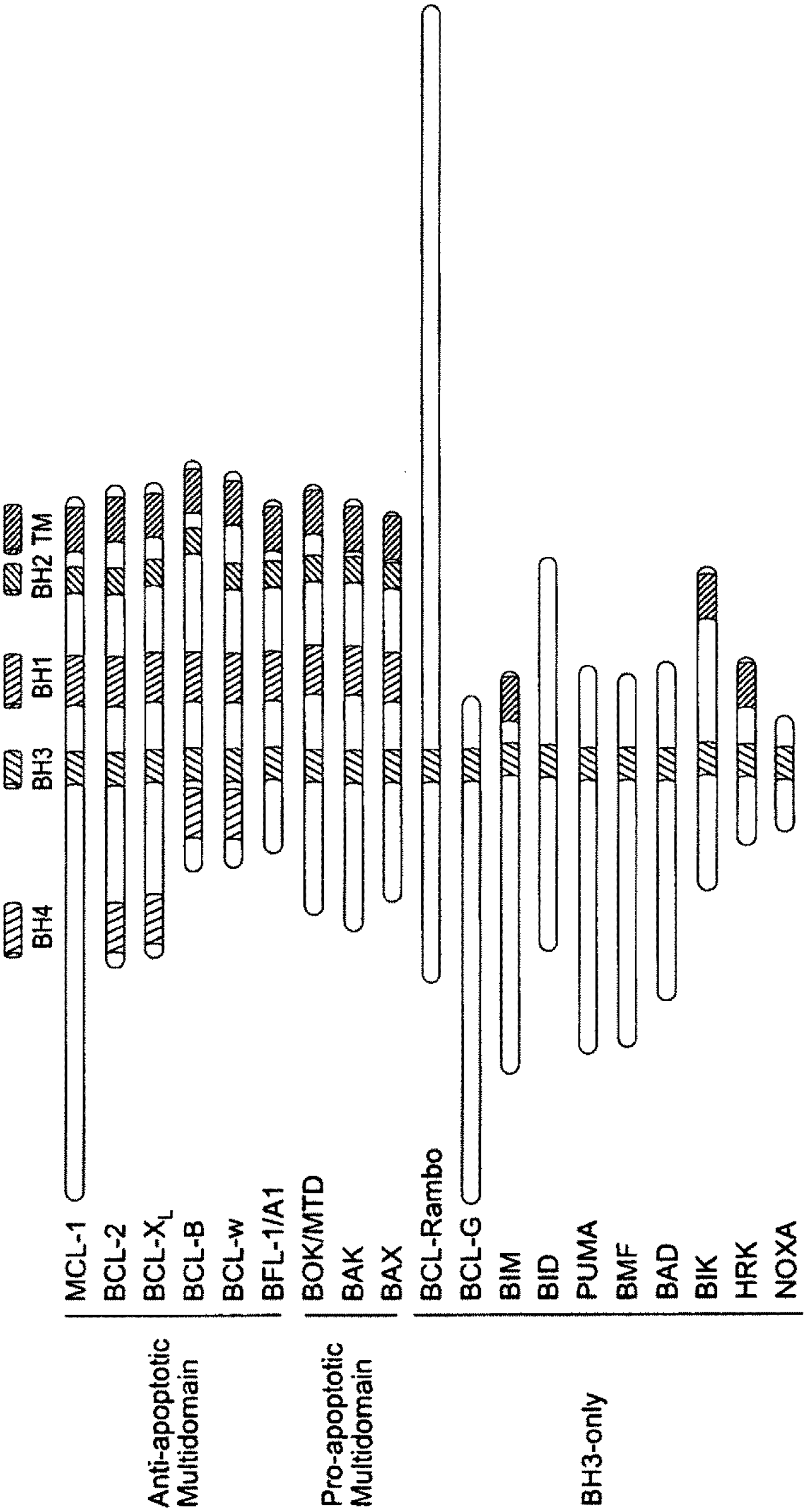


BCL-2 family members share conserved BCL-2 homology (BH) domains.

FIG. 2

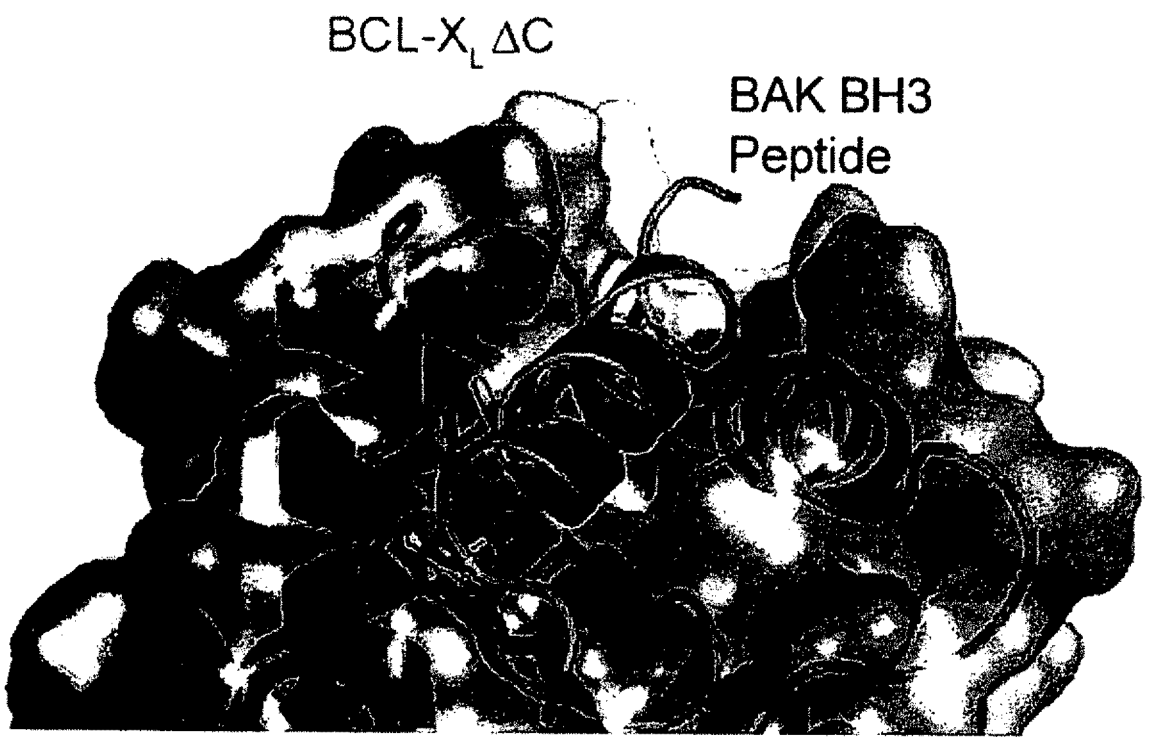


Anti-apoptotic BCL-2 proteins possess a BH3-binding pocket.

FIG. 3

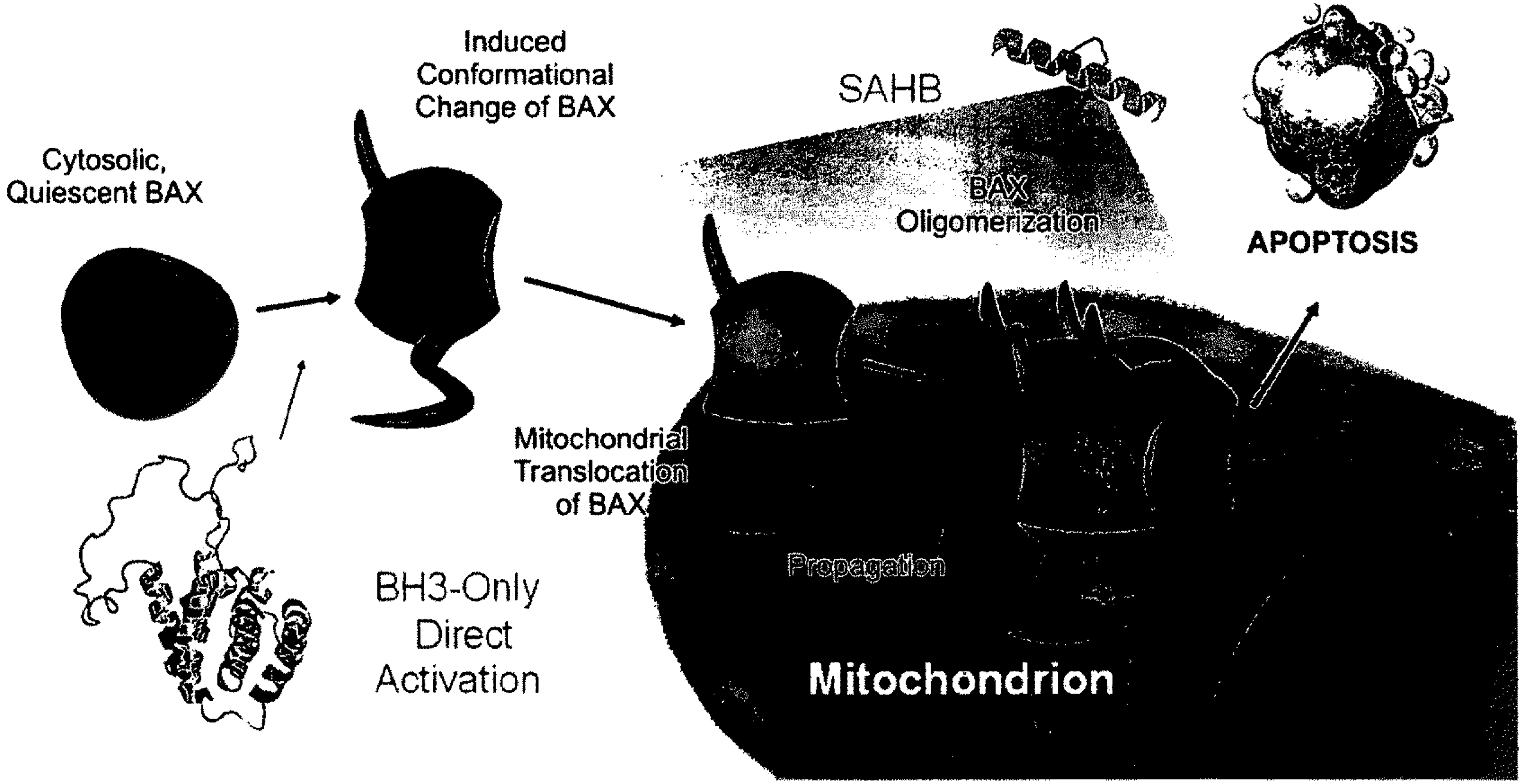


Cytosolic BAX is activated through key intermediate steps that ultimately lead to formation of a homo-oligomer within the outer mitochondrial membrane. Modulation of these steps by peptides (e.g. SAHB) or small molecules could pharmacologically regulate BAX-mediated cell death.

FIG. 4

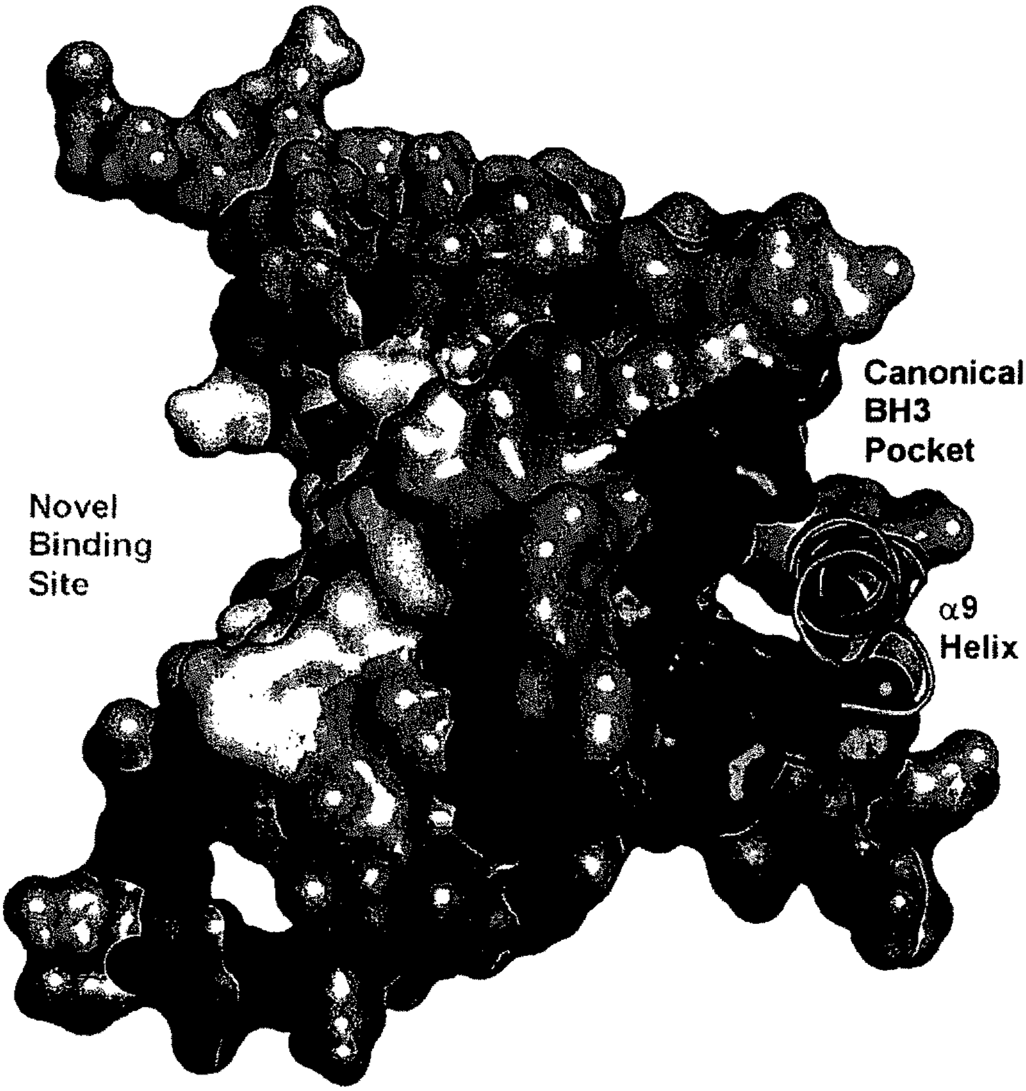


NMR analysis of the BIM SAHB-BAX interaction revealed a new binding site for BH3-mediated BAX activation. BIM SAHB engages BAX at a structural location that is distinct from the canonical BH3 binding site identified for anti-apoptotic proteins.

FIG. 5

FIG. 6A

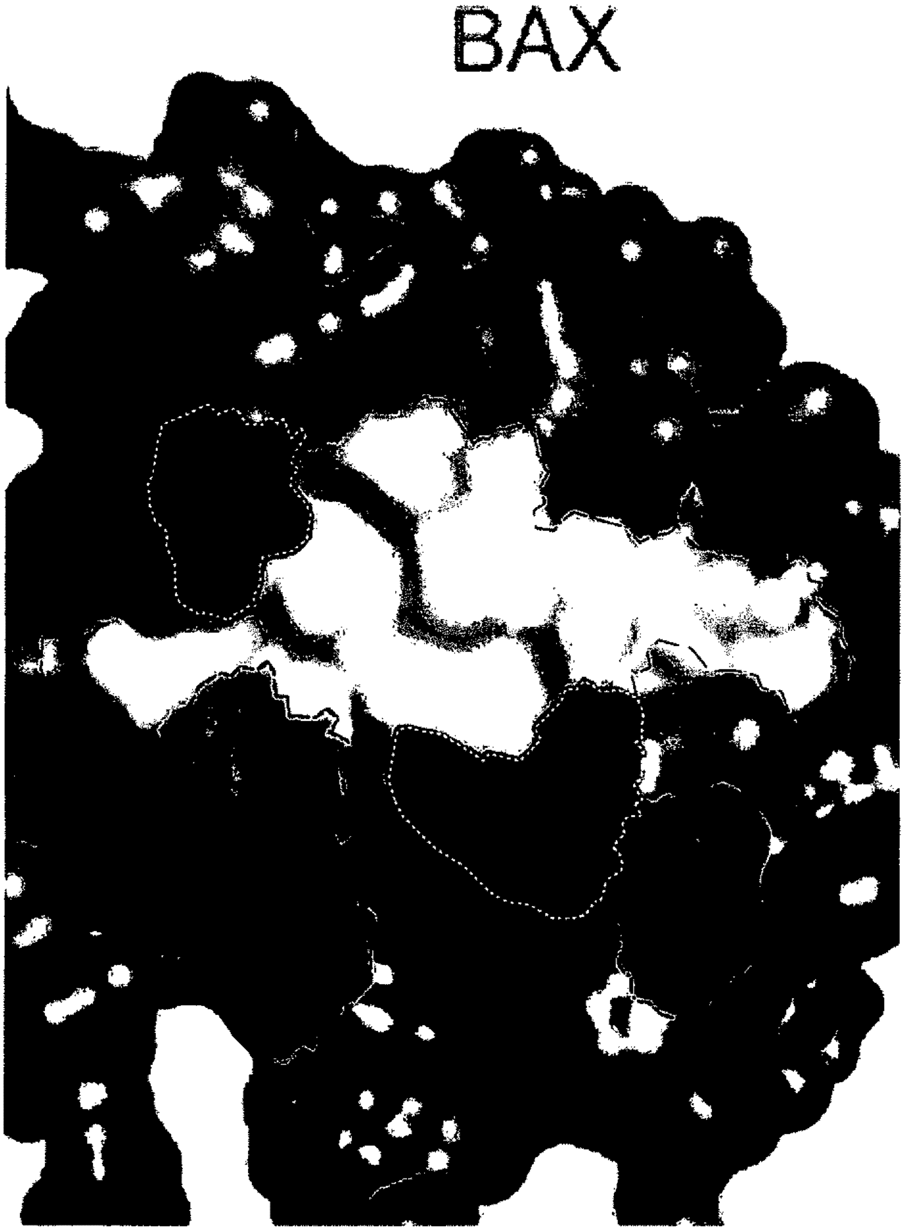


BH3 Interaction site on BAX

Surface diagram illustrating the novel BH3 interaction site on BAX. Solvent exposed residues at the binding site are indicated.

designates the side chains of the hydrophobic residues M20, A24, L27, I31, I133, M137, and L141.

designates the side chains of positively (K21, R134) charged residues.

designates the side chains of negatively (E131) charged residues.

designates side chains of the hydrophilic residues (Q28, Q32).

FIG. 6B

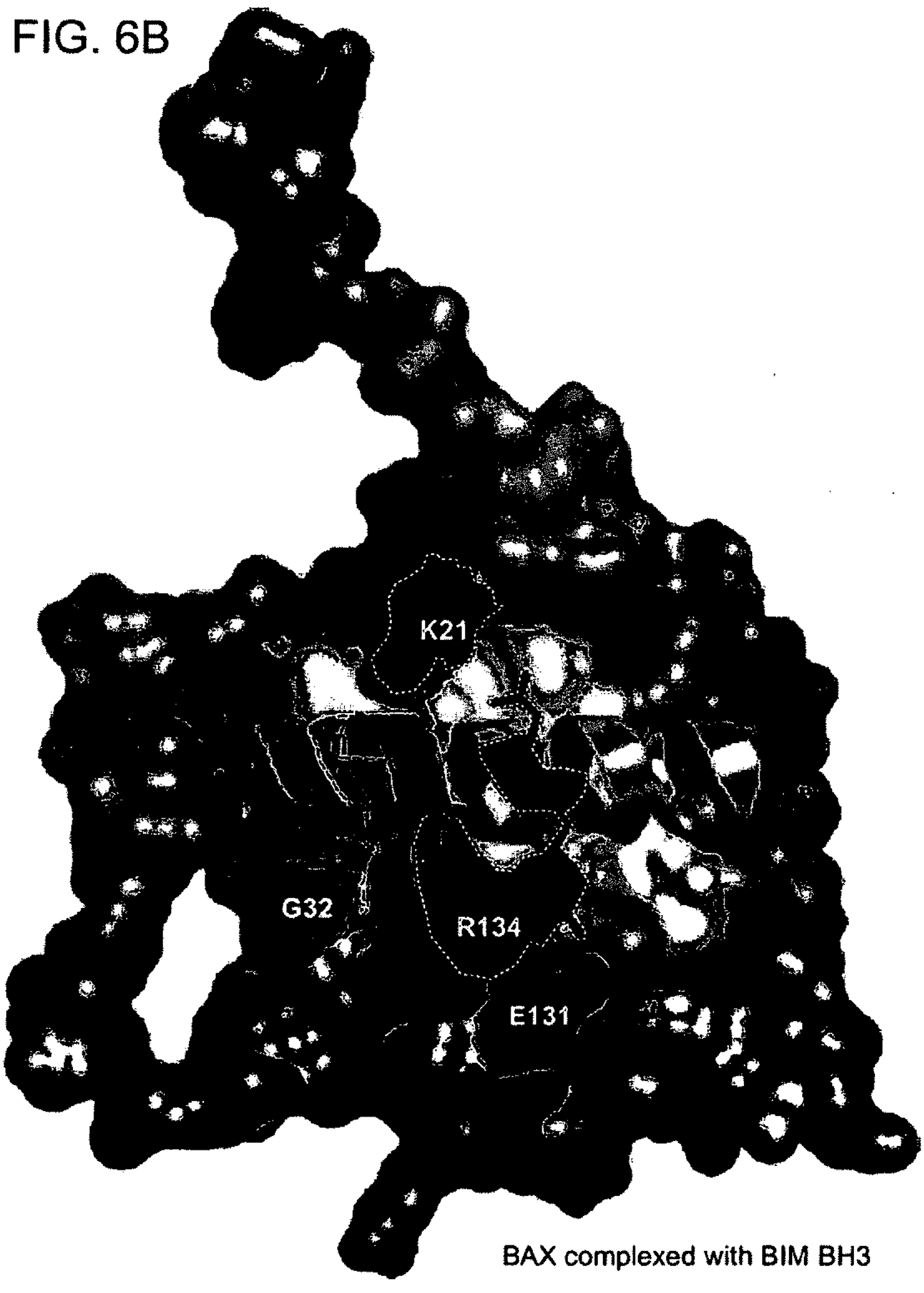


BAX complexed with BIM BH3

Orientation of BIM BH3 at the BAX binding site. The C- to N-terminus is oriented right to left with engagement of BIM BH3 residues :

- N160 (green),
- D157 (red), and
- R153 (blue)

with complementary polar:

- (Gln32, green)
- positively charged (Arg134, blue) residues
- negatively charged (Glu131, red) residues

# FIG. 7A

```
Human BAX                                                    SEQ ID NO:1

                               α1
1     MDGSGEQPRGGGPTSSEQIMKTGALLLQGFIQDRAGRMGGEAPELALDPV
                     α2               α3             α4
51    PQDASTKKLSECLKRIGDELDSNMELQRMIAAVDTDSPREVFFRVAADMF
                        α5                     α6
101   SDGNFNWGRVVALFYFASKLVLKALCTKVPELIRTIMGWTLDFLRERLLG
       α7       α8               α9
151   WIQDQGGWDGLLSYFGTPTWQTVTIFVAGVLTASLTIWKKMG
```

Amino acid sequence of BAX.

☐ Highlighted residues demarcate alpha-helices 1-9.

The residues of the novel BH3 binding site on BAX are bolded and underlined.

FIG. 7B

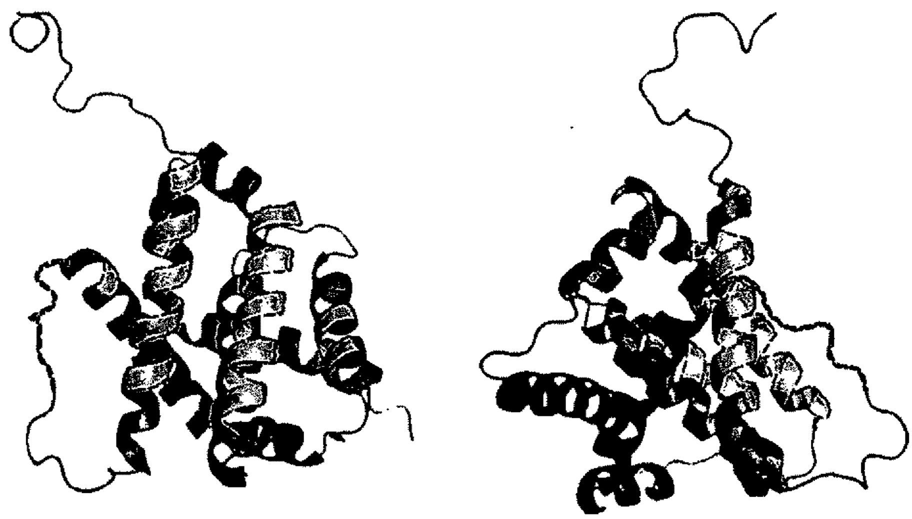

Ribbon diagrams of BAX.
--- Residues engaged in BIM BH3 interactions are outlined.

FIG. 7C

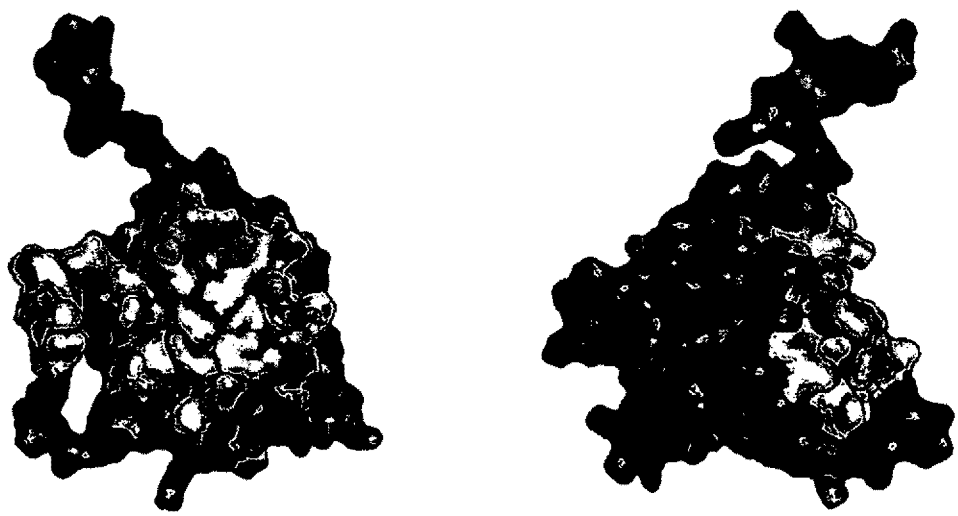

Surface diagrams of BAX.
--- Residues engaged in BIM BH3 interactions are outlined.

# FIG. 8

**Human $BIM_{EL}$** **SEQ ID NO:2**

MAKQPSDVSSECDREGRQLQPAERPPQLRPGAPTSLQTEPQGNPEGNHGGEGDSCPH
GSPQGPLAPPASPGPFATRSPLFIFMRRSSLLSRSSSGYFSFDTDRSPAPMSCDKST
QTPSPPCQAFNHYLSAMASMRQAEPADMRPEIWIAQELRRIGDEFNAYYARRVFLNN
YQAAEDHPRMVILRLLRYIVRLVWRMH

**BIM BH3:** $^{145}$EIWIAQELRRIGDEFNAYYARR$^{164}$ **SEQ ID: NO 3**

**BIM SAHBs:**

| | |
|---|---|
| BIM $SAHB_{A1}$ | IWIAQELR*IGD*FNAYYARR |
| BIM $SAHB_{A2}$ | EIWIAQELR*IGD*FNAYYA |
| BIM $SAHB_{B1}$ | I*IAQ*LRRIGDEFNAYYARR |
| BIM $SAHB_{B2}$ | EI*IAQ*LRRIGDEFNAYYA |
| BIM $SAHB_{C1}$ | IW*AQE*RRIGDEFNAYYARR |
| BIM $SAHB_{C2}$ | EIW*AQE*RRIGDEFNAYYA |
| BIM $SAHB_{D1}$ | IWI*QEL*RIGDEFNAYYARR |
| BIM $SAHB_{D2}$ | EIWI*QEL*RIGDEFNAYYA |
| BIM $SAHB_{E1}$ | IWIAQELRRIGD*FNA*YARR |
| BIM $SAHB_{E2}$ | EIWIAQELRRIGD*FNA*YA |
| BIM $SAHB_{F1}$ | IWIA*ELR*IGDEFNAYYARR |
| BIM $SAHB_{F2}$ | EIWIA*ELR*IGDEFNAYYA |
| BIM $SAHB_{G1}$ | IWIAQELRRIGDEF*AYY*RR |
| BIM $SAHB_{G2}$ | EIWIAQELRRIGDEF*AYY* |

# FIG. 8 (continued)

**Human BID** **SEQ ID NO:4**

MDCEVNNGSSLRDECITNLLVFGFLQSCSDNSFRRELDALGHELPVLAPQWEGYDEL
QTDGNRSSHSRLGRIEADSESQEDIIRNIARHLAQVGDSMDRSIPPGLVNGLALQLR
NTSRSEEDRNRDLATALEQLLQAYPRDMEKEKTMLVLALLLAKKVASHTPSLLRDVF
HTTVNFINQNLRTYVRSLARNGMD

**BID BH3**: $^{77}$ESQEDIIRNIARHLAQVGDEMDRSI$^{101}$ **SEQ ID: NO 5**

**BID SAHBs:**

BID $SAHB_{A1}$ DIIRNIARHLA*VGD*BDRSI
BID $SAHB_{A2}$ ESQEDIIRNIARHLA*VGD*BDRSI
BID $SAHB_{B1}$ DIIR*IAR*LAQVGDEBDRSI
BID $SAHB_{B2}$ ESQEDIIR*IAR*LAQVGDEBDRSI

**Human PUMA-α** **SEQ ID NO:6**

MARARQEGSSPEPVEGLARDGPRPFPLGRLVPSAVSCGLCEPGLAAAPAAPTLLPAA
YLCAPTAPPAVTAALGGSRWPGGPRSRPRGPRPDGPQPSLSLAEQHLESPVPSAPGA
LAGGPTQAAPGVRGEEEQWAREIGAQLRRMADDLNAQYERRRQEEQQRHRPSPWRVL
YNLIMGLLPLPRGHRAPEMEPN

FIG. 8 (continued)

**PUMA BH3:** $^{129}$EEEQWAREIGAQLRRMADDLNAQYERRR$^{156}$ **SEQ ID NO:7**

**PUMA SAHBs:**

PUMA $SAHB_{A1}$ QWAREIGLQAR*BAD*LNAQY
PUMA $SAHB_{A2}$ EQWAREIGLQAR*BAD*LNAQY
PUMA $SAHB_{A3}$ EQWAREIGLQAR*BAD*LNAQYE
PUMA $SAHB_{A1}$ QW*REI*LQARRBADDLNAQY
PUMA $SAHB_{B3}$ EQW*REI*LQARRBADDLNAQYE

**Human BAX** **SEQ ID NO:1**

MDGSGEQPRGGGPTSSEQIMKTGALLLQGFIQDRAGRMGGEAPELALDPVPQDASTK
KLSECLKRIGDELDSNMELQRMIAAVDTDSPREVFFRVAADMFSDGNFNWGRVVALF
YFASKLVLKALCTKVPELIRTIMGWTLDFLRERLLGWIQDQGGWDGLLSYFGTPTWQ
TVTIFVAGVLTASLTIWKKMG

**BAX BH3**: $^{52}$QDASTKKLSECLKRIGDELDSNMELQR$^{78}$ **SEQ ID NO: 8**

**BAX SAHBs**:

BAX $SAHB_A$ QDASTKKLSECLK*IGD*LDSN
BAX $SAHB_B$ QDASTK*LSE*LKRIGDELDSN
BAX $SAHB_C$ QDASTKK*SEC*KRIGDELDSN
BAX $SAHB_D$ QDASTKKL*ECL*RIGDELDSN

FIG. 8 (continued)

**BAX Activator BH3 Consensus** **SEQ ID NO:9**

I/L A/S/G X X L K/R R I/M/V D D/E L/F N/D A/S X Y/M

as derived from conserved residues among BIM, PUMA, and BAX BH3 domains at alpha-helical interaction surface:

```
BIM BH3       EIWIAQELRRIGDEFNAYYARR
PUMA BH3    EQWAREIGAQLRRMADDLNAQYER
BAX BH3    QDASTKKLSECLKRIGDELDSNMELQR
```

☐ Amino acid identity
☐ Conserved change

Amino acid sequences of BAX activator BH3-only proteins, their BH3 domain peptides, examples of hydrocarbon stapled derivatives, and a BAX activator BH3 consensus sequence.

FIG. 9A

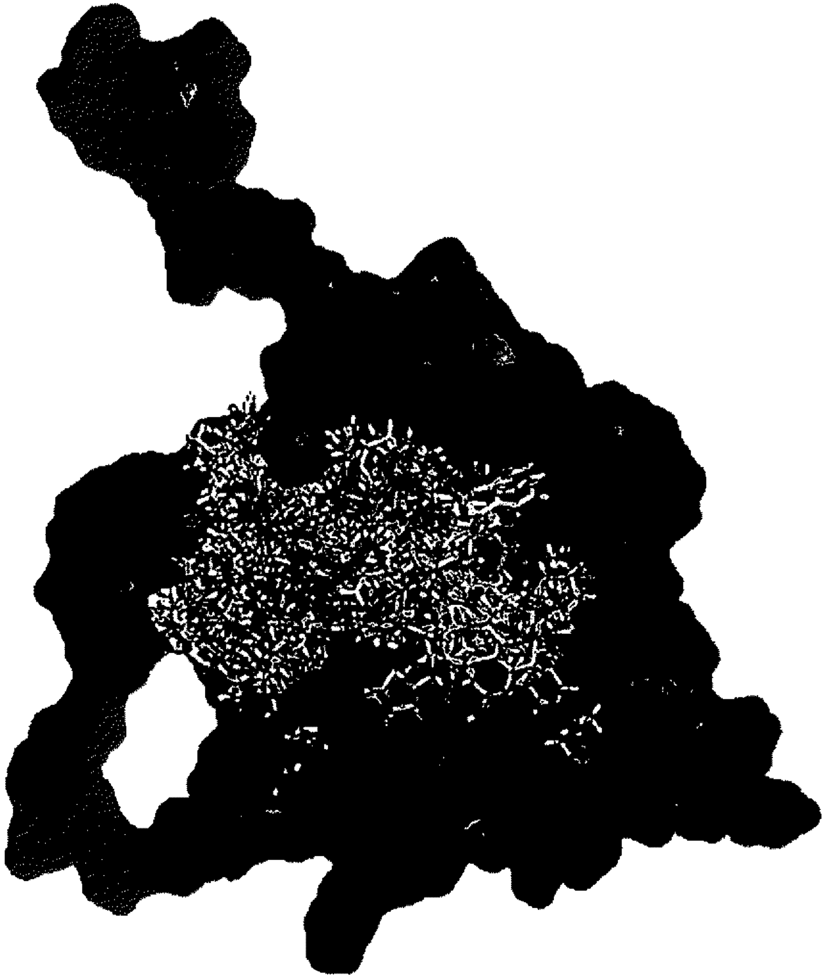

Molecules identified by virtual screen decorate the novel BAX activation site along the horizontal hydrophobic groove.

FIG. 9B

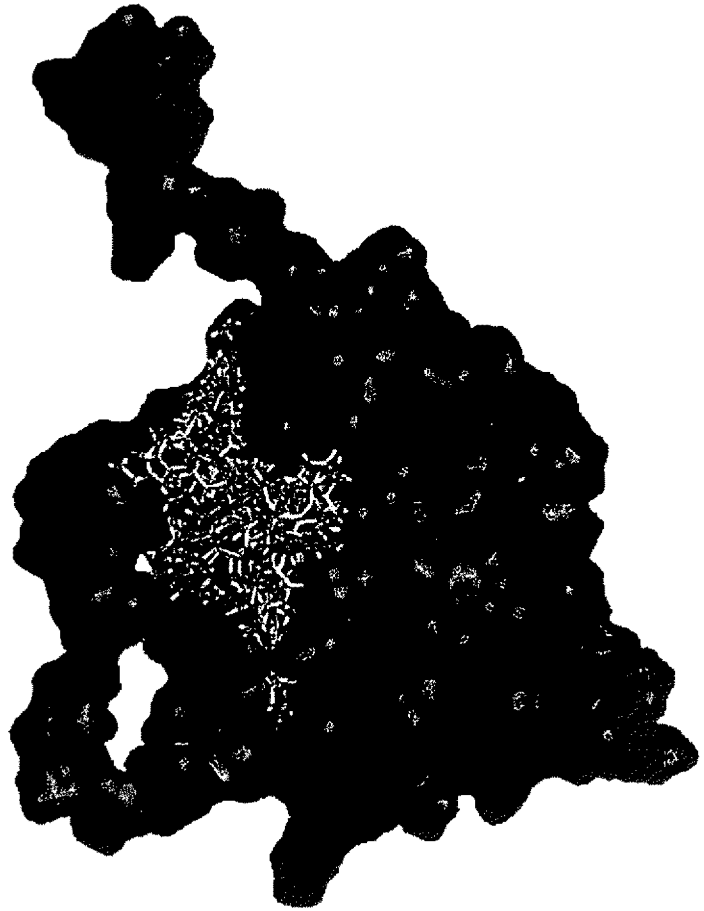

Molecules identified by virtual screen decorate the novel BAX activation site at the superior juxta-loop region.

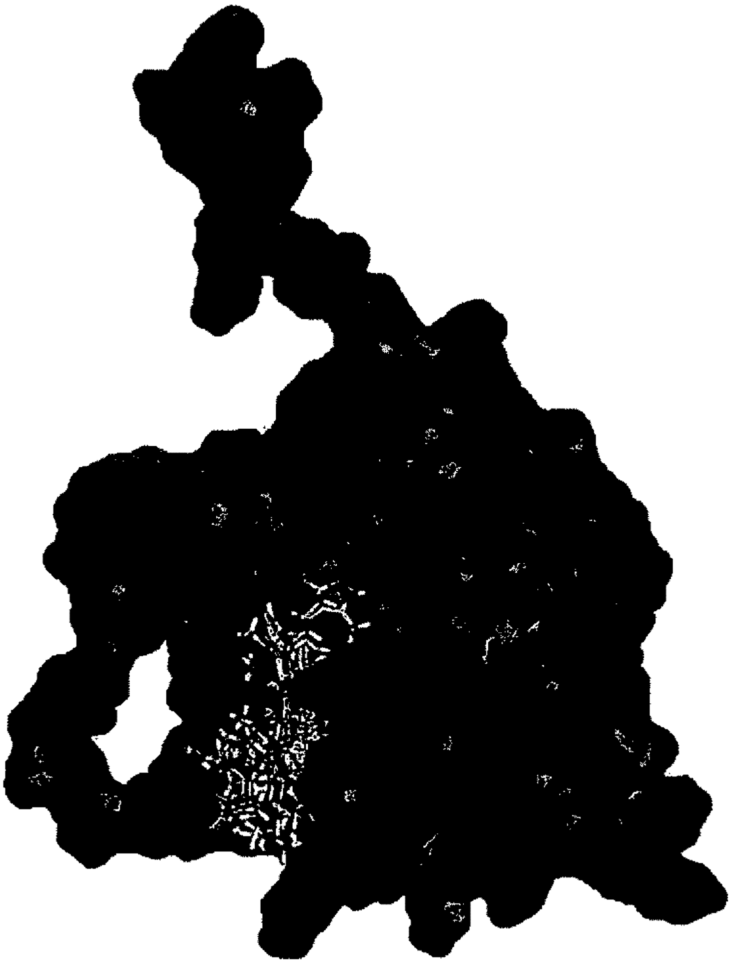

Molecules identified by virtual screen decorate the novel BAX activation site at the inferior juxta-loop region.

FIG. 9C

α1

1 MDGSGEQPRGGGPTSS**EQ**I**MKT**G**AL**L**LQG**F**IQDRAGRMGGEA**PELA**LDPV**

α2 α3 α4

51 **PQDASTKKLSE**CL**K**RIGDELDSNMELQRMIAAVDTDSP**R**EV**FF**RVAADMF

α5 α6

101 SDGNFNWGRVVALFYFASKLV**LK**A**L**C**TKVPELIRT**I**MGW**T**LDF**L**RER**L**LG**

α7 α8 α9

151 WIQDQ**GGWD**G**LL**SYFGTPTWQTVTIFVAGVLTASLTIWKKMG

Amino acid sequence of BAX.

☐ Highlighted residues demarcate alpha-helices 1-9.

The residues involved with ligand interactions at or adjacent to the novel BH3 binding site on BAX are bolded and underlined.

FIG. 10A

FIG. 10B

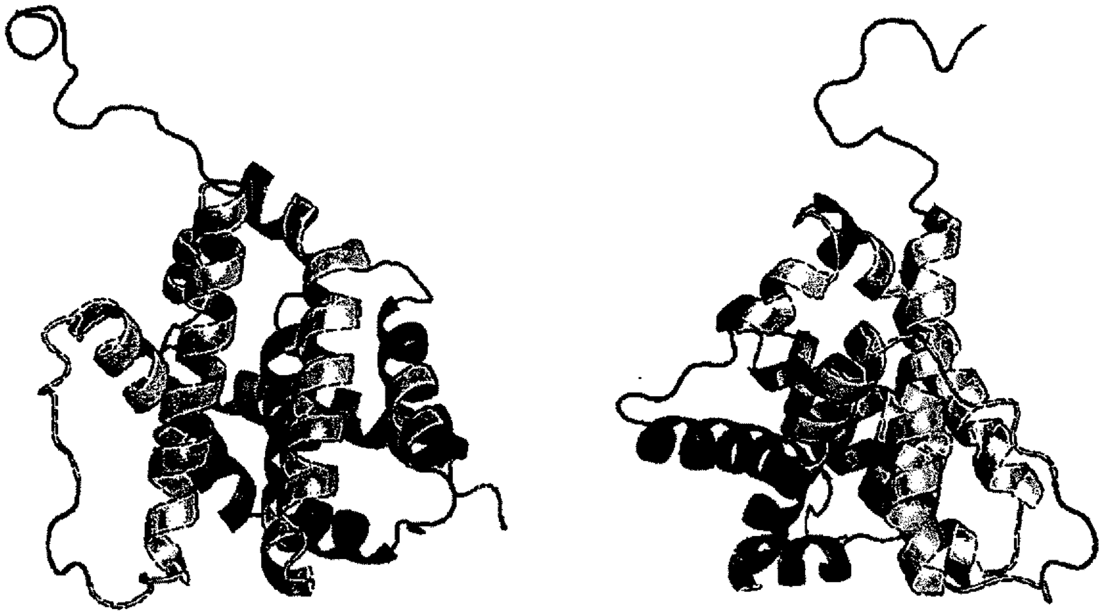

Ribbon diagrams of BAX.
--- Residues engaged in ligand interactions are outlined.

FIG. 10C

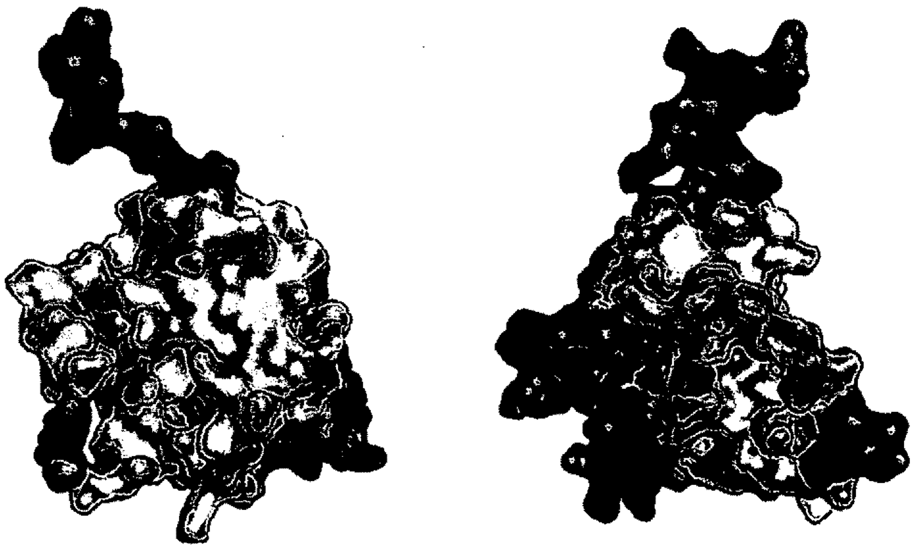

Surface diagrams of BAX.
--- Residues engaged in ligand interactions are outlined.

FIG. 11

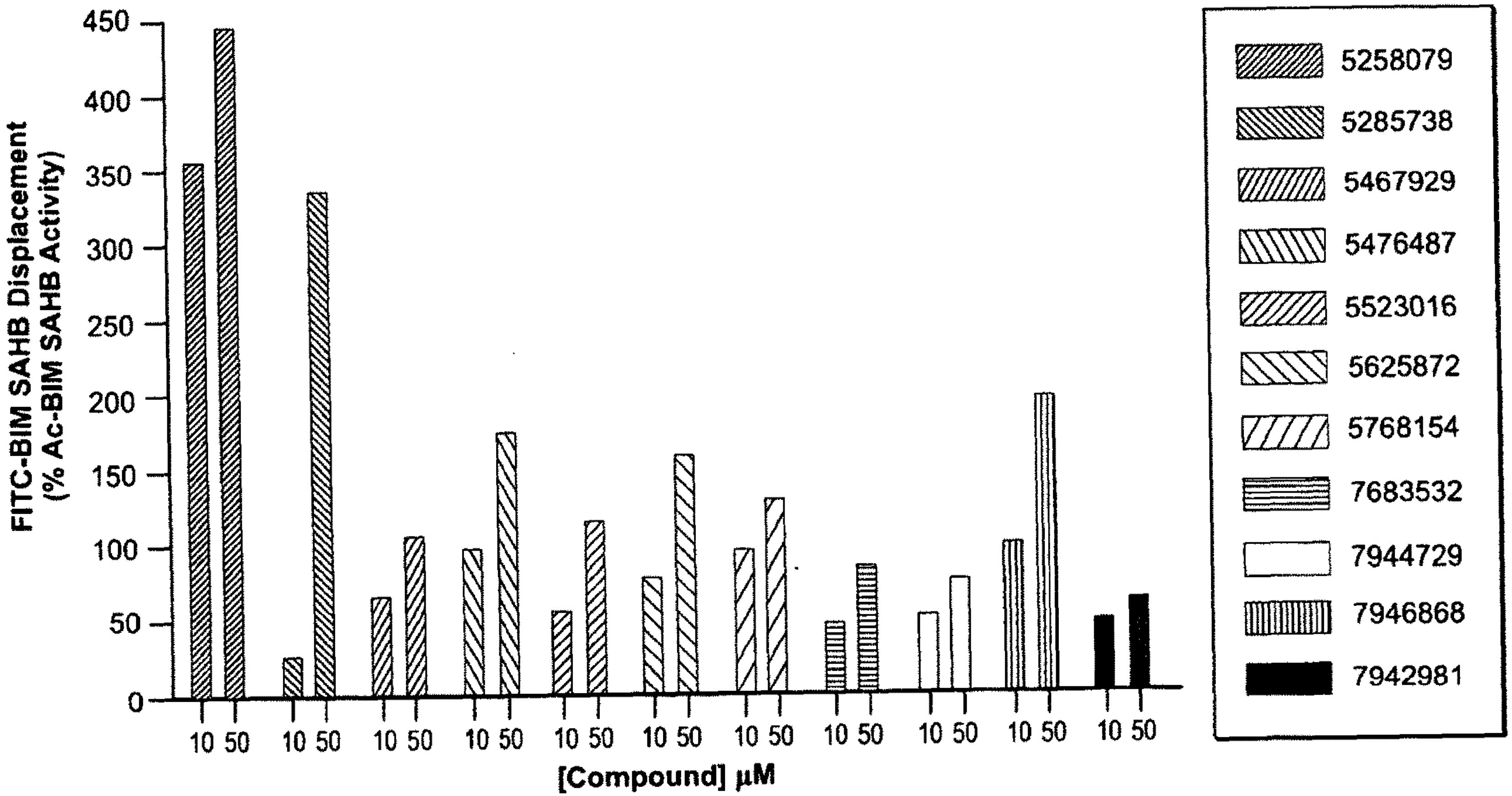


Competitive FPA demonstrating that test compounds effectively and dose-responsively compete with FITC-BIM SAHB for BAX binding as referenced to Ac-BIM SAHB competition (ie. 100% displacement). In several cases, the test molecules exhibit even greater competition for BAX than Ac-BIM SAHB, which is reflected by compound values >100%.

FIG. 12A

BIM SAHB directly initiates BAX oligomerization in vitro as demonstrated by size exclusion chromatography-based monitoring of the monomer-oligomer states.

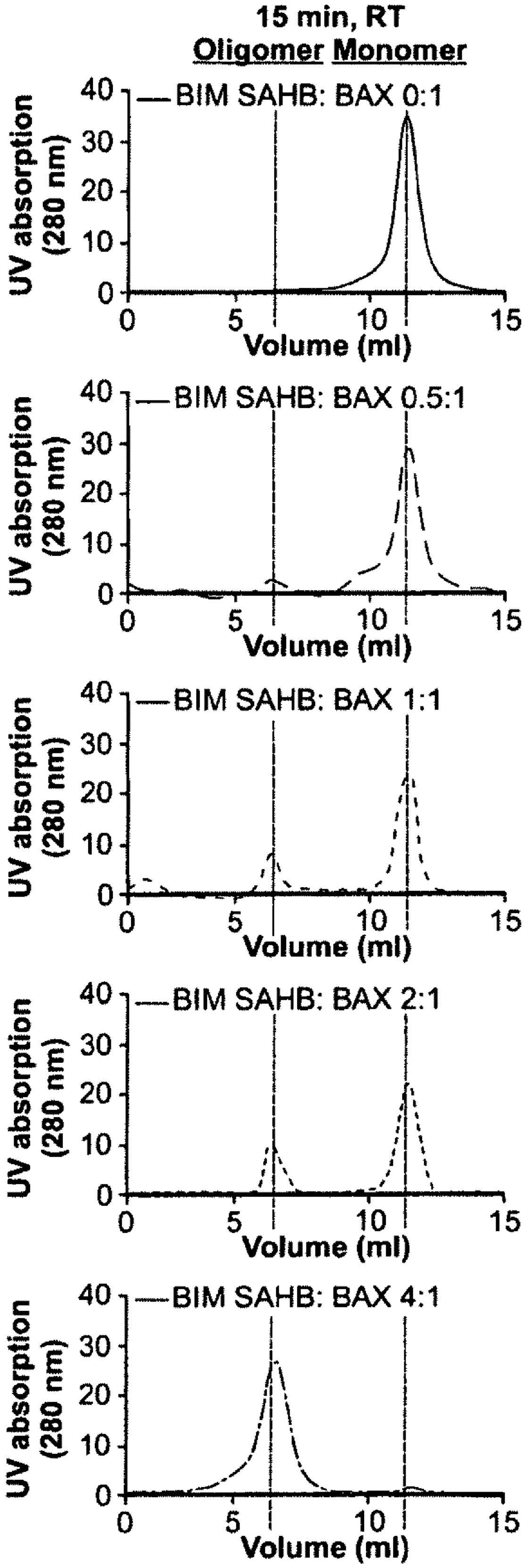

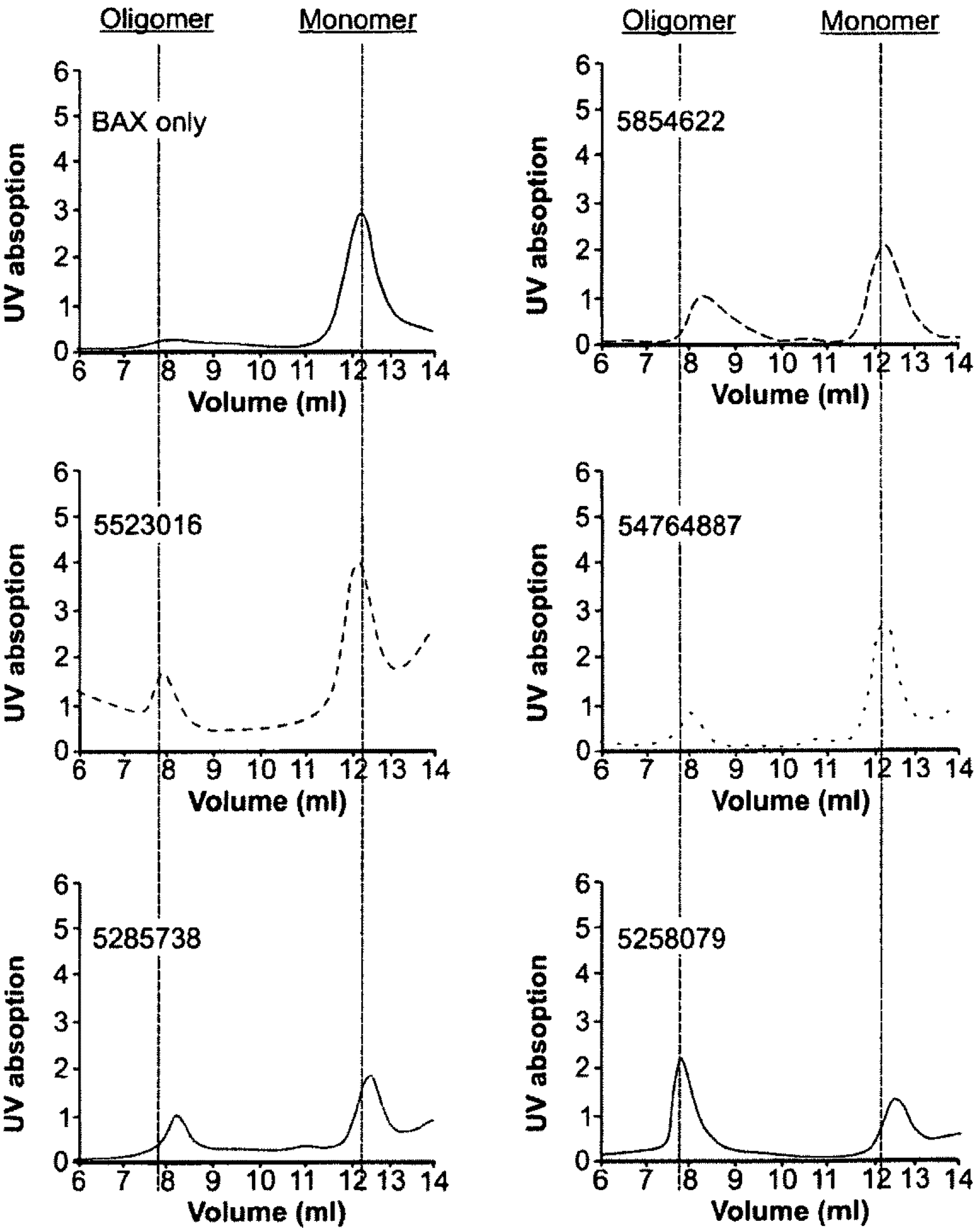


Test compounds trigger BAX oligomerization as detected by conversion of the BAX monomer to its oligomeric form using size-exclusion chromatography.

FIG. 12B

FIG. 13

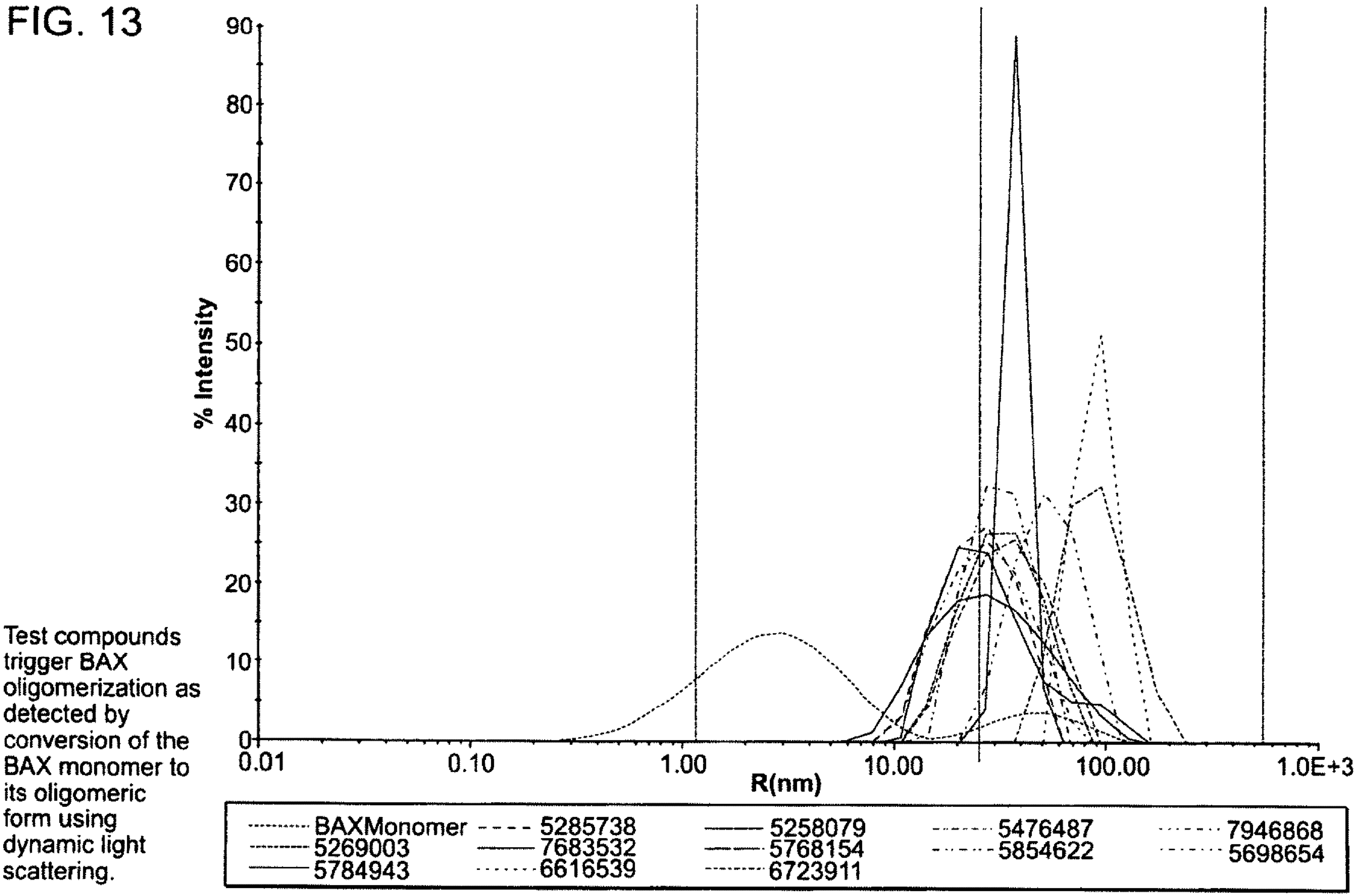


Test compounds trigger BAX oligomerization as detected by conversion of the BAX monomer to its oligomeric form using dynamic light scattering.

FIG. 14

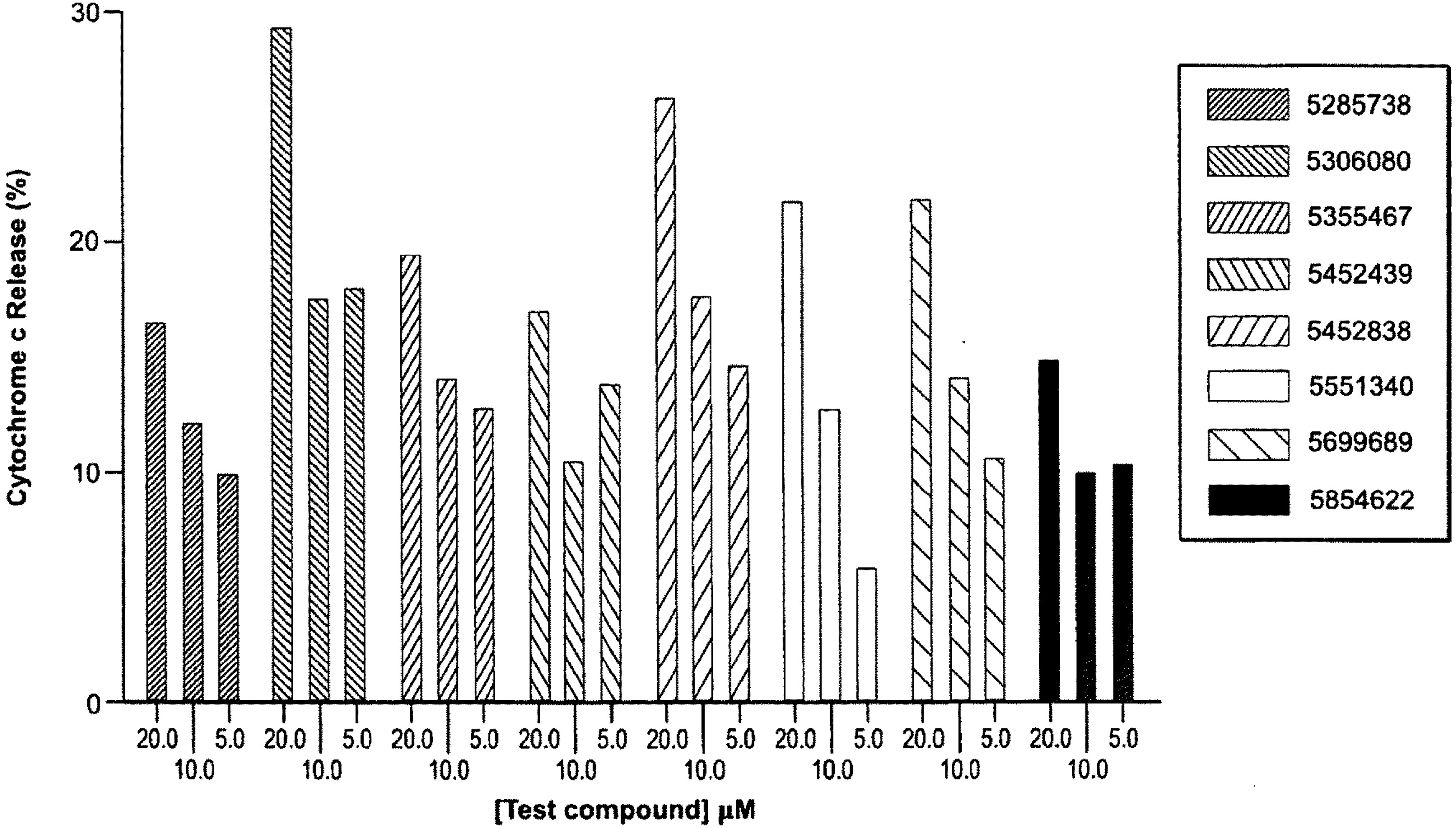


Test compounds dose-responsively trigger recombinant BAX-mediated cytochrome *c* release from $Bax^{-/-}Bak^{-/-}$ mitochondria, as detected by ELISA assay.

Test ligands induce BAX-dependent apoptosis. Compounds 5285738 (A) and 5258079 (B) selectively trigger dose-responsive apoptosis in BAX-reconstituted Bax$^{-/-}$ Bak$^{-/-}$ mouse embryo fibroblasts (DKO MEF), but not in DKO MEFs.

FIG. 15A

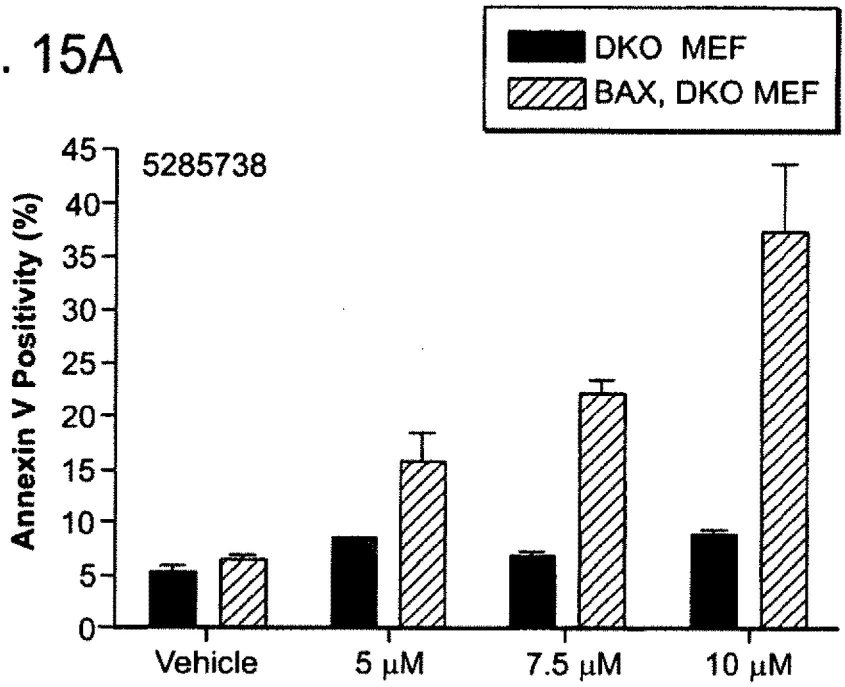


FIG. 15B

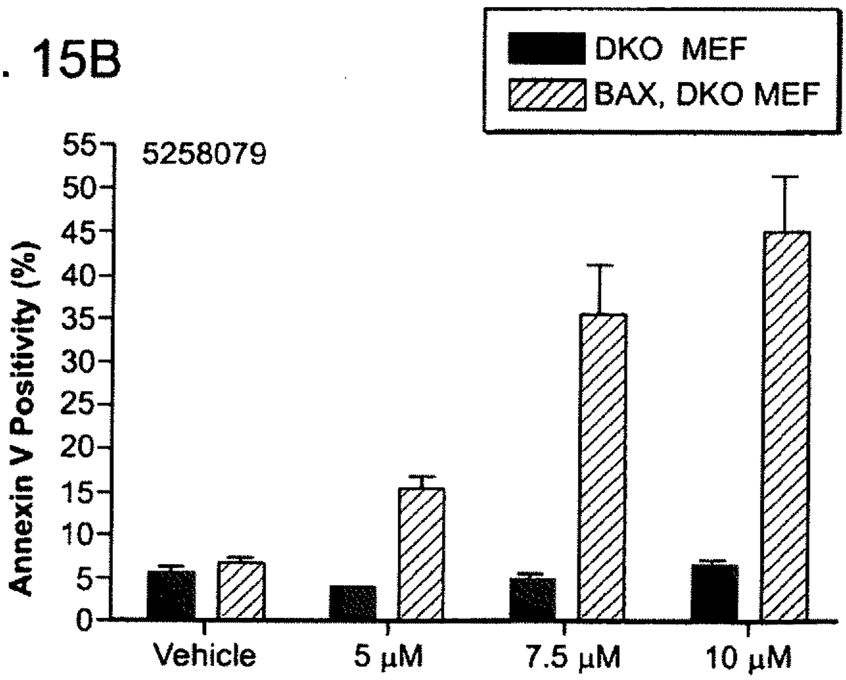

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050250680 A
- US 5223409 A, Ladner
- US 409 A, Ladner
- US 5166208 A, Kelly
- US 4584377 A, Yokoi
- US 5155208 A, Kelly
- US 5679377 A
- US 5916597 A
- US 5912015 A
- US 5989463 A
- US 5128326 A
- WO 9915154 A
- WO 9920253 A
- US 61136906 B


### Non-patent literature cited in the description


- **THOMPSON, C. B.** Apoptosis in the pathogenesis and treatment of disease. *Science,* 1995, vol. 267, 1456-1462
- **JACOBSON, M. D. ; WEIL, M. ; RAFF, M. C.** Programmed cell death in animal development. *Cell,* 1997, vol. 88, 347-354
- **ADAMS, J. M. ; CORY, S.** The Bcl-2 apoptotic switch in cancer development and therapy. *Oncogene,* 2007, vol. 26, 1324-1337
- **KRAMMER, P. H.** CD95's deadly mission in the immune system. *Nature,* 2000, vol. 407, 789-795
- **YUAN, J. ; YANKNER, B. A.** Apoptosis in the nervous system. *Nature,* 2000, vol. 407, 802-809
- **KANG, P. M. ; IZUMO, S.** Apoptosis in heart: basic mechanisms and implications in cardiovascular diseases. *Trends Mol Med,* 2003, vol. 9, 177-182
- **DANIAL, N. N. ; KORSMEYER, S. J.** Cell death: critical control points. *Cell,* 2004, vol. 116, 205-219
- **YOULE, R. J. ; STRASSER, A.** The BCL-2 protein family: opposing activities that mediate cell death. *Nat Rev Mol Cell Biol,* 2008, vol. 9, 47-59
- **WEI, M. C. ; ZONG, W. X. ; CHENG, E. H. ; LINDSTEN, T. ; PANOUTSAKOPOULOU, V. ; ROSS, A. J. ; ROTH, K. A. ; MACGREGOR, G. R. ; THOMPSON, C. B. ; KORSMEYER, S. J.** Proapoptotic BAX and BAK: a requisite gateway to mitochondrial dysfunction and death. *Science,* 2001, vol. 292, 727-730
- **GREEN, D. R.** Apoptotic pathways: ten minutes to dead. *Cell,* 2005, vol. 121, 671-674
- **LETAI, A. ; BASSIK, M. C. ; WALENSKY, L. D. ; SORCINELLI, M. D. ; WEILER, S. ; KORSMEYER, S. J.** Distinct BH3 domains either sensitize or activate mitochondrial apoptosis, serving as prototype cancer therapeutics. *Cancer Cell,* 2002, vol. 2, 183-192
- **CHEN, L. ; WILLIS, S. N. ; WEI, A. ; SMITH, B. J. ; FLETCHER, J. I. ; HINDS, M. G. ; COLMAN, P. M. ; DAY, C. L. ; ADAMS, J. M. ; HUANG, D. C.** Differential targeting of prosurvival Bcl-2 proteins by their BH3-only ligands allows complementary apoptotic function. *Mol Cell,* 2005, vol. 17, 393-403
- **RIEDL, S. J. ; SALVESEN, G. S.** The apoptosome: signalling platform of cell death. *Nat Rev Mol Cell Biol,* 2007, vol. 8, 405-413
- **LI, P. ; NIJHAWAN, D. ; BUDIHARDJO, I. ; SRINIVASULA, S. M. ; AHMAD, M. ; ALNEMRI, E. S. ; WANG, X.** Cytochrome c and dATP-dependent formation of Apaf-1/caspase-9 complex initiates an apoptotic protease cascade. *Cell,* 1997, vol. 91, 479-489
- **LUO, X. ; BUDIHARDJO, I. ; ZOU, H. ; SLAUGHTER, C. ; WANG, X.** Bid, a Bcl2 interacting protein, mediates cytochrome c release from mitochondria in response to activation of cell surface death receptors. *Cell,* 1998, vol. 94, 481-490
- **WALENSKY, L. D. ; KUNG, A. L. ; ESCHER, I. ; MALIA, T. J. ; BARBUTO, S. ; WRIGHT, R. D. ; WAGNER, G. ; VERDINE, G. L. ; KORSMEYER, S. J.** Activation of apoptosis in vivo by a hydrocarbon-stapled BH3 helix. *Science,* 2004, vol. 305, 1466-1470
- **WALENSKY, L. D. ; PITTER, K. ; MORASH, J. ; OH, K. J. ; BARBUTO, S. ; FISHER, J. ; SMITH, E. ; VERDINE, G. L. ; KORSMEYER, S. J.** *Molecular Cell,* 2006, vol. 24, 199-210
- **GAVATHIOTIS, E. ; SUZUKI, M. ; DAVIS, M. L. ; PITTER, K. ; BIRD, G. H. ; KATZ, S. G. ; TU, H.-C. ; KIM, H. ; CHENG, E. H.-Y. ; TJANDRA, N.** *Nature,* 2008
- *Hit2Lead | Search | Screening Compounds | Search by ID; 5258079; 3-methyl-1-(4-phenyl-1,3-thiazol-2-yl)-1H-pyrazole-4,5-dione 4-[(4-ethoxyphenyl)hydrazine,* 28 July 2005

- *SID 3315682 - PubChem,* 28 July 2005, https://pubchem.ncbi.nlm.nih.gov/substance/3315682
- **PETROS et al.** *Biochimica et Biophysica Acta,* 2004, vol. 1644, 83-94
- **SUZUKI et al.** *Cell,* 2000, vol. 103, 645-654
- **KROEMER et al.** *Nature Med,* 1997, vol. 6 (614), 20
- **CHITTENDEN et al.** *EMBO,* 1995, vol. 14, 5589
- **WANG et al.** *Genes Dev,* 1996, vol. 10, 2859
- **LETAI, A. et al.** *Cancer Cell,* 2002, vol. 2, 183-192
- **WALENSKY.** *Mol Cell,* 2006
- **MUCHMORE, S. W. ; SATTLER, M. ; LIANG, H. ; MEADOWS, R. P. ; HARLAN, J. E. ; YOON, H. S. ; NETTESHEIM, D. ; CHANG, B. S. ; THOMPSON, C. B. ; WONG, S. L.** X-ray and NMR structure of human Bcl-xL, an inhibitor of programmed cell death. *Nature,* 1996, vol. 381, 335-341
- **NOURAINI, S. ; SIX, E. ; MATSUYAMA, S. ; KRAJEWSKI, S. ; REED, J. C.** The putative pore-forming domain of Bax regulates mitochondrial localization and interaction with Bcl-X(L). *Mol Cell Biol,* 2000, vol. 20, 1604-1615
- **NARITA, M. ; SHIMIZU, S. ; ITO, T. ; CHITTENDEN, T. ; LUTZ, R. J. ; MATSUDA, H. ; TSUJIMOTO, Y.** Bax interacts with the permeability transition pore to induce permeability transition and cytochrome c release in isolated mitochondria. *Proc Natl Acad Sci U S A,* 1998, vol. 95, 14681-14686
- **PETROS, A. M. ; NETTESHEIM, D. G. ; WANG, Y. ; OLEJNICZAK, E. T. ; MEADOWS, R. P. ; MACK, J. ; SWIFT, K. ; MATAYOSHI, E. D. ; ZHANG, H. ; THOMPSON, C. B.** Rationale for Bcl-xL/Bad peptide complex formation from structure, mutagenesis, and biophysical studies. *Protein Sci,* 2000, vol. 9, 2528-2534
- **DENISOV, A. Y. ; CHEN, G. ; SPRULES, T. ; MOLDOVEANU, T. ; BEAUPARLANT, P. ; GEHRING, K.** Structural model of the BCL-w-BID peptide complex and its interactions with phospholipid micelles. *Biochemistry,* 2006, vol. 45, 2250-2256
- **DAY, C. L. ; CHEN, L. ; RICHARDSON, S. J. ; HARRISON, P. J. ; HUANG, D. C. ; HINDS, M. G.** Solution structure of prosurvival Mcl-1 and characterization of its binding by proapoptotic BH3-only ligands. *J Biol Chem,* 2005, vol. 280, 4738-4744
- **SMITS, C. ; CZABOTAR, P. E. ; HINDS, M. G. ; DAY, C. L.** Structural plasticity underpins promiscuous binding of the prosurvival protein AI. *Structure,* 2008, vol. 16, 818-829
- **WALENSKY, L. D. et al.** Activation of apoptosis in vivo by a hydrocarbon-stapled BH3 helix. *Science,* 2004, vol. 305, 1466-1470
- **OLTERSDORF, T. et al.** An inhibitor of Bcl-2 family proteins induces regression of solid tumours. *Nature,* 2005, vol. 435, 677-681
- **NGUYEN, M. et al.** Small molecule obatoclax (GX15-070) antagonizes MCL-1 and overcomes MCL-1-mediated resistance to apoptosis. *Proc Natl Acad Sci USA,* 2007, vol. 104, 19512-195
- **SUZUKI, M. ; YOULE, R. J. ; TJANDRA, N.** Structure of Bax: coregulation of dimer formation and intracellular localization. *Cell,* 2000, vol. 103, 645-654
- **MCDONNELL, J. M. et al.** Solution structure of the proapoptotic molecule BID: a structural basis for apoptotic agonists and antagonists. *Cell,* 1999, vol. 96, 625-634
- **CHOU, J. J. et al.** Solution structure of BID, an intracellular amplifier of apoptotic signaling. *Cell,* 1999, vol. 96, 615-624
- **PETROS, A. M. et al.** Solution structure of the antiapoptotic protein bcl-2. *Proc Natl Acad Sci USA,* 2001, vol. 98, 3012-3017
- **MUCHMORE, S. W. et al.** X-ray and NMR structure of human Bcl-xL, an inhibitor of programmed cell death. *Nature,* 1996, vol. 381, 335-341
- **ITO, T. ; DENG, X. ; CARR, B. ; MAY, W. S.** Bcl-2 phosphorylation required for anti-apoptosis function. *The Journal of biological chemistry,* 1997, vol. 272, 11671-11673
- **CHENG, E. H. ; KIRSCH et al.** Conversion of Bcl-2 to a Bax-like death effector by caspases. *Science,* 1997, vol. 278, 1966-1968
- **LI, H. et al.** Cleavage of BID by caspase 8 mediates the mitochondrial damage in the Fas pathway of apoptosis. *Cell,* 1998, vol. 94, 491-501
- **CARTRON, P. F. et al.** The p18 truncated form of Bax behaves like a Bcl-2 homology domain 3-only protein. *J Biol Chem,* 2004, vol. 279, 11503-11512
- **WOOD, D. E. et al.** Bax cleavage is mediated by calpain during drug-induced apoptosis. *Oncogene,* 1998, vol. 17, 1069-1078
- **SATTLER, M. et al.** Structure of Bcl-xL-Bak peptide complex: recognition between regulators of apoptosis. *Science,* 1997, vol. 275, 983-986
- **HSU, Y. T. ; YOULE, R. J.** Bax in murine thymus is a soluble monomeric protein that displays differential detergent-induced conformations. *J Biol Chem,* 1998, vol. 273, 10777-1078
- **SOANE, L. ; FISKUM, G.** Inhibition of mitochondrial neural cell death pathways by protein transduction of Bcl-2 family proteins. *J Bioenerg Biomembr,* 2005, vol. 37, 179-190
- **TAN, Y. J. ; BEERHEIDE, W. ; TING, A. E.** Biophysical characterization of the oligomeric state of Bax and its complex formation with Bcl-XL. *Biochem Biophys Res Commun,* 1999, vol. 255, 334-339

- **SCHINZEL, A. ; KAUFMANN, T. ; SCHULER, M. ; MARTINALBO, J. ; GRUBB, D. ; BORNER, C.** Conformational control of Bax localization and apoptotic activity by Pro168. *J Cell Biol,* 2004, vol. 164, 1021-1032
- **HSU, Y. T. ; YOULE, R. J.** Bax in murine thymus is a soluble monomeric protein that displays differential detergent-induced conformations. *J Biol Chem,* 1998, vol. 273, 10777-10783
- **YETHON, J. A. ; EPAND, R. F. ; LEBER, B. ; EPAND, R. M. ; ANDREWS, D. W.** Interaction with a membrane surface triggers a reversible conformational change in Bax normally associated with induction of apoptosis. *J Biol Chem,* 2003, vol. 278, 48935-48941
- **GAVATHIOTIS, E. ; SUZUKI, M. ; DAVIS, M.L. ; PITTER, K. ; BIRD, G.H. ; KATZ, S.G. ; TU, H.-C. ; KIM, H. ; CHENG, E. H.-Y. ; TJANDRA, N.** BAX activation is initiated at a novel interaction site. *Nature,* 2008
- **WALENSKY, L. D. et al.** A stapled BID BH3 helix directly binds and activates BAX. *Mol Cell,* 2006, vol. 24, 199-210
- **MENG et al.** *J. Comp. Chem.,* 1992, vol. 13, 505-524
- **LAM.** *Anticancer Drug Des,* 1997, vol. 12, 145
- **RAREY et al.** *J. Mol. Biol.,* 1996, vol. 261, 470-489
- **KUNTZ et al.** *J. Mol. Biol,* 1982, vol. 161, 269-288
- **BOEHM.** *J. Comp. Aid. Mol. Des.,* 1992, vol. 6, 61-78
- **BARTLETT et al.** Molecular Recognition in Chemical and Biological Problems. *The Royal Chem. Soc.,* 1989, vol. 78, 182-196
- **MIRANKER et al.** *Proteins,* 1991, vol. 11, 29-34
- **DEWITT et al.** *Proc. Natl. Acad. Sci. U.S.A.,* 1993, vol. 90, 6909
- **ERB et al.** *Proc. Natl. Acad. Sci. USA,* 1994, vol. 91, 11422
- **ZUCKERMANN et al.** *J. Med. Chem.,* 1994, vol. 37, 2678
- **CHO et al.** *Science,* 1993, vol. 261, 1303
- **CARRELL et al.** *Angew. Chem. Int. Ed. Engl.,* 1994, vol. 33, 2059
- **CARELL et al.** *Angew. Chem. Int. Ed. Engl.,* 1994, vol. 33, 2061
- **GALLOP et al.** *J. Med. Chem.,* 1994, vol. 37, 1233
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 87, 6378-6382
- **FELICI.** *J. Mol. Biol.,* 1991, vol. 222, 301-310
- **GREER.** *Science,* 1985, vol. 228, 1055
- **BUNDELL et al.** *Eur. J. Biochem.,* 1988, vol. 172, 513
- **KNIGHTON et al.** *Science,* 1992, vol. 258, 130-135, http://biochem.vt.edu/courses/modeling/homology.htm
- **GOODFORD, P. J.** A Computational Procedure for Determining Energetically Favorable Binding Sites on Biologically Important Macromolecules. *J. Med. Chem.,* 1985, vol. 28, 849-857
- **MIRANKER, A. ; M. KARPLUS.** Functionality Maps of Binding Sites: A Multiple Copy Simultaneous Search Method. *Proteins: Structure. Function and Genetics,* 1991, vol. 11, 29-34
- **GOODSELL, D. S. ; A. J. OLSEN.** Automated Docking of Substrates to Proteins by Simulated Annealing. *Proteins: Structure. Function, and Genetics,* 1990, vol. 8, 195-202
- **KUNTZ, I. D. et al.** A Geometric Approach to Macromolecule-Ligand Interactions. *J. Mol. Biol.,* 1982, vol. 161, 269-288
- CAVEAT: A Program to Facilitate the Structure-Derived Design of Biologically Active Molecules. **BARTLETT, P. A. et al.** Molecular Recognition in Chemical and Biological Problems. Royal Chem. Soc, 1989, vol. 78, 182-196
- **MARTIN, Y. C.** 3D Database Searching in Drug Design. *J. Med. Chem.,* 1992, vol. 35, 2145-2154
- **BOHM, H.-J.** The Computer Program LUDI: A New Method for the De Novo Design of Enzyme Inhibitors. *J. Comp. Aid. Molec. Design,* 1992, vol. 6, 61-78
- **NISHIBATA, Y. ; A. ITAI.** *Tetrahedron,* 1991, vol. 47, 8985
- **COHEN, N. C. et al.** Molecular Modeling Software and Methods for Medicinal Chemistry. *J. Med. Chem.,* 1990, vol. 33, 883-894
- **NAVIA, M. A. ; M. A. MURCKO.** The Use of Structural Information in Drug Design. *Current Opinions in Structural Biology,* 1992, vol. 2, 202-210
- **SHUKERS, S. B. et al.** *Science,* 1996, vol. 274, 1531-1534
- **FEJZO J ; LEPRE CA ; PENG JW ; BEMIS GW ; AJAY ; MURCKO MA ; MOORE JM.** *Chem Biol,* October 1999, vol. 6 (10), 755-69
- **NIENABER, V. L. et al.** Fragments of Active Structures. *Nat. Biotechnol.,* 2000, vol. 18, 1105-1108, www.stromix.com
- **CONGREVE, M. S. et al.** *Angew. Chem., Int. Ed.,* 2003, vol. 42, 4479-4482
- **CONGREVE et al.** *J. Med. Chem.,* 2008, vol. 51 (13), 3661-3680

- NMR-based screening: a powerful tool in fragment-based drug discovery. **KLAGESA, J. ; COLESB, M. ; KESSLERA, H.** Exploiting Chemical Diversity for Drug Discovery. RSC Publishing, 2006
- **ERLANSON, D.A. ; WELLS, J.A. ; BRAISTED, A.C.** TETHERING: Fragment-Based Drug Discovery. *Annual Review of Biophysics and Biomolecular Structure,* 2004, vol. 33, 199-223
- **ZARTLER ER ; SHAPIRO MJ.** Fragonomics: fragment-based drug discovery. *Curr Opin Chem Biol,* 2005, vol. 9 (4), 366-70
- **SJOLANDER, S. ; URBANICZKY, C.** *Anal. Chem.,* 1991, vol. 63, 2338-2345
- **SZABO et al.** *Curr. Opin. Struct. Biol.,* 1995, vol. 5, 699-705
- **WALENSKY et al.** *Science,* 2004
- **WALENSKY et al.** *Mol Cell,* 2006
- **GAVATHIOTIS et al.** *Nature,* 2008
- **PANDLEY et al.** *J. Antibiot.,* 1981, vol. 3 (11), 1389-401
- **WARNICK-PICKLE et al.** *J. Antibiot.,* 1981, vol. 34 (11), 1402-7
- Principals of Neoplasia. Harrison's: Principals of Internal Medicine. McGraw-Hill, 1814
- **LOVEJOY et al.** *J. Pathol.,* 1997, vol. 181, 130-135
- **WANG et al.** *Ann. Thorac. Surg.,* 1997, vol. 64, 216-219
- **YONO ; SONE.** *Gan To Kagaku Ryoho,* 1997, vol. 24, 489-494
- **GUTMAN ; FIDLER.** *World J. Surg.,* 1995, vol. 19, 226-234
- **WARREN.** *Aliment. Pharmacol. Ther. Supp,* 1996, vol. 12, 45-47
- **POLAKIS.** *Biochim. Biophys. Acta,* 1997, vol. 1332, F127-F147
- **DANKORT ; MULLER.** *Cancer Treat. Res.,* 1996, vol. 83, 71-88
- **AMUNDADITTIR et al.** *Breast Cancer Res. Treat.,* 1996, vol. 39, 119-135
- **RUSSO ; RUSSO.** *Breast Cancer Res. Treat.,* 1996, vol. 39, 7-20
- **ROYAL et al.** *Semin. Oncol.,* 1996, vol. 23, 35-40
- **OYASU.** *Food Chem. Toxicol,* 1995, vol. 33, 747-755
- **JARRETT et al.** *J. Endourol.,* 1995, vol. 9, 1-7
- **APPELBAUM.** *Leukemia,* 1997, vol. 11 (4), S15-S17
- **DONEHOWER.** *Semin. Cancer Biol.,* 1996, vol. 7, 269-278
- **SHOEMAKER et al.** *Biochem. Biophys. Acta,* 1997, vol. 1332, F25-F48
- **FREY.** *Methods,* 1997, vol. 12, 173-188
- **VAICKUS, L.** *Crit Rev. in Oncol./Hemotol.,* 1991, vol. 11, 267-97
- Guidance for Industry Estimating the Maximum Safe Starting Dose in Initial Clinical Trials for Therapeutics in Adult Healthy Volunteers. Pharmacology and Toxicology. U.S. Department of Health and Human Services Food and Drug Administration Center for Drug Evaluation and Research (CDER), July 2005
- Goodman & Gilman's The Pharmacological Basis Of Basis Of Therapeutics. Mc-Graw-Hill, 2001
- Physician's Desk Reference. 2006
- **SEFTON.** *CRC Crit. Ref Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574
- Medical Applications of Controlled Release. CRC Pres, 1974
- Controlled Drug Bioavailability, Drug Product Design and Performance. Wiley, 1984
- **RANGER ; PEPPAS.** *J. Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 71, 105
- **GOODSON.** *Medical Applications of Controlled Release,* 1984, vol. 2, 115-138
- **LANGER.** *Science,* 1990, vol. 249, 1527-1533
- **BIRD, G.H. ; BERNAL, F. ; PITTER, K. ; WALENSKY, L.D.** Synthesis and biophysical characterization of stabilized alpha-helices of BCL-2 domains. *Methods in Enzymology,* 2008, vol. 446, 369-386
- **PITTER, K. ; BERNAL, F. ; LABELLE, J.L. ; WALENSKY, L.D.** Dissection of the BCL-2 family signaling network using stabilized alpha-helices of BCL-2 domains. *Methods in Enzymology,* 2008, vol. 446, 387-408
- **CHENG, E. H. et al.** BCL-2, BCL-X(L) sequester BH3 domain-only molecules preventing BAX- and BAK-mediated mitochondrial apoptosis. *Mol Cell,* 2001, vol. 8, 705-711
- **KIM, H. et al.** Hierarchical regulation of mitochondrion-dependent apoptosis by BCL-2 subfamilies. *Nat Cell Biol,* 2006, vol. 8, 1348-1358

SEQUENCE LISTING

<110> DANA FARBER CANCER INSTITUTE, INC.

<120> CHEMICAL MODULATORS OF PRO-APOPTOTIC BAX AND BCL-2 POLYPEPTIDES

<130> 22455EP1

<140>
<141> 2009-10-09

<150> US 61/136,906
<151> 2008-10-10

<160> 39

<170> PatentIn version 3.5

<210> 1
<211> 192
<212> PRT
<213> Homo sapiens

<400> 1

```
Met Asp Gly Ser Gly Glu Gln Pro Arg Gly Gly Gly Pro Thr Ser Ser
1               5                   10                  15

Glu Gln Ile Met Lys Thr Gly Ala Leu Leu Leu Gln Gly Phe Ile Gln
            20                  25                  30

Asp Arg Ala Gly Arg Met Gly Gly Glu Ala Pro Glu Leu Ala Leu Asp
        35                  40                  45

Pro Val Pro Gln Asp Ala Ser Thr Lys Lys Leu Ser Glu Cys Leu Lys
    50                  55                  60

Arg Ile Gly Asp Glu Leu Asp Ser Asn Met Glu Leu Gln Arg Met Ile
65                  70                  75                  80

Ala Ala Val Asp Thr Asp Ser Pro Arg Glu Val Phe Phe Arg Val Ala
                85                  90                  95

Ala Asp Met Phe Ser Asp Gly Asn Phe Asn Trp Gly Arg Val Val Ala
            100                 105                 110

Leu Phe Tyr Phe Ala Ser Lys Leu Val Leu Lys Ala Leu Cys Thr Lys
        115                 120                 125

Val Pro Glu Leu Ile Arg Thr Ile Met Gly Trp Thr Leu Asp Phe Leu
    130                 135                 140

Arg Glu Arg Leu Leu Gly Trp Ile Gln Asp Gln Gly Gly Trp Asp Gly
145                 150                 155                 160

Leu Leu Ser Tyr Phe Gly Thr Pro Thr Trp Gln Thr Val Thr Ile Phe
                165                 170                 175

Val Ala Gly Val Leu Thr Ala Ser Leu Thr Ile Trp Lys Lys Met Gly
            180                 185                 190
```

<210> 2
<211> 198
<212> PRT
<213> Homo sapiens

<400> 2

Met Ala Lys Gln Pro Ser Asp Val Ser Ser Glu Cys Asp Arg Glu Gly
1               5                   10                  15

Arg Gln Leu Gln Pro Ala Glu Arg Pro Pro Gln Leu Arg Pro Gly Ala
            20                  25                  30

Pro Thr Ser Leu Gln Thr Glu Pro Gln Gly Asn Pro Glu Gly Asn His
        35                  40                  45

Gly Gly Glu Gly Asp Ser Cys Pro His Gly Ser Pro Gln Gly Pro Leu
    50                  55                  60

Ala Pro Pro Ala Ser Pro Gly Pro Phe Ala Thr Arg Ser Pro Leu Phe
65                  70                  75                  80

Ile Phe Met Arg Arg Ser Ser Leu Leu Ser Arg Ser Ser Ser Gly Tyr
                85                  90                  95

Phe Ser Phe Asp Thr Asp Arg Ser Pro Ala Pro Met Ser Cys Asp Lys
            100                 105                 110

Ser Thr Gln Thr Pro Ser Pro Pro Cys Gln Ala Phe Asn His Tyr Leu
        115                 120                 125

Ser Ala Met Ala Ser Met Arg Gln Ala Glu Pro Ala Asp Met Arg Pro
    130                 135                 140

Glu Ile Trp Ile Ala Gln Glu Leu Arg Arg Ile Gly Asp Glu Phe Asn
145                 150                 155                 160

Ala Tyr Tyr Ala Arg Arg Val Phe Leu Asn Asn Tyr Gln Ala Ala Glu
                165                 170                 175

Asp His Pro Arg Met Val Ile Leu Arg Leu Leu Arg Tyr Ile Val Arg
            180                 185                 190

Leu Val Trp Arg Met His
        195

<210> 3
<211> 22
<212> PRT
<213> Homo sapiens

<400> 3

Glu Ile Trp Ile Ala Gln Glu Leu Arg Arg Ile Gly Asp Glu Phe Asn
1 5 10 15

Ala Tyr Tyr Ala Arg Arg
20

<210> 4
<211> 195
<212> PRT
<213> Homo sapiens

<400> 4

Met Asp Cys Glu Val Asn Asn Gly Ser Ser Leu Arg Asp Glu Cys Ile
1 5 10 15

Thr Asn Leu Leu Val Phe Gly Phe Leu Gln Ser Cys Ser Asp Asn Ser
20 25 30

Phe Arg Arg Glu Leu Asp Ala Leu Gly His Glu Leu Pro Val Leu Ala
35 40 45

Pro Gln Trp Glu Gly Tyr Asp Glu Leu Gln Thr Asp Gly Asn Arg Ser
50 55 60

Ser His Ser Arg Leu Gly Arg Ile Glu Ala Asp Ser Glu Ser Gln Glu
65 70 75 80

Asp Ile Ile Arg Asn Ile Ala Arg His Leu Ala Gln Val Gly Asp Ser
85 90 95

Met Asp Arg Ser Ile Pro Pro Gly Leu Val Asn Gly Leu Ala Leu Gln
100 105 110

Leu Arg Asn Thr Ser Arg Ser Glu Glu Asp Arg Asn Arg Asp Leu Ala
115 120 125

Thr Ala Leu Glu Gln Leu Leu Gln Ala Tyr Pro Arg Asp Met Glu Lys
130 135 140

Glu Lys Thr Met Leu Val Leu Ala Leu Leu Leu Ala Lys Lys Val Ala
145 150 155 160

Ser His Thr Pro Ser Leu Leu Arg Asp Val Phe His Thr Thr Val Asn
165 170 175

Phe Ile Asn Gln Asn Leu Arg Thr Tyr Val Arg Ser Leu Ala Arg Asn
180 185 190

Gly Met Asp
195

<210> 5
<211> 25
<212> PRT
<213> Homo sapiens

<400> 5

Glu Ser Gln Glu Asp Ile Ile Arg Asn Ile Ala Arg His Leu Ala Gln
1 5 10 15

Val Gly Asp Ser Met Asp Arg Ser Ile
20 25

<210> 6
<211> 193
<212> PRT
<213> Homo sapiens

<400> 6

```
Met Ala Arg Ala Arg Gln Glu Gly Ser Ser Pro Glu Pro Val Glu Gly
1               5                   10                  15

Leu Ala Arg Asp Gly Pro Arg Pro Phe Pro Leu Gly Arg Leu Val Pro
            20                  25                  30

Ser Ala Val Ser Cys Gly Leu Cys Glu Pro Gly Leu Ala Ala Ala Pro
        35                  40                  45

Ala Ala Pro Thr Leu Leu Pro Ala Ala Tyr Leu Cys Ala Pro Thr Ala
    50                  55                  60

Pro Pro Ala Val Thr Ala Ala Leu Gly Gly Ser Arg Trp Pro Gly Gly
65                  70                  75                  80

Pro Arg Ser Arg Pro Arg Gly Pro Arg Pro Asp Gly Pro Gln Pro Ser
                85                  90                  95

Leu Ser Leu Ala Glu Gln His Leu Glu Ser Pro Val Pro Ser Ala Pro
            100                 105                 110

Gly Ala Leu Ala Gly Gly Pro Thr Gln Ala Ala Pro Gly Val Arg Gly
        115                 120                 125

Glu Glu Glu Gln Trp Ala Arg Glu Ile Gly Ala Gln Leu Arg Arg Met
    130                 135                 140

Ala Asp Asp Leu Asn Ala Gln Tyr Glu Arg Arg Arg Gln Glu Glu Gln
145                 150                 155                 160

Gln Arg His Arg Pro Ser Pro Trp Arg Val Leu Tyr Asn Leu Ile Met
                165                 170                 175

Gly Leu Leu Pro Leu Pro Arg Gly His Arg Ala Pro Glu Met Glu Pro
            180                 185                 190

Asn
```

<210> 7
<211> 28
<212> PRT
<213> Homo sapiens

<400> 7

Glu Glu Glu Gln Trp Ala Arg Glu Ile Gly Ala Gln Leu Arg Arg Met
1               5                   10                  15

Ala Asp Asp Leu Asn Ala Gln Tyr Glu Arg Arg Arg
            20                  25

<210> 8
<211> 27
<212> PRT
<213> Homo sapiens

<400> 8

Gln Asp Ala Ser Thr Lys Lys Leu Ser Glu Cys Leu Lys Arg Ile Gly
1               5                   10                  15

Asp Glu Leu Asp Ser Asn Met Glu Leu Gln Arg
            20                  25

<210> 9
<211> 15
<212> PRT
<213> Artificial Sequence

<220>
<223> Description of Artificial Sequence: Synthetic Consensus sequence

<220>
<221> MOD_RES
<222> (1)..(1)
<223> Ile or Leu

<220>
<221> MOD_RES
<222> (2)..(2)
<223> Ala, Ser, or Gly

<220>
<221> MOD_RES
<222> (3)..(4)
<223> Any amino acid

<220>
<221> MOD_RES
<222> (6)..(6)
<223> Lys or Arg

<220>
<221> MOD_RES
<222> (8)..(8)
<223> Ile, Met, or Val

<220>
<221> MOD_RES
<222> (10)..(10)
<223> Asp or Glu

<220>
<221> MOD_RES
<222> (11)..(11)
<223> Leu or Phe

<220>
<221> MOD_RES
<222> (12)..(12)
<223> Asn or Asp

<220>
<221> MOD_RES
<222> (13)..(13)
<223> Ala or Ser

<220>
<221> MOD_RES
<222> (14)..(14)
<223> Any amino acid

<220>
<221> MOD_RES
<222> (15)..(15)
<223> Tyr or Met

<400> 9

```
Xaa Xaa Xaa Xaa Leu Xaa Arg Xaa Asp Xaa Xaa Xaa Xaa Xaa Xaa
1               5                   10                  15
```

<210> 10
<211> 21
<212> PRT
<213> Artificial Sequence

<220>
<223> Description of Artificial Sequence: Synthetic peptide

<220>
<221> MOD_RES
<222> (9)..(9)
<223> Non-natural amino acid used for cross linking

<220>
<221> MOD_RES
<222> (13)..(13)
<223> Non-natural amino acid used for cross linking

<400> 10

```
Ile Trp Ile Ala Gln Glu Leu Arg Xaa Ile Gly Asp Xaa Phe Asn Ala
1               5                   10                  15

Tyr Tyr Ala Arg Arg
            20
```

<210> 11

<211> 20
<212> PRT
<213> Artificial Sequence

<220>
<223> Description of Artificial Sequence: Synthetic peptide

<220>
<221> MOD_RES
<222> (10)..(10)
<223> Non-natural amino acid used for cross linking

<220>
<221> MOD_RES
<222> (14)..(14)
<223> Non-natural amino acid used for cross linking

<400> 11

```
Glu Ile Trp Ile Ala Gln Glu Leu Arg Xaa Ile Gly Asp Xaa Phe Asn
1               5                   10                  15

Ala Tyr Tyr Ala
            20
```

<210> 12
<211> 21
<212> PRT
<213> Artificial Sequence

<220>
<223> Description of Artificial Sequence: Synthetic peptide

<220>
<221> MOD_RES
<222> (2)..(2)
<223> Non-natural amino acid used for cross linking

<220>
<221> MOD_RES
<222> (6)..(6)
<223> Non-natural amino acid used for cross linking

<400> 12

```
Ile Xaa Ile Ala Gln Xaa Leu Arg Arg Ile Gly Asp Glu Phe Asn Ala
1               5                   10                  15

Tyr Tyr Ala Arg Arg
            20
```

<210> 13
<211> 20
<212> PRT
<213> Artificial Sequence

<220>
<223> Description of Artificial Sequence: Synthetic peptide

<220>
<221> MOD_RES
<222> (3)..(3)
<223> Non-natural amino acid used for cross linking

<220>
<221> MOD_RES
<222> (7)..(7)
<223> Non-natural amino acid used for cross linking

<400> 13

```
Glu Ile Xaa Ile Ala Gln Xaa Leu Arg Arg Ile Gly Asp Glu Phe Asn
1               5                   10                  15

Ala Tyr Tyr Ala
            20
```

<210> 14
<211> 21
<212> PRT
<213> Artificial Sequence

<220>
<223> Description of Artificial Sequence: Synthetic peptide

<220>
<221> MOD_RES
<222> (3)..(3)
<223> Non-natural amino acid used for cross linking

<220>
<221> MOD_RES
<222> (7)..(7)
<223> Non-natural amino acid used for cross linking

<400> 14

```
Ile Trp Xaa Ala Gln Glu Xaa Arg Arg Ile Gly Asp Glu Phe Asn Ala
1               5                   10                  15

Tyr Tyr Ala Arg Arg
            20
```

<210> 15
<211> 20
<212> PRT
<213> Artificial Sequence

<220>
<223> Description of Artificial Sequence: Synthetic peptide

<220>

<221> MOD_RES
<222> (4)..(4)
<223> Non-natural amino acid used for cross linking

<220>
<221> MOD_RES
<222> (8)..(8)
<223> Non-natural amino acid used for cross linking

<400> 15

```
Glu Ile Trp Xaa Ala Gln Glu Xaa Arg Arg Ile Gly Asp Glu Phe Asn
1               5                   10                  15

Ala Tyr Tyr Ala
            20
```

<210> 16
<211> 21
<212> PRT
<213> Artificial Sequence

<220>
<223> Description of Artificial Sequence: Synthetic peptide

<220>
<221> MOD_RES
<222> (4)..(4)
<223> Non-natural amino acid used for cross linking

<220>
<221> MOD_RES
<222> (8)..(8)
<223> Non-natural amino acid used for cross linking

<400> 16

```
Ile Trp Ile Xaa Gln Glu Leu Xaa Arg Ile Gly Asp Glu Phe Asn Ala
1               5                   10                  15

Tyr Tyr Ala Arg Arg
            20
```

<210> 17
<211> 20
<212> PRT
<213> Artificial Sequence

<220>
<223> Description of Artificial Sequence: Synthetic peptide

<220>
<221> MOD_RES
<222> (5)..(5)
<223> Non-natural amino acid used for cross linking

<220>
<221> MOD_RES
<222> (9)..(9)
<223> Non-natural amino acid used for cross linking

<400> 17

**Glu Ile Trp Ile Xaa Gln Glu Leu Xaa Arg Ile Gly Asp Glu Phe Asn**
**1 5 10 15**

**Ala Tyr Tyr Ala**
**20**

<210> 18
<211> 21
<212> PRT
<213> Artificial Sequence

<220>
<223> Description of Artificial Sequence: Synthetic peptide

<220>
<221> MOD_RES
<222> (13)..(13)
<223> Non-natural amino acid used for cross linking

<220>
<221> MOD_RES
<222> (17)..(17)
<223> Non-natural amino acid used for cross linking

<400> 18

**Ile Trp Ile Ala Gln Glu Leu Arg Arg Ile Gly Asp Xaa Phe Asn Ala**
**1 5 10 15**

**Xaa Tyr Ala Arg Arg**

**20**

<210> 19
<211> 20
<212> PRT
<213> Artificial Sequence

<220>
<223> Description of Artificial Sequence: Synthetic peptide

<220>
<221> MOD_RES
<222> (14)..(14)
<223> Non-natural amino acid used for cross linking

<220>

<221> MOD_RES
<222> (18)..(18)
<223> Non-natural amino acid used for cross linking

<400> 19

```
Glu Ile Trp Ile Ala Gln Glu Leu Arg Arg Ile Gly Asp Xaa Phe Asn
1               5                   10                  15

Ala Xaa Tyr Ala
            20
```

<210> 20
<211> 21
<212> PRT
<213> Artificial Sequence

<220>
<223> Description of Artificial Sequence: Synthetic peptide

<220>
<221> MOD_RES
<222> (5)..(5)
<223> Non-natural amino acid used for cross linking

<220>
<221> MOD_RES
<222> (9)..(9)
<223> Non-natural amino acid used for cross linking

<400> 20

```
Ile Trp Ile Ala Xaa Glu Leu Arg Xaa Ile Gly Asp Glu Phe Asn Ala
1               5                   10                  15

Tyr Tyr Ala Arg Arg
            20
```

<210> 21
<211> 20
<212> PRT
<213> Artificial Sequence

<220>
<223> Description of Artificial Sequence: Synthetic peptide

<220>
<221> MOD_RES
<222> (6)..(6)
<223> Non-natural amino acid used for cross linking

<220>
<221> MOD_RES
<222> (10)..(10)
<223> Non-natural amino acid used for cross linking

<400> 21

```
Glu Ile Trp Ile Ala Xaa Glu Leu Arg Xaa Ile Gly Asp Glu Phe Asn
1               5                   10                  15

Ala Tyr Tyr Ala
            20
```

<210> 22
<211> 21
<212> PRT
<213> Artificial Sequence

<220>
<223> Description of Artificial Sequence: Synthetic peptide

<220>
<221> MOD_RES
<222> (15)..(15)
<223> Non-natural amino acid used for cross linking

<220>
<221> MOD_RES
<222> (19)..(19)
<223> Non-natural amino acid used for cross linking

<400> 22

```
Ile Trp Ile Ala Gln Glu Leu Arg Arg Ile Gly Asp Glu Phe Xaa Ala
1               5                   10                  15

Tyr Tyr Xaa Arg Arg
            20
```

<210> 23
<211> 20
<212> PRT
<213> Artificial Sequence

<220>
<223> Description of Artificial Sequence: Synthetic peptide

<220>
<221> MOD_RES
<222> (16)..(16)
<223> Non-natural amino acid used for cross linking

<220>
<221> MOD_RES
<222> (20)..(20)
<223> Non-natural amino acid used for cross linking

<400> 23

```
Glu Ile Trp Ile Ala Gln Glu Leu Arg Arg Ile Gly Asp Glu Phe Xaa
1               5                   10                  15

Ala Tyr Tyr Xaa
            20
```

<210> 24
<211> 21
<212> PRT
<213> Artificial Sequence

<220>
<223> Description of Artificial Sequence: Synthetic peptide

<220>
<221> MOD_RES
<222> (12)..(12)
<223> Non-natural amino acid used for cross linking

<220>
<221> MOD_RES
<222> (16)..(16)
<223> Non-natural amino acid used for cross linking

<400> 24

```
Asp Ile Ile Arg Asn Ile Ala Arg His Leu Ala Xaa Val Gly Asp Xaa
1               5                   10                  15

Asx Asp Arg Ser Ile
            20
```

<210> 25
<211> 25
<212> PRT
<213> Artificial Sequence

<220>
<223> Description of Artificial Sequence: Synthetic peptide

<220>
<221> MOD_RES
<222> (16)..(16)
<223> Non-natural amino acid used for cross linking

<220>
<221> MOD_RES
<222> (20)..(20)
<223> Non-natural amino acid used for cross linking

<400> 25

```
Glu Ser Gln Glu Asp Ile Ile Arg Asn Ile Ala Arg His Leu Ala Xaa
1               5                   10                  15

Val Gly Asp Xaa Asx Asp Arg Ser Ile
            20                  25
```

<210> 26
<211> 21
<212> PRT
<213> Artificial Sequence

<220>
<223> Description of Artificial Sequence: Synthetic peptide

<220>
<221> MOD_RES
<222> (5)..(5)
<223> Non-natural amino acid used for cross linking

<220>
<221> MOD_RES
<222> (9)..(9)
<223> Non-natural amino acid used for cross linking

<400> 26

```
Asp Ile Ile Arg Xaa Ile Ala Arg Xaa Leu Ala Gln Val Gly Asp Glu
1               5                   10                  15

Asx Asp Arg Ser Ile
            20
```

<210> 27
<211> 25
<212> PRT
<213> Artificial Sequence

<220>
<223> Description of Artificial Sequence: Synthetic peptide

<220>
<221> MOD_RES
<222> (9)..(9)
<223> Non-natural amino acid used for cross linking

<220>
<221> MOD_RES
<222> (13)..(13)
<223> Non-natural amino acid used for cross linking

<400> 27

```
Glu Ser Gln Glu Asp Ile Ile Arg Xaa Ile Ala Arg Xaa Leu Ala Gln
1               5                   10                  15

Val Gly Asp Glu Asx Asp Arg Ser Ile
            20                  25
```

<210> 28
<211> 21
<212> PRT
<213> Artificial Sequence

<220>
<223> Description of Artificial Sequence: Synthetic peptide

<220>
<221> MOD_RES
<222> (12)..(12)
<223> Non-natural amino acid used for cross linking

<220>
<221> MOD_RES
<222> (16)..(16)
<223> Non-natural amino acid used for cross linking

<400> 28

```
Gln Trp Ala Arg Glu Ile Gly Leu Gln Ala Arg Xaa Asx Ala Asp Xaa
1               5                   10                  15

Leu Asn Ala Gln Tyr
            20
```

<210> 29
<211> 22
<212> PRT
<213> Artificial Sequence

<220>
<223> Description of Artificial Sequence: Synthetic peptide

<220>
<221> MOD_RES
<222> (13)..(13)
<223> Non-natural amino acid used for cross linking

<220>
<221> MOD_RES
<222> (17)..(17)
<223> Non-natural amino acid used for cross linking

<400> 29

```
Glu Gln Trp Ala Arg Glu Ile Gly Leu Gln Ala Arg Xaa Asx Ala Asp
```

1 5 10 15

Xaa Leu Asn Ala Gln Tyr
20

<210> 30
<211> 23
<212> PRT
<213> Artificial Sequence

<220>
<223> Description of Artificial Sequence: Synthetic peptide

<220>
<221> MOD_RES
<222> (13)..(13)
<223> Non-natural amino acid used for cross linking

<220>
<221> MOD_RES
<222> (17)..(17)
<223> Non-natural amino acid used for cross linking

<400> 30

Glu Gln Trp Ala Arg Glu Ile Gly Leu Gln Ala Arg Xaa Asx Ala Asp
1 5 10 15

Xaa Leu Asn Ala Gln Tyr Glu
20

<210> 31
<211> 21
<212> PRT
<213> Artificial Sequence

<220>
<223> Description of Artificial Sequence: Synthetic peptide

<220>
<221> MOD_RES
<222> (3)..(3)
<223> Non-natural amino acid used for cross linking

<220>
<221> MOD_RES
<222> (7)..(7)
<223> Non-natural amino acid used for cross linking

<400> 31

```
Gln Trp Xaa Arg Glu Ile Xaa Leu Gln Ala Arg Arg Asx Ala Asp Asp
1               5                   10                  15

Leu Asn Ala Gln Tyr
            20
```

<210> 32
<211> 23
<212> PRT
<213> Artificial Sequence

<220>
<223> Description of Artificial Sequence: Synthetic peptide

<220>
<221> MOD_RES
<222> (4)..(4)
<223> Non-natural amino acid used for cross linking

<220>
<221> MOD_RES
<222> (8)..(8)
<223> Non-natural amino acid used for cross linking

<400> 32

```
Glu Gln Trp Xaa Arg Glu Ile Xaa Leu Gln Ala Arg Arg Asx Ala Asp
1               5                   10                  15

Asp Leu Asn Ala Gln Tyr Glu
            20
```

<210> 33
<211> 22
<212> PRT
<213> Artificial Sequence

<220>
<223> Description of Artificial Sequence: Synthetic peptide

<220>
<221> MOD_RES
<222> (14)..(14)
<223> Non-natural amino acid used for cross linking

<220>
<221> MOD_RES
<222> (18)..(18)
<223> Non-natural amino acid used for cross linking

<400> 33

Gln Asp Ala Ser Thr Lys Lys Leu Ser Glu Cys Leu Lys Xaa Ile Gly
1 5 10 15

Asp Xaa Leu Asp Ser Asn
20

<210> 34
<211> 22
<212> PRT
<213> Artificial Sequence

<220>
<223> Description of Artificial Sequence: Synthetic peptide

<220>
<221> MOD_RES
<222> (7)..(7)
<223> Non-natural amino acid used for cross linking

<220>
<221> MOD_RES
<222> (11)..(11)
<223> Non-natural amino acid used for cross linking

<400> 34

Gln Asp Ala Ser Thr Lys Xaa Leu Ser Glu Xaa Leu Lys Arg Ile Gly
1 5 10 15

Asp Glu Leu Asp Ser Asn
20

<210> 35
<211> 22
<212> PRT
<213> Artificial Sequence

<220>
<223> Description of Artificial Sequence: Synthetic peptide

<220>
<221> MOD_RES
<222> (8)..(8)
<223> Non-natural amino acid used for cross linking

<220>
<221> MOD_RES
<222> (12)..(12)
<223> Non-natural amino acid used for cross linking

<400> 35

```
Gln Asp Ala Ser Thr Lys Lys Xaa Ser Glu Cys Xaa Lys Arg Ile Gly
1               5                   10                  15

Asp Glu Leu Asp Ser Asn
            20
```

<210> 36
<211> 22
<212> PRT
<213> Artificial Sequence

<220>
<223> Description of Artificial Sequence: Synthetic peptide

<220>
<221> MOD_RES
<222> (9)..(9)
<223> Non-natural amino acid used for cross linking

<220>
<221> MOD_RES
<222> (13)..(13)
<223> Non-natural amino acid used for cross linking

<400> 36

```
Gln Asp Ala Ser Thr Lys Lys Leu Xaa Glu Cys Leu Xaa Arg Ile Gly
1               5                   10                  15

Asp Glu Leu Asp Ser Asn
            20
```

<210> 37
<211> 22
<212> PRT
<213> Homo sapiens

<400> 37

```
Glu Ile Trp Ile Ala Gln Glu Leu Arg Arg Ile Gly Asp Glu Phe Asn
1               5                   10                  15

Ala Tyr Tyr Ala Arg Arg
            20
```

<210> 38
<211> 24
<212> **PRT**
<213> Homo sapiens

<400> 38

```
Glu Gln Trp Ala Arg Glu Ile Gly Ala Gln Leu Arg Arg Met Ala Asp
1               5                   10                  15

Asp Leu Asn Ala Gln Tyr Glu Arg
            20
```

<210> 39
<211> 27
<212> PRT
<213> Homo sapiens

<400> 39

```
Gln Asp Ala Ser Thr Lys Lys Leu Ser Glu Cys Leu Lys Arg Ile Gly
1               5                   10                  15

Asp Glu Leu Asp Ser Asn Met Glu Leu Gln Arg
            20                  25
```